# EUROPEAN PATENT SPECIFICATION

(11) **EP 3 937 963 B1**
(45) Date of publication and mention of the grant of the patent: **07.05.2025**
(21) Application number: 20717052.3
(22) Date of filing: 12.03.2020
(51) Int. Cl.: A61K 38/00, C12N 9/22, C12N 15/10, C12N 15/63, C12N 15/90

(54) **NOVEL HIGH FIDELITY RNA-PROGRAMMABLE ENDONUCLEASE SYSTEMS AND USES THEREOF**
NEUARTIGE RNA-PROGRAMMIERBARE HIGH-FIDELITY-ENDONUKLEASESYSTEME UND VERWENDUNGEN DAVON
NOUVEAUX SYSTÈMES D'ENDONUCLÉASE À ARN PROGRAMMABLE HAUTE FIDÉLITÉ ET LEURS UTILISATIONS

(30) Priority: 12.03.2019 US 201962817514 P
(43) Date of publication of application: 19.01.2022
(73) Proprietor: CRISPR Therapeutics AG, 6300 Zug (CH); Bayer Healthcare LLC, Whippany, NJ 07981 (US)
(72) Inventor: SCHMIDT, Moritz, 50829 Cologne (DE); KNYPHAUSEN, Philipp, 50829 Cologne (DE); GALONSKA, Christina, 50829 Cologne (DE); COCO, Wayne M., 50829 Cologne (DE); COHEN, Andre, 50829 Cologne (DE)
(74) Representative: Vossius & Partner Patentanwälte Rechtsanwälte mbB
(86) International application number: PCT/US2020/022394
(87) International publication number: WO 2020/186059

(56) References cited:
- WO-A1-2013/176772
- WO-A1-2013/176772
- WO-A1-2015/089277
- WO-A1-2015/089277
- WO-A1-2017/040348
- WO-A1-2019/118935
- WO-A1-2019/118935
- HIROSHI NISHIMASU ET AL: "Crystal Structure of Staphylococcus aureus Cas9", CELL, vol. 162, no. 5, 1 August 2015 (2015-08-01), AMSTERDAM, NL, pages 1113 - 1126, XP055304450, ISSN: 0092-8674, DOI: 10.1016/j.cell.2015.08.007
- ANONYMOUS: "CAS9 FROM STAPHYLOCOCCUS AUREUS", 13 February 2019 (2019-02-13), XP055711995, Retrieved from the Internet <URL:https://www.uniprot.org/uniprot/J7RUA5.txt?version=32> [retrieved on 20200706]
- ANONYMOUS: "Cas 9 from Staphylococcus simulans", 10 October 2018 (2018-10-10), XP055712006, Retrieved from the Internet <URL:https://www.uniprot.org/uniprot/A0A2T4QQ61.txt?version=3> [retrieved on 20200706]
- ANONYMOUS: "CRISPR-associated endonuclease Cas1 [Staphylococcus aureus] - Protein - NCBI", 9 May 2015 (2015-05-09), XP055711777, Retrieved from the Internet <URL:https://www.ncbi.nlm.nih.gov/protein/cri31653.1> [retrieved on 20200706]
- MEHMET FATIH BOLUKBASI ET AL: "Creating and evaluating accurate CRISPR-Cas9 scalpels for genomic surgery", NATURE METHODS, vol. 13, no. 1, 1 January 2016 (2016-01-01), New York, pages 41 - 50, XP055283692, ISSN: 1548-7091, DOI: 10.1038/nmeth.3684

## Description

### RELATED APPLICATIONS

This application claims the benefit of U.S. Provisional Patent Application Serial No. 62/817,514, filed March 12, 2019.

### FIELD OF THE INVENTION

The present disclosure generally relates to the field of molecular biology, in particular a novel nuclease for gene editing as defined in the claims.

### BACKGROUND

Editing genomes using the RNA-guided DNA targeting principle of CRISPR (Clustered Regularly Interspaced Short Palindromic Repeats)-Cas (CRISPR associated proteins), has been widely exploited over the past few years. Three types of CRISPR-Cas systems (type I, type II and IIb, type III, and type V) have been described. Most uses of CRISPR-Cas for genome editing have been with a type II system. The main advantage provided by the bacterial type II CRISPR-Cas system lies in the minimal requirement for programmable DNA interference: an endonuclease, Cas9, guided by a customizable dual-RNA structure. As initially demonstrated in the original type II system of Streptococcus pyogenes, trans-activating CRISPR RNA (tracrRNA) binds to the invariable repeats of precursor CRISPR RNA (pre-crRNA) forming a dual-RNA that is essential for both RNA co-maturation by RNase III in the presence of Cas9, and invading DNA cleavage by Cas9. As demonstrated in *Streptococcus pyogenes,* Cas9 guided by the duplex formed between mature activating tracrRNA and targeting crRNA introduces site-specific double-stranded DNA (dsDNA) breaks in the invading cognate DNA. Cas9 is a multi-domain enzyme that uses an HNH nuclease domain to cleave the target strand (defined as complementary to the spacer sequence of crRNA) and a RuvC-like domain to cleave the non-target strand. The nuclease can act as a nickase by selective motif inactivation of the nuclease. DNA cleavage specificity is determined by two parameters: the variable, spacer-derived sequence of crRNA targeting the protospacer sequence (the sequence on the DNA target that is non-complementary to the spacer of crRNA) and a short sequence, the Protospacer Adjacent Motif (PAM), located immediately 3' (downstream) of the protospacer on the non-target DNA strand.

Studies have demonstrated that RNA-guided Cas9 can be employed as genome editing tool in a variety of cells including those of prokaryotes and eukaryotes including human. For example, WO 2013/176772, WO 2015/089288 and WO 2019/118935 (i.e., cited as document under Article 54(3) EPC) disclose genome editing based on a Cas9 endonuclease from *Staphylococcus lugdunensis.* The system is versatile, enabling multiplex genome engineering by programming Cas9 to edit several sites in a genome simultaneously by using multiple guide RNAs. The conversion of Cas9 into a nickase was shown to facilitate homology-directed repair in mammalian genomes with reduced mutagenic activity. In addition, the DNA-binding activity of a Cas9 catalytic inactive mutant has for example been exploited to engineer RNA-programmable transcriptional silencing and activating devices or epigenetic modifiers.

Genome editing in mammalian cells has been limited, in part, by the size of Cas9 proteins. Cas9 from *Staphylococcus pyogenes* (SpyCas9), the enzyme most widely used to date, comprises approximately 4.2kb of DNA (WO2013/176722) and a direct combination with cognate single guide RNAs (sgRNA) further increases the size. Adeno-associated viruses are among the vectors used for the delivery of Cas9 enzymes in gene therapy applications. However, AAV cargo size is restricted to about 4.5 kb. Because of the size constraints, delivering a Cas9 with its sgRNA and a potential DNA repair template can be an impediment to using the methods. Smaller Cas9 molecules have been characterized, but most of them suffer from a protospacer adjacent motif (PAM) sequence that is not as well defined as the one used by SpyCas9. For example, *Staphylococcus aureus* (SauCas9 uses an "NNGRR(T)" sequence, where R = A or G, and *Campylobacter jejuni* (Cja)Cas9 uses a "NNNACAC"/ "NNNRYAC" PAM (where Y = T or G), respectively. The PAM ambiguity increases the potential for undesirable activity of the enzyme at off-target sequences harbouring high or perfect sequence identity to the PAM. Specificity of these systems remains a concern, as targeting similar sites by accident ("off-targets") will increase the likelihood of adverse events.

Existing CRISPR-Cas 9 systems generally have one or more of the following disadvantages:
a) Their size is too large to be carried inside the genome of established therapeutically-suitable viral delivery systems like adeno associated viruses (AAVs).
b) Many of them are not substantially active in non-host environments, for example in eukaryotic cells, and in particular in mammalian cells.
c) Their nuclease can catalyze DNA strand cleavage when mismatches between spacer and protospacer sequences are present, leading to undesired off target effects that would for example make them unsuitable for gene therapeutic uses or other applications requiring high precision.
d) They may trigger an immune response that can limit their use for *in vivo* applications in mammals.
e) They require complex and/or long PAMs that restrict target selection for the DNA targeting segments.
f) They exhibit poor expression from plasmid or viral vectors.

### Summary of the Invention

Herein described are improved Cas nucleases that at least can have increased specificity compared to certain wildtype enzymes, as well as having a relatively small size.

Specifically, the present invention defines a M-SmallCas9 polypeptide comprising an amino acid sequence according to SEQ ID NO. 7 (M-SluCas9-R414A) and is further defined in the claims.

Additional features and advantages of the invention are more particularly described below.

### Brief Description of the Drawings

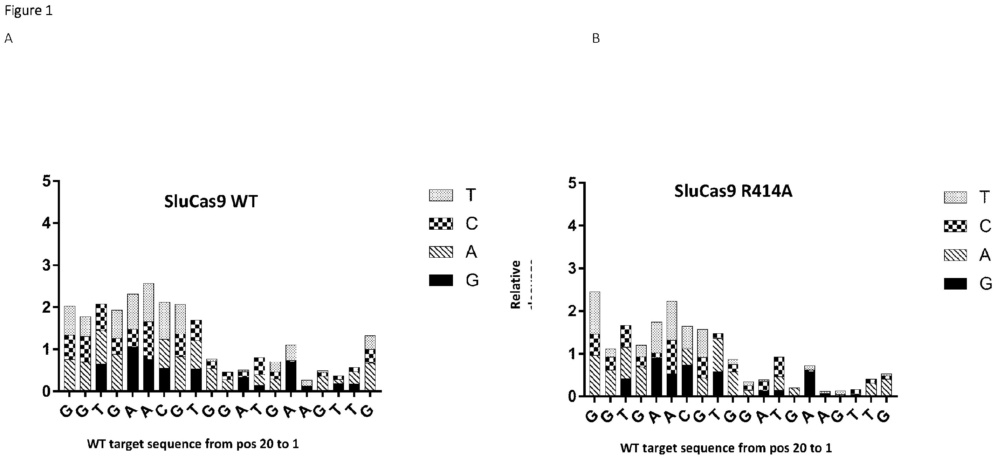

Figures 1A and 1B are bar graphs depicting the results of experiments testing the relative cleavage activity (y axis) of Cas nucleases as a function a) position of nucleic acid within the target sequence as shown on the x-axis and b) the respective nucleic acid residue in that position, which is visualized by differently shaded boxes that are stacked. The absence of, *e.g.,* the box for "G" in a column means that no cleavage activity was seen in that position when the residue "G" was present. Figure 1A shows these data for SluCas9 according to SEQ ID NO. 9; Figure 1B shows these data for MSluCas9R414A according to SEQ ID NO. 7.

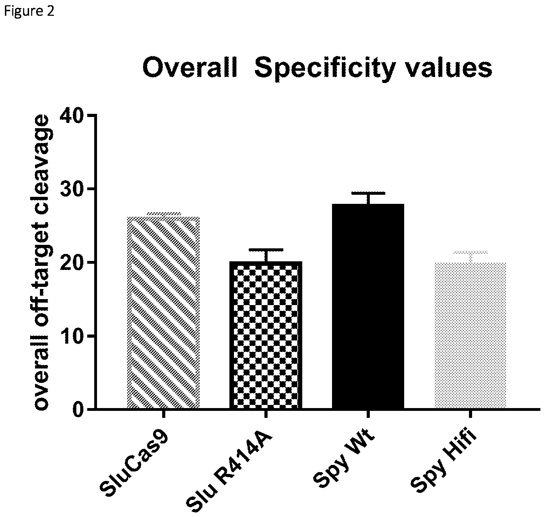

Figure 2 is a bar graph depicting the results of experiments detecting the total specificity values of four different Cas enzymes: SluCas9 (SEQ ID NO: 9), M-SluCas9R414A (SEQ ID NO. 7), *Staphylococcus pyogenes* wild type (New England Biolabs), *Staphylococcus pyogenes* HiFi (Integrated DNA Technologies).

### Detailed Description of the Invention

The present application provides novel CRISPR-Cas nucleases and gene editing systems based on such nucleases that have been derived by mutagenesis of existing small CRISPR-Cas9 nucleases from *Staphylococcus lugdunensis.* These nucleases exhibit advantages compared to existing CRISPR-Cas systems, in particular, advantages compared to parent nucleases from which they are derived. The novel nucleases are referred to herein as M-Small Cas nucleases (or M-SmallCas9 nucleases). Examples of improved activities of an M-Small Cas can include higher activity in prokaryotic, eukaryotic, and/or *in vitro* environments, or when expressed from a DNA plasmid in eukaryotic environments, such as, *e.g.,* a human host cell. In particular, they exhibit improved fidelity over existing CRISPR Cas9 systems combined with favorable enzyme sizes.

The novel CRISPR-Cas nuclease is defined in claim 1 and is denoted M-SluCas9-R414A. The M-Slu-Cas9 endonuclease as defined in the claims has improved gene editing fidelity compared to its corresponding wildtype nuclease. A suitable PAM Sequences for the corresponding M-SluCas9 endonuclease is "NNGG" (N being any of A, T, C, G).

The polynucleotide encoding M-SmallCas9 and the sgRNAs may contain a suitable promoter for the expression in a cellular or *in vitro* environment and/or a suitable nuclear localization signal.

### Multiplexing

In another aspect, provided herein is a method for editing or modifying DNA at multiple locations in a cell, the method consisting essentially of: i) introducing a M-SmallCas9 polypeptide or a nucleic acid encoding a M-SmallCas9 polypeptide into the cell; and ii) introducing a single heterologous nucleic acid comprising two or more pre-CRISPR RNAs (pre-crRNAs) either as RNA or encoded as DNA and under the control of one promoter into the cell, each pre-crRNA comprising a repeat-spacer array or repeat-spacer, wherein the spacer comprises a nucleic acid sequence that is complementary to a target sequence in the DNA and the repeat comprises a stem-loop structure, wherein the M-SmallCas9 polypeptide cleaves the two or more pre-crRNAs upstream of the stem-loop structure to generate two or more intermediate crRNAs, wherein the two or more intermediate crRNAs are processed into two or more mature crRNAs, and wherein each two or more mature crRNAs guides the M-SmallCas9 polypeptide to effect two or more double-strand breaks (DSBs) into the DNA. For example, one advantage of M-SmallCas9 is that it is possible to introduce only one pre-crRNA which comprises several repeat-spacer units, which upon introduction, is processed by M-SmallCas9 it into active repeat-spacer units targeting several different sequences on the DNA.

In another aspect, provided herein is a method for editing or modifying DNA at multiple locations in a cell consisting essentially of: i) introducing a form of M-SmallCas9 with reduced endoribonuclease activity, as a polypeptide or a nucleic acid encoding a M-SmallCas9 polypeptide into the cell; and ii) introducing a single heterologous nucleic acid comprising two or more pre-CRISPR RNAs (pre-crRNAs), intermediate crRNAs or mature crRNAs either as RNA or encoded as DNA and under the control of one or more promoters, each crRNA comprising a repeat-spacer array, wherein the spacer comprises a nucleic acid sequence that is complementary to a target sequence in the DNA and the repeat comprises a stem-loop structure, wherein the M-SmallCas9 polypeptide binds to one or more regions of the single heterologous RNA with reduced or absent endoribonuclease activity and with intact endonuclease activity as directed by one or more spacer sequences in the single heterologous nucleic acid.

The pre-crRNA sequences in the single heterologous nucleic acid may be joined together in specific locations, orientations, sequences or with specific chemical linkages to direct or differentially modulate the endonuclease activity of M-SmallCas9 at each of the sites specified by the different crRNA sequences.

In another aspect, provided herein is an example of a general method for editing or modifying the structure or function of DNA at multiple locations in a cell consisting essentially of: i) introducing an RNA-guided endonuclease, such as M-SmallCas9, as a polypeptide or a nucleic acid encoding the RNA-guided endonuclease into the cell; and ii) introducing a single heterologous nucleic acid comprising or encoding two or more guide RNAs, either as RNA or encoded as DNA and under the control of one or more promoters, wherein the activity or function of the RNA-guided endonuclease is directed by the guide RNA sequences in the single heterologous nucleic acid.

### Definitions

The terms "polynucleotide," "nucleic acid," and "nucleic acid," used interchangeably herein, refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA-RNA hybrids/triple helices, or a polymer including purine and pyrimidine bases or other natural, chemically or biochemically modified, non-natural, or derivatized nucleotide bases.

"Oligonucleotide" generally refers to polynucleotides of between about 5 and about 100 nucleotides of single- or double-stranded DNA. However, for the purposes of this disclosure, there is no upper limit to the length of an oligonucleotide. Oligonucleotides are also known as "oligomers" or "oligos" and may be isolated from genes, or chemically synthesized by methods known in the art. The terms "polynucleotide" and "nucleic acid" should be understood to include, as applicable to the embodiments being described, single-stranded (such as sense or antisense) and double-stranded polynucleotides.

Genomic DNA" refers to the DNA of a genome of an organism including, but not limited to, the DNA of the genome of a bacterium, fungus, archaeon, protist, virus, plant or animal.

The term "manipulating" DNA encompasses binding, nicking one strand, or cleaving, e.g. cutting both strands of the DNA; or encompasses modifying or editing the DNA or a polypeptide associated with the DNA. Manipulating DNA can silence, activate, or modulate (either increase or decrease) the expression of an RNA or polypeptide encoded by the DNA, or prevent or enhance the binding of a polypeptide to DNA.

A "stem-loop structure" refers to a nucleic acid having a secondary structure that includes a region of nucleotides which are known or predicted to form a double strand (stem portion) that is linked on one side by a region of predominantly single-stranded nucleotides (loop portion). The terms "hairpin" and "fold-back" structures are also used herein to refer to stem-loop structures. Such structures are well known in the art and these terms are used consistently with their known meanings in the art. As is known in the art, a stem-loop structure does not require exact base-pairing. Thus, the stem may include one or more base mismatches. Alternatively, the base-pairing may be exact, e.g., not include any mismatches.

By "hybridizable" or "complementary" or "substantially complementary" it is meant that a nucleic acid (e.g. RNA or DNA) includes a sequence of nucleotides that enables it to non-covalently bind, e.g., form Watson-Crick base pairs and/or G/U base pairs, "anneal", or "hybridize," to another nucleic acid in a sequence-specific, antiparallel, manner (*e.g.*, a nucleic acid specifically binds to a complementary nucleic acid) under the appropriate *in vitro* and/or *in vivo* conditions of temperature and solution ionic strength. As is known in the art, standard Watson-Crick base-pairing includes: adenine (A) pairing with thymidine (T), adenine (A) pairing with uracil (U), and guanine (G) pairing with cytosine (C) [DNA, RNA]. In addition, it is also known in the art that for hybridization between two RNA molecules (*e.g*. dsRNA), guanine (G) base pairs with uracil (U). For example, G/U base-pairing is partially responsible for the degeneracy (*e.g.*, redundancy) of the genetic code in the context of tRNA anti-codon base-pairing with codons in mRNA. In the context of this disclosure, a guanine (G) of a protein-binding segment (dsRNA duplex) of a guide RNA molecule is considered complementary to a uracil (U), and vice versa. As such, when a G/U base-pair can be made at a given nucleotide position a protein-binding segment (dsRNA duplex) of a guide RNA molecule, the position is not considered to be non-complementary, but is instead considered to be complementary.

Hybridization and washing conditions are well known and exemplified in Sambrook, J., Fritsch, E. F. and Maniatis, T. Molecular Cloning: A Laboratory Manual, Second Edition, Cold Spring Harbor Laboratory Press, Cold Spring Harbor (1989), particularly Chapter 11 and Table 11.1 therein; and Sambrook, J. and Russell, W., Molecular Cloning: A Laboratory Manual, Third Edition, Cold Spring Harbor Laboratory Press, Cold Spring Harbor (2001). The conditions of temperature and ionic strength determine the "stringency" of the hybridization.

Hybridization requires that the two nucleic acids contain complementary sequences, although mismatches between bases are possible. The conditions appropriate for hybridization between two nucleic acids depend on the length of the nucleic acids and the degree of complementation, variables well known in the art. The greater the degree of complementation between two nucleotide sequences, the greater the value of the melting temperature (Tm) for hybrids of nucleic acids having those sequences. For hybridizations between nucleic acids with short stretches of complementarity (*e.g.* complementarity over 35 or less, 30 or less, 25 or less, 22 or less, 20 or less, or 18 or less nucleotides) the position of mismatches becomes important (see Sambrook *et al.,* supra, 11.7-11.8). Generally, the length for a hybridizable nucleic acid is at least 10 nucleotides. Illustrative minimum lengths for a hybridizable nucleic acid are: at least 15 nucleotides; at least 20 nucleotides; at least 22 nucleotides; at least 25 nucleotides; and at least 30 nucleotides). Furthermore, the skilled artisan will recognize that the temperature and wash solution salt concentration maybe adjusted as necessary according to factors such as length of the region of complementation and the degree of complementation.

It is understood in the art that the sequence of polynucleotide need not be 100% complementary to that of its target nucleic acid to be specifically hybridizable. Moreover, a polynucleotide may hybridize over one or more segments such that intervening or adjacent segments are not involved in the hybridization event (*e.g.* a loop structure or hairpin structure). A polynucleotide can include at least 70%, at least 80%, at least 90%, at least 95%, at least 99%, or 100% sequence complementarity to a target region within the target nucleic acid sequence to which they are targeted. For example, an antisense nucleic acid in which 18 of 20 nucleotides of the antisense compound are complementary to a target region, and would therefore specifically hybridize, would represent 90 percent complementarity. In this example, the remaining non complementary nucleotides may be clustered or interspersed with complementary nucleotides and need not be contiguous to each other or to complementary nucleotides. Percent complementarity between particular stretches of nucleic acid sequences within nucleic acids can be determined routinely using BLAST programs (basic local alignment search tools) and PowerBLAST programs known in the art (Altschul et al., J. Mol. Biol. 1990,215, 403-410; Zhang and Madden, Genome Res., 1997,7, 649-656) or by using the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math. 1981(2) 482-489).

The terms "peptide", "polypeptide", and "protein" are used interchangeably herein, and refer to a polymeric form of amino acids of any length, which can include coded and non-coded amino acids, chemically or biochemically modified or derivatized amino acids, and polypeptides having modified peptide backbones.

"Binding" as used herein (*e.g*. with reference to an RNA-binding domain of a polypeptide) refers to a non-covalent interaction between macromolecules (*e.g*. between a protein and a nucleic acid). While in a state of non-covalent interaction, the macromolecules are said to be "associated" or "interacting" or "binding" (*e.g.* when a molecule X is said to interact with a molecule Y, it is meant the molecule X binds to molecule Y in a non-covalent manner). Not all components of a binding interaction need be sequence-specific (*e.g.* contacts with phosphate residues in a DNA backbone), but some portions of a binding interaction may be sequence-specific. Binding interactions are generally characterized by a dissociation constant (Kd) of less than 10⁻⁶ M, less than 10⁻⁷ M, less than 10⁻⁸ M, less than 10⁻⁹ M, less than 10⁻¹⁰ M, less than 10⁻¹¹ M, less than 10⁻¹² M, less than 10⁻¹³ M, less than 10⁻¹⁴ M, or less than 10⁻¹⁵ M. "Affinity" refers to the strength of binding, increased binding affinity being correlated with a lower Kd.

By "binding domain" it is meant a protein domain that is able to bind non-covalently to another molecule. A binding domain can bind to, for example, a DNA molecule (a DNA-binding protein), an RNA molecule (an RNA-binding protein) and/or a protein molecule (a protein-binding protein). In the case of a protein domain-binding protein, it can bind to itself (to form homo-dimers, homo-trimers, *etc.*) and/or it can bind to one or more molecules of a different protein or proteins.

The term "conservative amino acid substitution" refers to the interchangeability in proteins of amino acid residues having similar side chains. For example, a group of amino acids having aliphatic side chains consists of glycine, alanine, valine, leucine, and isoleucine; a group of amino acids having aliphatic-hydroxyl side chains consists of serine and threonine; a group of amino acids having amide containing side chains consisting of asparagine and glutamine; a group of amino acids having aromatic side chains consists of phenylalanine, tyrosine, and tryptophan; a group of amino acids having basic side chains consists of lysine, arginine, and histidine; a group of amino acids having acidic side chains consists of glutamate and aspartate; and a group of amino acids having sulfur containing side chains consists of cysteine and methionine. Exemplary conservative amino acid substitution groups are: valine-leucine-isoleucine, phenylalanine-tyrosine, lysine-arginine, alanine-valine, and asparagine-glutamine.

A polynucleotide or polypeptide has a certain percent "sequence identity" to another polynucleotide or polypeptide, meaning that, when aligned, that percentage of bases or amino acids are the same, and in the same relative position, when comparing the two sequences. Sequence identity can be determined in a number of different manners. To determine sequence identity, sequences can be aligned using various methods and computer programs (*e.g*. BLAST, T-COFFEE, MUSCLE, MAFFT, *etc.*)*,* available over the world-wide-web at sites including ncbi.nlm.nili.gov/BLAST, ebi.ac.uk/Tools/msa/tcoffee, ebi.Ac.Uk/Tools/msa/muscle, mafft.cbrc/alignment/software. See, *e.g.* Altschul et al. (1990), J. Mol. Biol. 215:403-10. Sequence alignment standards in the art may be used to determine amino acid residues in M-SmallCas9 polypeptide or variant thereof that "correspond to" amino acid residues in another Cas9 endonuclease. The amino acid residues of M-SmallCas9 polypeptides or variants thereof that correspond to amino acid residues of other Cas9 endonucleases appear at the same position in alignments of the sequences.

A DNA sequence that "encodes" a particular RNA is a DNA nucleic acid sequence that is transcribed into the RNA. A polydeoxyribonucleotide may encode an RNA (mRNA) that is translated into protein, or a polydeoxyribonucleotide may encode an RNA that is not translated into protein (e.g. tRNA, rRNA, or a guide RNA; also called "non-coding" RNA or "ncRNA"). A "protein coding sequence" or a sequence that encodes a particular protein or polypeptide, is a nucleic acid sequence that is transcribed into mRNA (in the case of DNA) and is translated (in the case of mRNA) into a polypeptide *in vitro* or *in vivo* when placed under the control of appropriate regulatory sequences. The boundaries of the coding sequence are determined by a start codon at the 5' terminus (N-terminus) and a translation stop nonsense codon at the 3' terminus (C-terminus). A coding sequence can include, but is not limited to, cDNA from prokaryotic or eukaryotic mRNA, genomic DNA sequences from prokaryotic or eukaryotic DNA, and synthetic nucleic acids. A transcription termination sequence will generally be located at 3' of the coding sequence.

As used herein, a "promoter sequence" or "promoter" is a DNA regulatory region capable of binding RNA polymerase and initiating transcription of a downstream (3' direction) coding or non-coding sequence. As used herein, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site, as well as protein binding domains responsible for the binding of RNA polymerase. Eukaryotic promoters will often, but not always, contain "TATA" boxes and "CAAT" boxes. Various promoters, including inducible promoters, may be used to drive the various vectors of the present disclosure. A promoter can be a constitutively active promoter (*e.g*., a promoter that is constitutively in an active "ON" state), it may be an inducible promoter (*e.g*., a promoter whose state, active/"ON" or inactive/"OFF", is controlled by an external stimulus, *e.g.* the presence of a particular temperature, compound, or protein.), it may be a spatially restricted promoter (*e.g.,* transcriptional control element, enhancer, *etc.*)(*e.g.* tissue specific promoter, cell type specific promoter, *etc.),* and it may be a temporally restricted promoter (*e.g.,* the promoter is in the "ON" state or "OFF" state during specific stages of embryonic development or during specific stages of a biological process, *e.g*. hair follicle cycle in mice). Suitable promoters can be derived from viruses and can therefore be referred to as viral promoters, or they can be derived from any organism, including prokaryotic or eukaryotic organisms. Suitable promoters can be used to drive expression by any RNA polymerase (*e.g*. pol I, pol II, pol III). Exemplary promoters include, but are not limited to the SV40 early promoter, mouse mammary tumor virus long terminal repeat (LTR) promoter; adenovirus major late promoter (Ad MLP); a herpes simplex virus (HSV) promoter, a cytomegalovirus (CMV) promoter such as the CMV immediate early promoter region (CMVIE), a Rous sarcoma virus (RSV) promoter, a human U6 small nuclear promoter (U6) (Miyagishi et al. , Nature Biotechnology 20, 497-500 (2002)), an enhanced U6 promoter (*e.g.* Xia et al., Nucleic Acids Res. 2003 Sep 1;31(17)), a human H1 promoter (H1), and the like. Examples of inducible promoters include, but are not limited to T7 RNA polymerase promoter, T3 RNA polymerase promoter, isopropyl-beta-D-thiogalactopyranoside (IPTG)-regulated promoter, lactose induced promoter, heat shock promoter, Tetracycline-regulated promoter, Steroid- regulated promoter, Metal-regulated promoter, estrogen receptor-regulated promoter, *etc.* Inducible promoters can therefore be regulated by molecules including, but not limited to, doxycycline; RNA polymerase, *e.g.* T7 RNA polymerase; an estrogen receptor; an estrogen receptor fusion; *etc.*

The promoter may be a spatially restricted promoter (*e.g*., cell type specific promoter, tissue specific promoter, *etc.*) such that in a multi-cellular organism, the promoter is active (*e.g.,* "ON") in a subset of specific cells. Spatially restricted promoters may also be referred to as enhancers, transcriptional control elements, control sequences, *etc.* Any suitable spatially restricted promoter may be used and the choice of suitable promoter (*e.g*. a brain specific promoter, a promoter that drives expression in a subset of neurons, a promoter that drives expression in the germline, a promoter that drives expression in the lungs, a promoter that drives expression in muscles, a promoter that drives expression in islet cells of the pancreas, *etc.*) will depend on the organism. For example, various spatially restricted promoters are known for plants, flies, worms, mammals, mice, *etc.* Thus, a spatially restricted promoter can be used to regulate the expression of a nucleic acid encoding a site-specific modifying enzyme in a wide variety of different tissues and cell types, depending on the organism. Some spatially restricted promoters are also temporally restricted such that the promoter is in the "ON" state or "OFF" state during specific stages of embryonic development or during specific stages of a biological process (*e.g.* hair follicle cycle in mice). For illustration purposes, examples of spatially restricted promoters include, but are not limited to, neuron-specific promoters, adipocyte-specific promoters, cardiomyocyte-specific promoters, smooth muscle-specific promoters, photoreceptor-specific promoters, *etc.* Neuron-specific spatially restricted promoters include, but are not limited to, a neuron-specific enolase (NSE) promoter (see, *e.g*. EMBL HSEN02, X51956); an aromatic amino acid decarboxylase (AADC) promoter; a neurofilament promoter (see, *e.g.* GenBank HUMNFL, L04147); a synapsin promoter (see, *e.g.* GenBank HUMSYNIB, M55301); a thy-1 promoter (see, *e.g.* Chen et al. (1987) Cell 51:7-19; and Llewellyn, et al. (2010) Nat. Med. 16(10):1161-1166); a serotonin receptor promoter (see, *e.g*. GenBank S62283); a tyrosine hydroxylase promoter (TH) (see, *e.g.* Oh et al. (2009) Gene Ther. 16:437; Sasaoka et al. (1992) Mol. Brain Res. 16:274; Boundy et al.(1998) J. Neurosci. 18:9989; and Kaneda et al. (1991) Neuron 6:583-594); a GnRH promoter (see, *e.g.* Radovick et al. (1991) Proc. Natl. Acad. Sci. USA 88:3402-3406); an L7 promoter (see, *e.g.* Oberdick et al.(1990) Science 248:223-226); a DNMT promoter (see, *e.g.* Bartge et al. (1988) Proc. Natl. Acad. Sci. USA 85:3648-3652); an enkephalin promoter (see, *e.g.* Comb et al. (1988) EMBO J. 17:3793-3805); a myelin basic protein (MBP) promoter; a Ca2+-calmodulin-dependent protein kinase 11-alpha (CamKIM) promoter (see, *e.g.* Mayford et al. (1996) Proc. Natl. Acad. Sci. USA 93:13250; and Casanova et al. (2001) Genesis 31:37); a CMV enhancer/platelet-derived growth factor-p promoter (see, *e.g.* Liu et al. (2004) Gene Therapy 11:52-60); and the like.

The terms "DNA regulatory sequences," "control elements," and "regulatory elements," used interchangeably herein, refer to transcriptional and translational control sequences, such as promoters, enhancers, polyadenylation signals, terminators, protein degradation signals, and the like, that provide for and/or regulate transcription of a non-coding sequence (*e.g*. guide RNA) or a coding sequence (*e.g*. M-SmallCas9 polypeptide or variant thereof) and/or regulate translation of an encoded polypeptide.

The term "naturally-occurring" or "unmodified" as used herein as applied to a nucleic acid, a polypeptide, a cell, or an organism, refers to a nucleic acid, polypeptide, cell, or organism that is found in nature. For example, a polypeptide or polynucleotide sequence that is present in an organism (including viruses) that can be isolated from a source in nature and which has not been intentionally modified by a human in the laboratory is naturally occurring.

The term "chimeric" as used herein as applied to a nucleic acid or polypeptide refers to one entity that is composed of structures derived from different sources. For example, where "chimeric" is used in the context of a chimeric polypeptide (*e.g*. a chimeric M-SmallCas9 protein), the chimeric polypeptide includes amino acid sequences that are derived from different polypeptides. A chimeric polypeptide may include either modified or naturally-occurring polypeptide sequences (*e.g*. a first amino acid sequence from a modified or unmodified M-SmallCas9 protein; and a second amino acid sequence other than the M-SmallCas9 protein). Similarly, "chimeric" in the context of a polynucleotide encoding a chimeric polypeptide includes nucleotide sequences derived from different coding regions (*e.g.* a first nucleotide sequence encoding a modified or unmodified M-SmallCas9 protein; and a second nucleotide sequence encoding a polypeptide other than a M-SmallCas9 protein).

The term "chimeric polypeptide" refers to a polypeptide which is not naturally occurring, *e.g*. is made by the artificial combination (*e.g*., "fusion") of two or more otherwise separated segments of amino sequence through human intervention. A polypeptide that includes a chimeric amino acid sequence is a chimeric polypeptide. Some chimeric polypeptides can be referred to as "fusion variants."

"Heterologous," as used herein, means a nucleotide or peptide that is not found in the native nucleic acid or protein, respectively. A M-SmallCas9 fusion protein described herein may comprise the RNA-binding domain of the M-SmallCas9 polypeptide (or a variant thereof) fused to a heterologous polypeptide sequence (*e.g*., a polypeptide sequence from a protein other than M-SmallCas9). The heterologous polypeptide may exhibit an activity (*e.g*. enzymatic activity) that will also be exhibited by the M-SmallCas9 fusion protein (*e.g.* methyltransferase activity, acetyltransferase activity, kinase activity, ubiquitinating activity, *etc.*)*.* A heterologous nucleic acid may be linked to a naturally-occurring nucleic acid (or a variant thereof) (*e.g*. by genetic engineering) to generate a fusion polynucleotide encoding a fusion polypeptide. As another example, in a fusion variant M-SmallCas9 polypeptide, a variant M-SmallCas9 polypeptide may be fused to a heterologous polypeptide (*e.g.,* a polypeptide other than M-SmallCas9), which exhibits an activity that will also be exhibited by the fusion variant M-SmallCas9 polypeptide. A heterologous nucleic acid may be linked to a variant M-SmallCas9 polypeptide (*e.g.* by genetic engineering) to generate a polynucleotide encoding a fusion variant M-SmallCas9 polypeptide. "Heterologous," as used herein, additionally means a nucleotide or polypeptide in a cell that is not its native cell.

The term "cognate" refers to two biomolecules that normally interact or co-exist in nature.

"Recombinant," as used herein, means that a particular nucleic acid (DNA or RNA) or vector is the product of various combinations of cloning, restriction, polymerase chain reaction (PCR) and/or ligation steps resulting in a construct having a structural coding or non-coding sequence distinguishable from endogenous nucleic acids found in natural systems. DNA sequences encoding polypeptides can be assembled from cDNA fragments or from a series of synthetic oligonucleotides, to provide a synthetic nucleic acid which is capable of being expressed from a recombinant transcriptional unit contained in a cell or in a cell-free transcription and translation system. Genomic DNA comprising the relevant sequences can also be used in the formation of a recombinant gene or transcriptional unit. Sequences of non-translated DNA may be present 5' or 3' from the open reading frame, where such sequences do not interfere with manipulation or expression of the coding regions, and may indeed act to modulate production of a desired product by various mechanisms (see "DNA regulatory sequences", below). In addition or alternatively, DNA sequences encoding RNA (*e.g.* guide RNA) that is not translated may also be considered recombinant. Thus, *e.g*. the term "recombinant" nucleic acid refers to one which is not naturally occurring, *e.g.* is made by the artificial combination of two otherwise separated segments of sequence through human intervention. This artificial combination is often accomplished by either chemical synthesis means, or by the artificial manipulation of isolated segments of nucleic acids, *e.g.* by genetic engineering techniques. Such is generally done to replace a codon with a codon encoding the same amino acid, a conservative amino acid, or a non-conservative amino acid. In addition or alternatively, it is performed to join together nuclei acid segments of desired functions to generate a desired combination of functions. This artificial combination is often accomplished by either chemical synthesis means, or by the artificial manipulation of isolated segments of nucleic acids, *e.g.* by genetic engineering techniques. When a recombinant polynucleotide encodes a polypeptide, the sequence of the encoded polypeptide can be naturally occurring ("wild type") or can be a variant (*e.g*. a mutant) of the naturally occurring sequence. Thus, the term "recombinant" polypeptide does not necessarily refer to a polypeptide whose sequence does not naturally occur. Instead, a "recombinant" polypeptide is encoded by a recombinant DNA sequence, but the sequence of the polypeptide can be naturally occurring ("wild type") or non-naturally occurring (*e.g.* a variant, a mutant, *etc.*)*.* Thus, a "recombinant" polypeptide is the result of human intervention, but may be a naturally occurring amino acid sequence. The term "non-naturally occurring" includes molecules that are markedly different from their naturally occurring counterparts, including chemically modified or mutated molecules.

A "vector" or "expression vector" is a replicon, such as plasmid, phage, virus, or cosmid, to which another DNA segment, *e.g*., an "insert", may be attached so as to bring about the replication of the attached segment in a cell.

An "expression cassette" includes a DNA coding sequence operably linked to a promoter. "Operably linked" refers to a juxtaposition wherein the components so described are in a relationship permitting them to function in their intended manner. For instance, a promoter is operably linked to a coding sequence if the promoter affects its transcription or expression. The terms "recombinant expression vector," or "DNA construct" are used interchangeably herein to refer to a DNA molecule comprising a vector and at least one insert. Recombinant expression vectors are generally generated for the purpose of expressing and/or propagating the insert(s), or for the construction of other recombinant nucleotide sequences. The nucleic acid(s) may or may not be operably linked to a promoter sequence and may or may not be operably linked to DNA regulatory sequences.

The term "operably linked", as used herein, denotes a physical or functional linkage between two or more elements, e.g., polypeptide sequences or polynucleotide sequences, which permits them to operate in their intended fashion. For example, an operably linkage between a polynucleotide of interest and a regulatory sequence (for example, a promoter) is functional link that allows for expression of the polynucleotide of interest. In this sense, the term "operably linked" refers to the positioning of a regulatory region and a coding sequence to be transcribed so that the regulatory region is effective for regulating transcription or translation of the coding sequence of interest. Disclosed herein, the term "operably linked" denotes a configuration in which a regulatory sequence is placed at an appropriate position relative to a sequence that encodes a polypeptide or functional RNA such that the control sequence directs or regulates the expression or cellular localization of the mRNA encoding the polypeptide, the polypeptide, and/or the functional RNA. Thus, a promoter is in operable linkage with a nucleic acid sequence if it can mediate transcription of the nucleic acid sequence. Operably linked elements may be contiguous or non-contiguous.

A cell has been "genetically modified" or "transformed" or "transfected" by exogenous DNA, *e.g.* a recombinant expression vector, when such DNA has been introduced inside the cell. The presence of the exogenous DNA results in permanent or transient genetic change. The transforming DNA may or may not be integrated (covalently linked) into the genome of the cell.

In prokaryotes, yeast, and mammalian cells for example, the transforming DNA may be maintained on an episomal element such as a plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones that include a population of daughter cells containing the transforming DNA. A "clone" is a population of cells derived from a single cell or common ancestor by mitosis. A "cell line" is a clone of a primary cell that is capable of stable growth *in vitro* for many generations.

Suitable methods of genetic modification (also referred to as "transformation") include but are not limited to, *e.g*. viral or bacteriophage infection, transfection, conjugation, protoplast fusion, lipofection, electroporation, calcium phosphate precipitation, polyethyleneimine (PEI)-mediated transfection, DEAE-dextran mediated transfection, liposome-mediated transfection, particle gun technology, calcium phosphate precipitation, direct micro injection, nanoparticle-mediated nucleic acid delivery (see, *e.g.,* Panyam et al., Adv Drug Deliv Rev. 2012 Sep 13. pp: S0169-409X(12)00283-9. doi:10.1016/j.addr.2012.09.023 ), and the like.

A "host cell," as used herein, denotes an *in vivo* or *in vitro* eukaryotic cell, a prokaryotic cell (*e.g.* bacterial or archaeal cell), or a cell from a multicellular organism (*e.g.* a cell line) cultured as a unicellular entity, which eukaryotic or prokaryotic cells can be, or have been, used as recipients for a nucleic acid, and include the progeny of the original cell which has been transformed by the nucleic acid. It is understood that the progeny of a single cell may not necessarily be completely identical in morphology or in genomic or total DNA complement as the original parent, due to natural, accidental, or deliberate mutation. A "recombinant host cell" (also referred to as a "genetically modified host cell") is a host cell into which has been introduced a heterologous nucleic acid, *e.g*. an expression vector. For example, a bacterial host cell is a genetically modified bacterial host cell by virtue of introduction into a suitable bacterial host cell of an exogenous nucleic acid (*e.g.* a plasmid or recombinant expression vector) and a eukaryotic host cell is a genetically modified eukaryotic host cell (*e.g.* a mammalian germ cell), by virtue of introduction into a suitable eukaryotic host cell of an exogenous nucleic acid.

A "target DNA" as used herein is a polydeoxyribonucleotide that includes a "target site" or "target sequence." The terms "target site," "target sequence," "target protospacer DNA, " or "protospacer-like sequence" are used interchangeably herein to refer to a nucleic acid sequence present in a target DNA to which a DNA-targeting segment (also referred to as a "spacer") of a guide RNA will bind, provided sufficient conditions for binding exist. For example, the target site (or target sequence) 5'- GAGCATATC-3' within a target DNA is targeted by (or is bound by, or hybridizes with, or is complementary to) the RNA sequence 5'-GAUAUGCUC-3'. Suitable DNA/RNA binding conditions include physiological conditions normally present in a cell. Other suitable DNA/RNA binding conditions *(e.g.* conditions in a cell-free system) are known in the art; see, *e.g.* Sambrook, supra. The strand of the target DNA that is complementary to and hybridizes with the guide RNA is referred to as the "complementary strand" and the strand of the target DNA that is complementary to the "complementary strand" (and is therefore not complementary to the guide RNA) is referred to as the "non-complementary strand" or "non-complementary strand."

By "site-specific modifying enzyme" or "RNA-binding site-specific modifying enzyme" is meant a polypeptide that binds RNA and is targeted to a specific DNA sequence, such as a M-SmallCas9 polypeptide. A site-specific modifying enzyme as described herein is targeted to a specific DNA sequence by the RNA molecule to which it is bound. The RNA molecule includes a sequence that binds, hybridizes to, or is complementary to a target sequence within the target DNA, thus targeting the bound polypeptide to a specific location within the target DNA (the target sequence). By "cleavage" it is meant the breakage of the covalent backbone of a DNA molecule. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. DNA cleavage can result in the production of either blunt ends or staggered ends. A complex comprising a guide RNA and a site-specific modifying enzyme may be used for targeted double-stranded DNA cleavage.

"Nuclease" and "endonuclease" are used interchangeably herein to mean an enzyme which possesses endonucleolytic catalytic activity for polynucleotide cleavage.

By "cleavage domain" or "active domain" or "nuclease domain" of a nuclease it is meant the polypeptide sequence or domain within the nuclease which possesses the catalytic activity for DNA cleavage. A cleavage domain can be contained in a single polypeptide chain or cleavage activity can result from the association of two (or more) polypeptides. A single nuclease domain may consist of more than one isolated stretch of amino acids within a given polypeptide.

The "guide sequence" or "DNA-targeting segment" or "DNA-targeting sequence" or "spacer" includes a nucleotide sequence that is complementary to a specific sequence within a target DNA (the complementary strand of the target DNA) designated the "protospacer-like" sequence herein. The protein-binding segment (or "protein-binding sequence") interacts with a site-specific modifying enzyme. When the site-specific modifying enzyme is a M-SmallCas9 or M-SmallCas9-related polypeptide (described in more detail below), site-specific cleavage of the target DNA occurs at locations determined by both (i) base pairing complementarity between the guide RNA and the target DNA; and (ii) a short motif (referred to as the protospacer adjacent motif (PAM)) in the target DNA. The protein-binding segment of a guide RNA includes, in part, two complementary stretches of nucleotides that hybridize to one another to form a double stranded RNA duplex (dsRNA duplex). A nucleic acid (*e.g.* a guide RNA, a nucleic acid comprising a nucleotide sequence encoding a guide RNA; a nucleic acid encoding a site-specific modifying enzyme; *etc.*) may include a modification or sequence that provides for an additional desirable feature (*e.g*. modified or regulated stability; subcellular targeting; tracking, *e.g.* a fluorescent label; a binding site for a protein or protein complex; *etc.).* Non-limiting examples include: a 5' cap (*e.g.* a 7-methylguanylate cap (m7G)); a 3' polyadenylated tail (*e.g.,* a 3' poly(A) tail); a riboswitch sequence (*e.g.* to allow for regulated stability and/or regulated accessibility by proteins and/or protein complexes); a stability control sequence; a sequence that forms a dsRNA duplex (*e.g.,* a hairpin)); a modification or sequence that targets the RNA to a subcellular location (*e.g*. nucleus, mitochondria, chloroplasts, and the like); a modification or sequence that provides for tracking (*e.g*. direct conjugation to a fluorescent molecule, conjugation to a moiety that facilitates fluorescent detection, a sequence that allows for fluorescent detection, *etc.*); a modification or sequence that provides a binding site for proteins (*e.g*. proteins that act on DNA, including transcriptional activators, transcriptional repressors, DNA methyltransferases, DNA demethylases, histone acetyltransferases, histone deacetylases, and the like); and combinations thereof.

A guide RNA may include an additional segment at either the 5' or 3' end that provides for any of the features described above. For example, a suitable third segment can include a 5' cap (*e.g.* a 7-methylguanylate cap (m7G)); a 3' polyadenylated tail (*e.g.,* a 3' poly(A) tail); a riboswitch sequence (*e.g.* to allow for regulated stability and/or regulated accessibility by proteins and protein complexes); a stability control sequence; a sequence that forms a dsRNA duplex (*e.g.,* a hairpin)); a sequence that targets the RNA to a subcellular location (*e.g*. nucleus, mitochondria, chloroplasts, and the like); a modification or sequence that provides for tracking (*e.g*. direct conjugation to a fluorescent molecule, conjugation to a moiety that facilitates fluorescent detection, a sequence that allows for fluorescent detection, *etc.*); a modification or sequence that provides a binding site for proteins (*e.g.* proteins that act on DNA. including transcriptional activators, transcriptional repressors, DNA methyltransferases, DNA demethylases, histone acetyltransferases, histone deacetylases, and the like); and combinations thereof.

A guide RNA and a site-specific modifying enzyme such as a M-SmallCas9 polypeptide or variant thereof may form a ribonucleoprotein complex (*e.g*., bind via non-covalent interactions). The guide RNA provides target specificity to the complex by comprising a nucleotide sequence that is complementary to a sequence of a target DNA. The site-specific modifying enzyme of the complex provides the endonuclease activity. In other words, the site-specific modifying enzyme is guided to a target DNA sequence (*e.g*. a target sequence in a chromosomal nucleic acid; a target sequence in an extrachromosomal nucleic acid, *e.g.* an episomal nucleic acid, a minicircle, *etc.;* a target sequence in a mitochondrial nucleic acid; a target sequence in a chloroplast nucleic acid; a target sequence in a plasmid; *etc.*) by virtue of its association with the protein-binding segment of the guide RNA. RNA aptamers are known in the art and are generally a synthetic version of a riboswitch. The terms "RNA aptamer" and "riboswitch" are used interchangeably herein to encompass both synthetic and natural nucleic acid sequences that provide for inducible regulation of the structure (and therefore the availability of specific sequences) of the RNA molecule of which they are part. RNA aptamers generally include a sequence that folds into a particular structure (*e.g*. a hairpin), which specifically binds a particular drug (*e.g.* a small molecule). Binding of the drug causes a structural change in the folding of the RNA, which changes a feature of the nucleic acid of which the aptamer is a part. As non-limiting examples: (i) an activator-RNA with an aptamer may not be able to bind to the cognate targeter RNA unless the aptamer is bound by the appropriate drug; (ii) a targeter-RNA with an aptamer may not be able to bind to the cognate activator-RNA unless the aptamer is bound by the appropriate drug; and (iii) a targeter-RNA and an activator-RNA, each comprising a different aptamer that binds a different drug, may not be able to bind to each other unless both drugs are present. As illustrated by these examples, a two-molecule guide RNA can be designed to be inducible.

Examples of aptamers and riboswitches can be found, for example, in: Nakamura et al., Genes Cells. 2012 May;17(5):344-64; Vavalle et al., Future Cardiol. 2012 May;8(3):371-82; Citartan et al., Biosens Bioelectron. 2012 Apr 15;34(1):1-11; and Liberman et al., Wiley Interdiscip Rev RNA. 2012 May-Jun;3(3):369-84.

The choice of method of genetic modification is generally dependent on the type of cell being transformed and the circumstances under which the transformation is taking place (*e.g. in vitro,* ex vivo, or *in vivo*)*.* A general discussion of these methods can be found in Ausubel, et al., Short Protocols in Molecular Biology, 3rd ed., Wiley & Sons, 1995.

Examples of aptamers and riboswitches can be found, for example, in: Nakamura et al., Genes Cells. 2012 May;17(5):344-64; Vavalle et al., Future Cardiol. 2012 May;8(3):371-82; Citartan et al., Biosens Bioelectron. 2012 Apr 15;34(1):1-11; and Liberman et al., Wiley Interdiscip Rev RNA. 2012 May-Jun;3(3):369-84.

The term "stem cell" is used herein to refer to a cell (*e.g*. plant stem cell, vertebrate stem cell) that has the ability both to self-renew and to generate a differentiated cell type (see Morrison et al. (1997) Cell 88:287-298). In the context of cell ontogeny, the adjective "differentiated", or "differentiating" is a relative term. A "differentiated cell" is a cell that has progressed further down the developmental pathway than the cell it is being compared with. Thus, pluripotent stem cells (described below) can differentiate into lineage-restricted progenitor cells (*e.g*. mesodermal stem cells), which in turn can differentiate into cells that are further restricted (*e.g*. neuron progenitors), which can differentiate into end-stage cells (*e.g.,* terminally differentiated cells, *e.g.* neurons. cardiomyocytes, *etc.*)*,* which play a characteristic role in a certain tissue type, and may or may not retain the capacity to proliferate further. Stem cells may be characterized by both the presence of specific markers (*e.g.* proteins, RNAs, *etc*.) and the absence of specific markers. Stem cells may also be identified by functional assays both *in vitro* and *in vivo,* particularly assays relating to the ability of stem cells to give rise to multiple differentiated progeny.

Stem cells of interest include pluripotent stem cells (PSCs). The term "pluripotent stem cell" or "PSC" is used herein to mean a stem cell capable of producing all cell types of the organism. Therefore, a PSC can give rise to cells of all germ layers of the organism (*e.g.* the endoderm, mesoderm, and ectoderm of a vertebrate). Pluripotent cells are capable of forming teratomas and of contributing to ectoderm, mesoderm, or endoderm tissues in a living organism. Pluripotent stem cells of plants are capable of giving rise to all cell types of the plant (*e.g.* cells of the root, stem, leaves, *etc.*)*.*

PSCs of animals can be derived in a number of different ways. For example, disclosed herein for reference only and not as part of the present invention, embryonic stem cells (ESCs) are derived from the inner cell mass of an embryo (Thomson et. al, Science. 1998 Nov 6;282(5391):1145-7). Moreover, induced pluripotent stem cells (iPSCs) are derived from somatic cells (Takahashi et. al, Cell. 2007 Nov 30;131(5):861-72; Takahashi et. al, Nat Protoc. 2007;2(12):3081-9; Yu et. al, Science. 2007 Dec 21;318(5858):1917-20. Epub 2007 Nov 20).

Because the term PSC refers to pluripotent stem cells regardless of their derivation, the term PSC encompasses the terms ESC and iPSC, as well as the term embryonic germ stem cells (EGSC), which are another example of a PSC. PSCs may be in the form of an established cell line, they may be obtained directly from primary embryonic tissue, or they may be derived from a somatic cell. PSCs can be target cells of the methods described herein.

By "embryonic stem cell" (ESC) is meant a PSC that was isolated from an embryo, generally from the inner cell mass of the blastocyst. ESC lines are listed in the NIH Human Embryonic Stem Cell Registry, *e.g*., as disclosed herein for reference only, hESBGN-01, hESBGN-02, hESBGN-03, hESBGN-04 (BresaGen, Inc.); HES-1, HES-2, HES-3, HES-4, HES-5, HES-6 (ES Cell International); Miz-hES1 (MizMedi Hospital-Seoul National University); HSF-1, HSF-6 (University of California at San Francisco); and H1, H7, H9, H13, H14 (Wisconsin Alumni Research Foundation (WiCell Research Institute)). Stem cells of interest also include embryonic stem cells from other primates, such as Rhesus stem cells and marmoset stem cells. The stem cells may be obtained from any mammalian species, *e.g*. human, equine, bovine, porcine, canine, feline, rodent, *e.g.* mice, rats. hamster, primate, *etc.* (Thomson et al. (1998) Science 282:1145; Thomson et al. (1995) Proc. Natl. Acad. Sci. USA 92:7844; Thomson et al. (1996) Biol. Reprod. 55:254; Shamblott et al., Proc. Natl. Acad. Sci. USA 95:13726, 1998). In culture, ESCs generally grow as flat colonies with large nucleo-cytoplasmic ratios, defined borders and prominent nucleoli. In addition, ESCs express SSEA-3, SSEA-4, TRA-1-60, TRA-1-81, and Alkaline Phosphatase, but not SSEA-1. Examples of methods of generating and characterizing ESCs may be found in, for example, US Patent No. 7,029,913, US Patent No. 5,843,780, and US Patent No. 6,200,806. Methods for proliferating hESCs in the undifferentiated form are described in WO 99/20741, WO 01/51616, and WO 03/020920. By "embryonic germ stem cell" (EGSC) or "embryonic germ cell" or "EG cell" is meant a PSC that is derived from germ cells and/or germ cell progenitors, *e.g.* primordial germ cells, *e.g*., those that would become sperm and eggs. Embryonic germ cells (EG cells) are thought to have properties similar to embryonic stem cells as described above. Examples of methods of generating and characterizing EG cells may be found in, for example, US Patent No. 7,153,684; Matsui, Y., et al., (1992) Cell 70:841; Shamblott, M., et al. (2001) Proc. Natl. Acad. Sci. USA 98: 113; Shamblott, M., et al. (1998) Proc. Natl. Acad. Sci. USA, 95:13726; and Koshimizu, U., et al. (1996) Development, 122:1235.

By "induced pluripotent stem cell" or "iPSC" it is meant a PSC that is derived from a cell that is not a PSC (*e.g.,* from a cell this is differentiated relative to a PSC). iPSCs can be derived from multiple different cell types, including terminally differentiated cells. iPSCs have an ES cell-like morphology, growing as flat colonies with large nucleo-cytoplasmic ratios, defined borders and prominent nuclei. In addition, iPSCs express one or more key pluripotency markers known by one of ordinary skill in the art, including but not limited to Alkaline Phosphatase, SSEA3, SSEA4, Sox2, Oct3/4, Nanog, TRA160, TRA181, TDGF 1, Dnmt3b, Fox03, GDF3, Cyp26al, TERT, and zfp42.

Examples of methods of generating and characterizing iPSCs may be found in, for example, US Patent Publication Nos. US20090047263, US20090068742, US20090191159, US20090227032, US20090246875, and US20090304646. Generally, to generate iPSCs, somatic cells are provided with reprogramming factors (*e.g.* Oct4, SOX2, KLF4, MYC, Nanog, Lin28, *etc.*) known in the art to reprogram the somatic cells to become pluripotent stem cells.

By "somatic cell" it is meant any cell in an organism that, in the absence of experimental manipulation, does not ordinarily give rise to all types of cells in an organism. In other words, somatic cells are cells that have differentiated sufficiently that they will not naturally generate cells of all three germ layers of the body, *e.g.,* ectoderm, mesoderm and endoderm. For example, somatic cells would include both neurons and neural progenitors, the latter of which may be able to naturally give rise to all or some cell types of the central nervous system but cannot give rise to cells of the mesoderm or endoderm lineages.

By "mitotic cell" it is meant a cell undergoing mitosis.

By "post-mitotic cell" it is meant a cell that has exited from mitosis, *e.g.,* it is "quiescent", *e.g.,* it is no longer undergoing divisions. This quiescent state may be temporary, *e.g.,* reversible, or it may be permanent.

By "meiotic cell" it is meant a cell that is undergoing meiosis.

By "recombination" it is meant a process of exchange of genetic information between two polynucleotides. As used herein, "homology-directed repair (HDR)" refers to the specialized form DNA repair that takes place, for example, during repair of double-strand breaks in cells. This process requires nucleotide sequence homology, uses a "donor" molecule to template repair of a "target" molecule (*e.g.,* the one that experienced the double-strand break), and leads to the transfer of genetic information from the donor to the target. Homology-directed repair may result in an alteration of the sequence of the target molecule (*e.g.* insertion, deletion, mutation), if the donor polynucleotide differs from the target molecule and part or all of the sequence of the donor polynucleotide is incorporated into the target DNA. The donor polynucleotide, a portion of the donor polynucleotide, a copy of the donor polynucleotide, or a portion of a copy of the donor polynucleotide may integrate into the target DNA.

By "non-homologous end joining (NHEJ) it is meant the repair of double-strand breaks in DNA by direct ligation of the break ends to one another without the need for a homologous template (in contrast to homology-directed repair, which requires a homologous sequence to guide repair). NHEJ often results in the loss (deletion) of nucleotide sequence near the site of the double-strand break.

The terms "treatment", "treating" and the like are used herein to generally mean obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment" as used herein covers any treatment of a disease or symptom in a mammal, and includes: (a) preventing the disease or symptom from occurring in a subject which may be predisposed to acquiring the disease or symptom but has not yet been diagnosed as having it; (b) inhibiting the disease or symptom, *e.g.,* arresting its development; or (c) relieving the disease, *e.g.,* causing regression of the disease. The therapeutic agent may be administered before, during or after the onset of disease or injury. The treatment of ongoing disease, where the treatment stabilizes or reduces the undesirable clinical symptoms of the subject, is of particular interest. Such treatment is desirably performed prior to complete loss of function in the affected tissues. The therapy will desirably be administered during the symptomatic stage of the disease, and in some cases after the symptomatic stage of the disease.

The terms "individual," "subject," "host," and "patient," are used interchangeably herein and refer to any mammalian subject for whom diagnosis, treatment, or therapy is desired, particularly humans.

General methods in molecular and cellular biochemistry can be found in such standard textbooks as Molecular Cloning: A Laboratory Manual, 3rd Ed. (Sambrook et al., Harbor Laboratory Press 2001); Short Protocols in Molecular Biology, 4th Ed. (Ausubel et al. eds., John Wiley & Sons 1999); Protein Methods (Bollag et al., John Wiley & Sons 1996); Nonviral Vectors for Gene Therapy (Wagner et al. eds., Academic Press 1999); Viral Vectors (Kaplift & Loewy eds., Academic Press 1995); Immunology Methods Manual (1. Lefkovits ed., Academic Press 1997); and Cell and Tissue Culture: Laboratory Procedures in Biotechnology (Doyle & Griffiths, John Wiley & Sons 1998).

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges, and are also encompassed within the disclosure, subject to any specifically excluded limit in the stated range. Where the stated range includes one or both of the limits, ranges excluding either or both of those included limits are also included in the disclosure.

The phrase "consisting essentially of' is meant herein to exclude anything that is not the specified active component or components of a system, or that is not the specified active portion or portions of a molecule.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

### M-SmallCas9 Fusion Polypeptides

A M-SmallCas9 can be used to form a fusion protein having additional domains and activities compared to the M-SmallCas9 nuclease. By way of non-limiting illustration, a Fokl domain can be fused to a M-SmallCas9 polypeptide or variant thereof, which can contain a catalytically active endonuclease domain, or a Fokl domain can be fused to a M-SmallCas9 polypeptide or variant thereof, which has been modified to render the M-SmallCas9 endonuclease domain inactive. Other domains that can be fused to make fusion proteins with M-SmallCas9 include transcriptional modulators, epigenetic modifiers, tags and other labels or imaging agents, histones, and/or other modalities known in the art that modulate or modify the structure or activity of gene sequences.

A M-SmallCas9 polypeptide or variant thereof described herein may be fused to a transcriptional activator or repressor, or epigenetic modifier such as a methylase, demethylase, acetylase, or deacetylase.

A M-SmallCas9 polypeptide or variant thereof described herein may be fused to functional protein components for detection, inter-molecular interaction, translational activation, modification, or any other manipulation known in the art.

### Exemplary M-SmallCas9 Variant Polypeptides

A M-SmallCas9 polypeptide or variant thereof described herein may retain a) the capability of binding to a targeted site and, optionally, b) may retain its activity. The activity being retained is endonuclease activity. The endonuclease activity may not require tracrRNA.

The activity portion of the M-SmallCas9 polypeptide or variant thereof may be modified. The modification may comprise an amino acid change (*e.g.* deletion, insertion, or substitution) that reduces or increases the nuclease activity of the M-SmallCas9 polypeptide or variant thereof. For example, the modified M-SmallCas9 polypeptide or variant thereof may have less than 50%, less than 40%, less than 30%, less than 20%, less than 10%, less than 5%, or less than 1% of the nuclease activity of the corresponding unmodified M-SmallCas9 polypeptide or variant thereof. The modified M-SmallCas9 polypeptide or variant thereof may have no substantial nuclease activity. It may have 50%, 2-fold, 4-fold or up to over 10-fold more nuclease activity.

The activity portion of the M-SmallCas9 polypeptide or variant thereof may comprise a heterologous polypeptide that has DNA-modifying activity and/or transcription factor activity and/or DNA-associated polypeptide-modifying activity. A heterologous polypeptide may replace a portion of the M-SmallCas9 polypeptide or variant thereof that provides nuclease activity. The M-SmallCas9 polypeptide or variant thereof may comprise both a portion of the M-SmallCas9 polypeptide or variant thereof that normally provides nuclease activity (and that portion can be fully active or can instead be modified to have less than 100% of the corresponding unmodified activity) and a heterologous polypeptide. In other words, A M-SmallCas9 polypeptide or variant thereof can be a fusion polypeptide comprising both the portion of the M-SmallCas9 polypeptide or variant thereof that normally provides nuclease activity and the heterologous polypeptide.

For example, in a M-SmallCas9 fusion protein, a M-SmallCas9 polypeptide or variant thereof may be fused to a heterologous polypeptide sequence (*e.g.,* a polypeptide sequence from a protein other than M-SmallCas9). The heterologous polypeptide sequence may exhibit an activity (*e.g.* enzymatic activity) that will also be exhibited by the M-SmallCas9 fusion protein (*e.g.* methyltransferase activity, acetyltransferase activity, kinase activity, ubiquitinating activity, *etc.*). A heterologous nucleic acid sequence may be linked to another nucleic acid sequence (*e.g.* by genetic engineering) to generate a fusion nucleotide sequence encoding a fusion polypeptide. A M-SmallCas9 fusion polypeptide may be generated by fusing a M-SmallCas9 polypeptide or variant thereof with a heterologous sequence that provides for subcellular localization (*e.g.* a nuclear localization signal (NLS) for targeting to the nucleus; a mitochondrial localization signal for targeting to the mitochondria; a chloroplast localization signal for targeting to a chloroplast: an ER retention signal; and the like). The heterologous sequence can provide a tag for ease of tracking or purification (*e.g.* a fluorescent protein, *e.g.* green fluorescent protein (GFP), YFP, RFP, CFP, mCherry, tdTomato, and the like; a HIS tag, *e.g.* a 6XHis tag; a hemagglutinin (HA) tag; a FLAG tag; a Myc tag; and the like). The heterologous sequence can provide for increased or decreased stability. The heterologous sequence can provide a binding domain (*e.g.* to provide the ability of a M-SmallCas9 fusion polypeptide to bind to another protein of interest, *e.g.* a DNA or histone modifying protein, a transcription factor or transcription repressor, a recruiting protein, *etc.*) or to a nucleotide of interest (*e.g.,* an aptamer or target site of a nucleotide binding protein).

The M-SmallCas9 polypeptide variants described herein, may have reduced endodeoxyribonuclease activity. For example, a M-SmallCas9 polypeptide variant suitable for use in a transcription modulation method of the present disclosure exhibits less than about 20%, less than about 15%, less than about 10%, less than about 5%, less than about 1%, or less than about 0.1%, of the endodeoxyribonuclease activity of an unmodified M-SmallCas9 polypeptide.

The variant M-SmallCas9 polypeptide has substantially no detectable endodeoxyribonuclease activity (dM-SmallCas9). When a M-SmallCas9 polypeptide variant has reduced catalytic activity, the polypeptide can still bind to target DNA in a site-specific manner (because it is still guided to a target DNA sequence by a guide RNA) as long as it retains the ability to interact with the guide RNA. The variant M-SmallCas9 polypeptide may be a nickase that can cleave the complementary strand of the target DNA but has reduced ability to cleave the non-complementary strand of the target DNA.

The variant M-SmallCas9 polypeptide may be a nickase that can cleave the non-complementary strand of the target DNA but has reduced ability to cleave the complementary strand of the target DNA.

The variant M-SmallCas9 polypeptide may have a reduced ability to cleave both the complementary and the non-complementary strands of the target DNA. For example, alanine substitutions are contemplated.

The variant M-SmallCas9 polypeptide may be a fusion polypeptide (a "variant M-SmallCas9 fusion polypeptide"), *e.g.,* a fusion polypeptide comprising: i) a variant M-SmallCas9 polypeptide; and ii) a covalently linked heterologous polypeptide (also referred to as a "fusion partner").

The heterologous polypeptide may exhibit an activity (*e.g.* enzymatic activity) that will also be exhibited by the variant M-SmallCas9 fusion polypeptide (*e.g.* methyltransferase activity, acetyltransferase activity, kinase activity, ubiquitinating activity, *etc.).* A heterologous nucleic acid sequence may be linked to another nucleic acid sequence (*e.g.* by genetic engineering) to generate a fusion nucleotide sequence encoding a fusion polypeptide. A variant M-SmallCas9 fusion polypeptide may be generated by fusing a variant M-SmallCas9 polypeptide with a heterologous sequence that provides for subcellular localization (*e.g.,* the heterologous sequence is a subcellular localization sequence, *e.g.* a nuclear localization signal (NLS) for targeting to the nucleus; a mitochondrial localization signal for targeting to the mitochondria; a chloroplast localization signal for targeting to a chloroplast; an ER retention signal; and the like). The heterologous sequence can provide a tag (*e.g.,* the heterologous sequence is a detectable label) for ease of tracking and/or purification (*e.g*. a fluorescent protein, *e.g*. green fluorescent protein (GFP), YFP, RFP, CFP, mCherry, tdTomato, and the like; a histidine tag, *e.g.* a 6XHis tag; a hemagglutinin (HA) tag; a FLAG tag; a Myc tag; and the like). The heterologous sequence can provide for increased or decreased stability (*e.g*., the heterologous sequence is a stability control peptide, *e.g.* a degron, which in some cases is controllable (*e.g. a* temperature sensitive or drug controllable degron sequence, see below). The heterologous sequence can provide for increased or decreased transcription from the target DNA (*e.g*., the heterologous sequence is a transcription modulation sequence, *e.g.* a transcription factor/activator or a fragment thereof, a protein or fragment thereof that recruits a transcription factor/activator, a transcription repressor or a fragment thereof, a protein or fragment thereof that recruits a transcription repressor, a small molecule/drug-responsive transcription regulator, *etc.*)*.* The heterologous sequence can provide a binding domain (*e.g.,* the heterologous sequence is a protein binding sequence, *e.g.* to provide the ability of a fusion dM-SmallCas9 polypeptide to bind to another protein of interest, *e.g.* a DNA or histone modifying protein, a transcription factor or transcription repressor, a recruiting protein, *etc.*)*.*

Suitable fusion partners that provide for increased or decreased stability include, but are not limited to degron sequences. Degrons are readily understood by one of ordinary skill in the art to be amino acid sequences that control the stability of the protein of which they are part. For example, the stability of a protein comprising a degron sequence is controlled at least in part by the degron sequence. A suitable degron may be constitutive such that the degron exerts its influence on protein stability independent of experimental control (*e.g.,* the degron is not drug inducible, temperature inducible, *etc.*)*.* The degron may provide the variant M-SmallCas9 polypeptide with controllable stability such that the variant M-SmallCas9 polypeptide can be turned "on" (*e.g.,* stable) or "off" (*e.g.,* unstable, degraded) depending on the desired conditions. For example, if the degron is a temperature sensitive degron, the variant M-SmallCas9 polypeptide may be functional (*e.g.*, "on", stable) below a threshold temperature (*e.g*. 42 °C, 41 °C, 40 °C, 39 °C, 38 °C, 37 °C, 36 °C, 35 °C, 34 °C, 33 °C, 32 °C, 31 °C, 30 °C, *etc.)* but non-functional (*e.g.,* "off", degraded) above the threshold temperature. As another example, if the degron is a drug inducible degron, the presence or absence of drug can switch the protein from an "off" (*e.g.,* unstable) state to an "on" (*e.g.,* stable) state or vice versa. An exemplary drug inducible degron is derived from the FKBP12 protein. The stability of the degron is controlled by the presence or absence of a small molecule that binds to the degron.

Examples of suitable degrons include, but are not limited to those degrons controlled by Shield-1, DHFR, auxins, and/or temperature. Non-limiting examples of suitable degrons are known in the art (*e.g.* Dohmen et al., Science, 1994. 263(5151): p. 1273-1276: Heat-inducible degron: a method for constructing temperature-sensitive mutants; Schoeber et al., Am J Physiol Renal Physiol. 2009 Jan;296(1):F204-11 :Conditional fast expression and function of multimeric TRPV5 channels using Shield-1; Chu et al., Bioorg Med Chem Lett. 2008 Nov 15;18(22):5941-4: Recent progress with FKBP-derived destabilizing domains ; Kanemaki, Pflugers Arch. 2012 Dec 28: Frontiers of protein expression control with conditional degrons; Yang et al., Mol Cell. 2012 Nov 30;48(4):487-8: Titivated for destruction: the methyl degron; Barbour et al., Biosci Rep. 2013 Jan 18;33(1).: Characterization of the bipartite degron that regulates ubiquitin-independent degradation of thymidylate synthase; and Greussing et al., J Vis Exp. 2012 Nov 10;(69): Monitoring of ubiquitin-proteasome activity in living cells using a Degron (dgn)-destabilized green fluorescent protein (GFP)-based reporter protein.

Exemplary degron sequences have been well characterized and tested in both cells and animals. Thus, fusing M-SmallCas9 to a degron sequence produces a "tunable" and "inducible" M-SmallCas9 polypeptide. Any of the fusion partners described herein can be used in any desirable combination. As one non-limiting example to illustrate this point, a M-SmallCas9 fusion protein can comprise a YFP sequence for detection, a degron sequence for stability, and transcription activator sequence to increase transcription from the target DNA Furthermore, the number of fusion partners that can be used in a M-SmallCas9 fusion protein is unlimited. A M-SmallCas9 fusion protein may comprise one or more (*e.g.* two or more, three or more, four or more, or five or more) heterologous sequences.

Suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, crotonylation activity, decrotonylation activity, propionylation activity, depropionylationa activity, myristoylation activity, or demyristoylation activity, any of which can be directed at modifying the DNA directly (*e.g.,* methylation of DNA) or at modifying a DNA-associated polypeptide (*e.g.* a histone or DNA binding protein). Further suitable fusion partners include, but are not limited to boundary elements (*e.g.* CTCF), proteins and fragments thereof that provide periphery recruitment (*e.g.,* Lamin A, Lamin B, *etc.*)*,* and protein docking elements (*e.g.* FKBP/FRB, Pil 1/Aby 1, *etc.*)*.*

The M-SmallCas9 polypeptides or variants thereof may also be isolated and purified in accordance with conventional methods of recombinant synthesis. A lysate may be prepared of the expression host and the lysate purified using HPLC, exclusion chromatography, gel electrophoresis, affinity chromatography, or other purification technique. For the most part, the compositions which are used will comprise at least 20% by weight of the desired product, at least about 75% by weight, at least about 95% by weight, and for therapeutic purposes, typically at least 99.5% by weight, in relation to contaminants related to the method of preparation of the product and its purification. Generally, the percentages will be based upon total protein. To induce DNA cleavage and recombination, or any desired modification to a target DNA, or any desired modification to a polypeptide associated with target DNA, the guide RNA and/or the M-SmallCas9 polypeptide or variant thereof and/or the donor polynucleotide, whether they be introduced as nucleic acids or polypeptides, are provided to the cells for about 30 minutes to about 24 hours, *e.g.* 1 hour, 1.5 hours, 2 hours, 2.5 hours, 3 hours, 3.5 hours 4 hours, 5 hours, 6 hours, 7 hours, 8 hours, 12 hours, 16 hours, 18 hours, 20 hours, or any other period from about 30 minutes to about 24 hours, which may be repeated with a frequency of about every day to about every 4 days, *e.g.* every 1.5 days, every 2 days, every 3 days, or any other frequency from about every day to about every four days. The agent(s) may be provided to the cells one or more times, *e.g.* one time, twice, three times, or more than three times, and the cells allowed to incubate with the agent(s) for some amount of time following each contacting event *e.g.* 16-24 hours, after which time the media is replaced with fresh media and the cells are cultured further. In cases in which two or more different targeting complexes are provided to the cell (*e.g.* two different guide RNAs that are complementary to different sequences within the same or different target DNA), the complexes may be provided simultaneously (*e.g*. as two polypeptides and/or nucleic acids), or delivered simultaneously. Alternatively, they may be provided consecutively, *e.g.* the targeting complex being provided first, followed by the second targeting complex, *etc.* or vice versa.

### Nucleic acids

### Guide RNAs/sqRNAs

The systems, compositions, and methods described herein in some embodiments employ a genome-targeting nucleic acid that can direct the activities of an associated polypeptide (*e.g.,* a M-SmallCas9 polypeptide or variant thereof) to a specific target sequence within a target nucleic acid. In some embodiments, the genome-targeting nucleic acid is an RNA. A genome-targeting RNA is referred to as a "guide RNA" or "gRNA" herein. A guide RNA has at least a spacer sequence that can hybridize to a target nucleic acid sequence of interest and a CRISPR repeat sequence (such a CRISPR repeat sequence is also referred to as a "tracr mate sequence"). In Type II systems, the gRNA also has a second RNA called the tracrRNA sequence. In the Type II guide RNA (gRNA), the CRISPR repeat sequence and tracrRNA sequence hybridize to each other to form a duplex. In the Type V guide RNA (gRNA), the crRNA forms a duplex. In both systems, the duplex binds a site-specific polypeptide such that the guide RNA and site-direct polypeptide form a complex. The genome-targeting nucleic acid provides target specificity to the complex by virtue of its association with the site-specific polypeptide. The genome-targeting nucleic acid thus directs the activity of the site-specific polypeptide.

The genome-targeting nucleic acid may be a double-molecule guide RNA. In some embodiments, the genome-targeting nucleic acid is a single-molecule guide RNA or single guide RNA (sgRNA). A double-molecule guide RNA has two strands of RNA. The first strand has in the 5' to 3' direction, an optional spacer extension sequence, a spacer sequence and a minimum CRISPR repeat sequence. The second strand has a minimum tracrRNA sequence (complementary to the minimum CRISPR repeat sequence), a 3' tracrRNA sequence and an optional tracrRNA extension sequence. A single-molecule guide RNA (sgRNA) in a Type II system has, in the 5' to 3' direction, an optional spacer extension sequence, a spacer sequence, a minimum CRISPR repeat sequence, a single-molecule guide linker, a minimum tracrRNA sequence, a 3' tracrRNA sequence and an optional tracrRNA extension sequence. The optional tracrRNA extension may have elements that contribute additional functionality (e.g., stability) to the guide RNA. The single-molecule guide linker links the minimum CRISPR repeat and the minimum tracrRNA sequence to form a hairpin structure. The optional tracrRNA extension has one or more hairpins. A single-molecule guide RNA (sgRNA) in a Type V system has, in the 5' to 3' direction, a minimum CRISPR repeat sequence and a spacer sequence.

Exemplary genome-targeting nucleic acids are described, for example, in WO2018002719.

In general, a CRISPR repeat sequence includes any sequence that has sufficient complementarity with a tracr sequence to promote one or more of: (1) excision of a DNA targeting segment flanked by CRISPR repeat sequences in a cell containing the corresponding tracr sequence; and (2) formation of a CRISPR complex at a target sequence, wherein the CRISPR complex includes the CRISPR repeat sequence hybridized to the tracr sequence. In general, degree of complementarity is with reference to the optimal alignment of the CRISPR repeat sequence and tracr sequence, along the length of the shorter of the two sequences. Optimal alignment may be determined by any suitable alignment algorithm and may further account for secondary structures, such as self-complementarity within either the tracr sequence or CRISPR repeat sequence. The degree of complementarity between the tracr sequence and CRISPR repeat sequence along the 30 nucleotides length of the shorter of the two when optimally aligned may be about or more than 25%, 30%, 40%, 50%, 60%, 70%, 80%, 90%, 95%, 97.5%, 99%, or higher. The tracr sequence may be about or more than 5, 6, 7, 8, 9, 10, 11, 12, 13, 14, 15, 16,17, 18, 19, 20, 25, 30, 40, 50, or more nucleotides in length. The tracr sequence and CRISPR repeat sequence are contained within a single transcript, such that hybridization between the two produces a transcript having a secondary structure, such as a hairpin. The transcript or transcribed polynucleotide sequence may have at least two or more hairpins.

The spacer of a guide RNA includes a nucleotide sequence that is complementary to a sequence in a target DNA. In other words, the spacer of a guide RNA interacts with a target DNA in a sequence-specific manner via hybridization (*e.g.,* base pairing). As such, the nucleotide sequence of the spacer may vary and determines the location within the target DNA that the guide RNA and the target DNA will interact. The DNA- targeting segment of a guide RNA can be modified (*e.g*. by genetic engineering) to hybridize to any desired sequence within a target DNA.

The spacer may have a length of from 10 nucleotides to 30 nucleotides. The spacer has a length of from 13 nucleotides to 25 nucleotides. The spacer has a length of from 15 nucleotides to 23 nucleotides. The spacer has a length of from 18 nucleotides to 22 nucleotides, *e.g*., from 20 to 22 nucleotides.

The percent complementarity between the DNA-targeting sequence of the spacer and the protospacer of the target DNA is at least 60% (*e.g.* at least 65%, at least 70%, at least 75%, at least 80%, at least 85%, at least 90%, at least 95%, at least 97%, at least 98%, at least 99%, or 100%) over the 20-22 nucleotides.

The protospacer is directly adjacent to a suitable PAM sequence on its 3' end or such PAM sequence is part of the DNA targeting sequence in its 3' portion.

Modifications of guide RNAs can be used to enhance the formation or stability of the CRISPR-Cas genome editing complex comprising guide RNAs and a Cas endonuclease such as M-SmallCas9. Modifications of guide RNAs can also or alternatively be used to enhance the initiation, stability or kinetics of interactions between the genome editing complex with the target sequence in the genome, which can be used for example to enhance on-target activity. Modifications of guide RNAs can also or alternatively be used to enhance specificity, *e.g.* the relative rates of genome editing at the on-target site as compared to effects at other (off-target) sites.

Modifications can also or alternatively used to increase the stability of a guide RNA, *e.g.* by increasing its resistance to degradation by ribonucleases (RNases) present in a cell, thereby causing its half-life in the cell to be increased. Modifications enhancing guide RNA half-life can be particularly useful in embodiments in which a Cas endonuclease such as a M-SmallCas9 is introduced into the cell to be edited via an RNA that needs to be translated in order to generate M-SmallCas9 endonuclease, since increasing the half-life of guide RNAs introduced at the same time as the RNA encoding the endonuclease can be used to increase the time that the guide RNAs and the encoded Cas endonuclease co-exist in the cell.

### Donor DNA or Donor Template

Site-specific polypeptides, such as a DNA endonuclease, can introduce double-strand breaks or single-strand breaks in nucleic acids, *e.g.,* genomic DNA. The double-strand break can stimulate a cell's endogenous DNA-repair pathways (*e.g*., homology-dependent repair (HDR) or non-homologous end joining or alternative non-homologous end joining (A-NHEJ) or microhomology-mediated end joining (MMEJ). NHEJ can repair cleaved target nucleic acid without the need for a homologous template. This can sometimes result in small deletions or insertions (indels) in the target nucleic acid at the site of cleavage, and can lead to disruption or alteration of gene expression. HDR, which is also known as homologous recombination (HR) can occur when a homologous repair template, or donor, is available.

The homologous donor template has sequences that are homologous to sequences flanking the target nucleic acid cleavage site. The sister chromatid is generally used by the cell as the repair template. However, for the purposes of genome editing, the repair template is often supplied as an exogenous nucleic acid, such as a plasmid, duplex oligonucleotide, single-strand oligonucleotide, double-stranded oligonucleotide, or viral nucleic acid. With exogenous donor templates, it is common to introduce an additional nucleic acid sequence (such as a transgene) or modification (such as a single or multiple base change or a deletion) between the flanking regions of homology so that the additional or altered nucleic acid sequence also becomes incorporated into the target locus. MMEJ results in a genetic outcome that is similar to NHEJ in that small deletions and insertions can occur at the cleavage site. MMEJ makes use of homologous sequences of a few base pairs flanking the cleavage site to drive a favored end-joining DNA repair outcome. It may be possible to predict likely repair outcomes based on analysis of potential microhomologies in the nuclease target regions.

Thus, in some cases, homologous recombination is used to insert an exogenous polynucleotide sequence into the target nucleic acid cleavage site. An exogenous polynucleotide sequence is termed a donor polynucleotide (or donor or donor sequence or polynucleotide donor template) herein. The donor polynucleotide, a portion of the donor polynucleotide, a copy of the donor polynucleotide, or a portion of a copy of the donor polynucleotide may be inserted into the target nucleic acid cleavage site. The donor polynucleotide may be an exogenous polynucleotide sequence, *i.e.,* a sequence that does not naturally occur at the target nucleic acid cleavage site.

When an exogenous DNA molecule is supplied in sufficient concentration inside the nucleus of a cell in which the double strand break occurs, the exogenous DNA can be inserted at the double strand break during the NHEJ repair process and thus become a permanent addition to the genome. These exogenous DNA molecules are referred to as donor templates. If the donor template contains a coding sequence for one or more system components described herein optionally together with relevant regulatory sequences such as promoters, enhancers, polyA sequences and/ or splice acceptor sequences, the one or more system components can be expressed from the integrated nucleic acid in the genome resulting in permanent expression for the life of the cell. Moreover, the integrated nucleic acid of the donor DNA template can be transmitted to the daughter cells when the cell divides.

In the presence of sufficient concentrations of a donor DNA template that contains flanking DNA sequences with homology to the DNA sequence either side of the double strand break (referred to as homology arms), the donor DNA template can be integrated via the HDR pathway. The homology arms act as substrates for homologous recombination between the donor template and the sequences either side of the double strand break. This can result in an error free insertion of the donor template in which the sequences either side of the double strand break are not altered from that in the un-modified genome.

Supplied donors for editing by HDR vary markedly but generally contain the intended sequence with small or large flanking homology arms to allow annealing to the genomic DNA. The homology regions flanking the introduced genetic changes can be 30 bp or smaller, or as large as a multi-kilobase cassette that can contain promoters, cDNAs, etc. Both single-stranded and double-stranded oligonucleotide donors can be used. These oligonucleotides range in size from less than 100 nt to over many kb, though longer ssDNA can also be generated and used. Double-stranded donors are often used, including PCR amplicons, plasmids, and mini-circles. In general, it has been found that an AAV vector is a very effective means of delivery of a donor template, though the packaging limits for individual donors is <5kb. Active transcription of the donor increased HDR three-fold, indicating the inclusion of promoter can increase conversion. Conversely, CpG methylation of the donor can decrease gene expression and HDR.

The donor DNA may be supplied with the nuclease or independently by a variety of different methods, for example by transfection, nanoparticle, micro-injection, or viral transduction. A range of tethering options can be used to increase the availability of the donors for HDR. Examples include attaching the donor to the nuclease, attaching to DNA binding proteins that bind nearby, or attaching to proteins that are involved in DNA end binding or repair.

In addition to genome editing by NHEJ or HDR, site-specific gene insertions can be conducted that use both the NHEJ pathway and HR. A combination approach can be applicable in certain settings, possibly including intron/exon borders. NHEJ can prove effective for ligation in the intron, while the error-free HDR can be better suited in the coding region.

### Vectors

In another aspect, provided herein is a nucleic acid comprising a codon-optimized polynucleotide sequence encoding a M-SmallCas9 polypeptide or variant thereof, a gRNA, and/or any nucleic acid or proteinaceous molecule necessary to carry out the embodiments of the disclosure. Such a nucleic acid may be a vector (*e.g.,* a recombinant expression vector).

Expression vectors contemplated include, but are not limited to, viral vectors based on vaccinia virus, poliovirus, adenovirus, adeno-associated virus, SV40, herpes simplex virus, human immunodeficiency virus, retrovirus (*e.g.,* Murine Leukemia Virus, spleen necrosis virus, and vectors derived from retroviruses such as Rous Sarcoma Virus, Harvey Sarcoma Virus, avian leukosis virus, a lentivirus, human immunodeficiency virus, myeloproliferative sarcoma virus, and mammary tumor virus) and other recombinant vectors. Other vectors contemplated for eukaryotic target cells include, but are not limited to, the vectors pXT1, pSG5, pSVK3, pBPV, pMSG, and pSVLSV40 (Pharmacia). Additional vectors contemplated for eukaryotic target cells include, but are not limited to, the vectors pCTx-1, pCTx-2, and pCTx-3. Other vectors can be used so long as they are compatible with the host cell.

A vector may have one or more transcription and/or translation control elements. Depending on the host/vector system utilized, any of a number of suitable transcription and translation control elements, including constitutive and inducible promoters, transcription enhancer elements, transcription terminators, etc. can be used in the expression vector. The vector may be a self-inactivating vector that either inactivates the viral sequences or the components of the CRISPR machinery or other elements.

Non-limiting examples of suitable eukaryotic promoters (*i.e.,* promoters functional in a eukaryotic cell) include those from cytomegalovirus (CMV) immediate early, herpes simplex virus (HSV) thymidine kinase, early and late SV40, long terminal repeats (LTRs) from retrovirus, human elongation factor-1 promoter (EF1), a hybrid construct having the cytomegalovirus (CMV) enhancer fused to the chicken beta-actin promoter (CAG), murine stem cell virus promoter (MSCV), phosphoglycerate kinase-1 locus promoter (PGK), and mouse metallothionein-I.

For expressing small RNAs, including guide RNAs used in connection with Cas endonuclease, various promoters such as RNA polymerase III promoters, including for example U6 and H1, can be advantageous. Descriptions of and parameters for enhancing the use of such promoters are known in art, and additional information and approaches are regularly being described; see, *e.g.,* Ma, H. et al., Molecular Therapy - Nucleic Acids 3, e161 (2014) doi:10.1038/mtna.2014.12.

The expression vector can also contain a ribosome binding site for translation initiation and a transcription terminator. The expression vector can also include appropriate sequences for amplifying expression. The expression vector can also include nucleotide sequences encoding non-native tags (*e.g.,* histidine tag, hemagglutinin tag, green fluorescent protein, etc.) that are fused to the site-specific polypeptide, thus resulting in a fusion protein.

A promoter may be an inducible promoter (*e.g.,* a heat shock promoter, tetracycline-regulated promoter, steroid-regulated promoter, metal-regulated promoter, estrogen receptor-regulated promoter, etc.). A promoter may be a constitutive promoter (*e.g.,* CMV promoter, UBC promoter). The promoter may be a spatially restricted and/or temporally restricted promoter (*e.g.,* a tissue specific promoter, a cell type specific promoter, etc.). A vector may not have a promoter for at least one gene to be expressed in a host cell if the gene is going to be expressed, after it is inserted into a genome, under an endogenous promoter present in the genome.

### Modifications of Nucleic Acids and Polypeptides

A polynucleotide described herein may comprise one or more modifications which can be used, for example, to enhance activity, stability or specificity, alter delivery, reduce innate immune responses in host cells, further reduce the protein size, or for other enhancements, as further described herein and known in the art.

### Codon-optimization

Modified polynucleotides may be used in a CRISPR-M-SmallCas9 system described herein, in which the guide RNAs and/or a DNA or an RNA comprising a polynucleotide sequence encoding a M-SmallCas9 polypeptide or variant thereof can be modified, as described and illustrated below. Such modified polynucleotides can be used in the CRISPR-M-SmallCas9 system to edit any one or more genomic loci. Such modifications in the polynucleotides of the disclosure may be achieved via codon-optimization, e.g., codon-optimized based on specific host cells in which the encoded polypeptide is expressed. It will be appreciated by the skilled artisan that any nucleotide sequence and/or recombinant nucleic acid of the present disclosure can be codon optimized for expression in any species of interest. Codon optimization is well known in the art and involves modification of a nucleotide sequence for codon usage bias using species specific codon usage tables. The codon usage tables are generated based on a sequence analysis of the most highly expressed genes for the species of interest. In a non-limiting example, when the nucleotide sequences are to be expressed in the nucleus, the codon usage tables are generated based on a sequence analysis of highly expressed nuclear genes for the species of interest. The modifications of the nucleotide sequences are determined by comparing the species specific codon usage table with the codons present in the native polynucleotide sequences.

A M-SmallCas9 polypeptide or variant thereof described herein may be expressed from a codon-optimized polynucleotide sequence. For example, if the intended target cell were a human cell, a human codon-optimized polynucleotide sequence encoding M-SmallCas9 (or a M-SmallCas9 variant, *e.g.* enzymatically inactive variant) would be a suitable. As another non-limiting example, if the intended host cell were a mouse cell, then a mouse codon-optimized polynucleotide sequence encoding M-SmallCas9 (or M-SmallCas9 variant, *e.g.* enzymatically inactive variant) would be suitable.

Strategies and methodologies for codon optimization are known in the art and have been described for various systems including, but not limited to yeast (Outchkourov et al., Protein Expr Purif, 24(1):18-24 (2002)) and *E. coli* (Feng et al., Biochemistry, 39(50):15399-15409 (2000)). The codon optimization was performed by using GeneGPS^{®} Expression Optimization Technology (ATUM) and using the manufacturer's recommended expression optimization algorithms. The polynucleotides of the disclosure may be codon-optimized for increased expression in a human cell. The polynucleotides of the disclosure may be codon-optimized for increased expression in an *E. coli* cell. The polynucleotides of the disclosure may be codon-optimized for increased expression in an insect cell. The polynucleotides of the disclosure may be codon-optimized for increased expression in a Sf9 insect cell. The expression optimization algorithms used in codon optimization procedure may be defined to avoid putative poly-A signals (*e.g.* AATAAA and ATTAAA) as well as long (greater than 4) stretches of A's which can lead to polymerase slippage.

As is well understood in the art, codon optimization of a nucleotide sequence results in a nucleotide sequence having less than 100% identity (e.g., less than 70%, 71 %. 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%) to the native nucleotide sequence but which still encodes a polypeptide having the same function as that encoded by the original, native nucleotide sequence. Thus, the nucleotide sequence and/or recombinant nucleic acid of the disclosure may be be codon optimized for expression in the particular species of interest.

A codon-optimized polynucleotide sequence may have at least 90%, 92%, 93%, 94%, 95%, 96%, 97%, 98%, 99%, 99.2%, 99.5%, 99.8%, 99.9%, or 100% sequence identity to SEQ ID NO: 1. The polynucleotides of the disclosure may be codon-optimized for increased expression of the encoded M-SmallCas9 polypeptide in a target cell. The polynucleotides of the disclosure may be codon-optimized for increased expression in a human cell. Generally, the polynucleotides of the disclosure may be codon-optimized for increased expression in any human cells. The polynucleotides of the disclosure may be codon-optimized for increased expression in an *E. coli* cell. The polynucleotides of the disclosure may be codon-optimized for increased expression in an insect cell. Generally, the polynucleotides of the disclosure may be codon-optimized for increased expression in any insect cells. The polynucleotides of the disclosure may be codon-optimized for increased expression in a Sf9 insect cell expression system.

Polyadenylation signals can also be chosen to optimize expression in the intended host.

### Other Modifications

Modifications can also or alternatively be used to decrease the likelihood or degree to which RNAs introduced into cells elicit innate immune responses. Such responses, which have been well characterized in the context of RNA interference (RNAi), including small-interfering RNAs (siRNAs), as described below and in the art, tend to be associated with reduced half-life of the RNA and/or the elicitation of cytokines or other factors associated with immune responses.

One or more types of modifications can also be made to RNAs encoding an endonuclease such as M-SmallCas9 that are introduced into a cell, including, without limitation, modifications that enhance the stability of the RNA (such as by decreasing its degradation by RNases present in the cell), modifications that enhance translation of the resulting product (*e.g.,* the endonuclease), and/or modifications that decrease the likelihood or degree to which the RNAs introduced into cells elicit innate immune responses. Combinations of modifications, such as the foregoing and others, can likewise be used. In the case of CRISPR-M-SmallCas9, for example, one or more types of modifications can be made to guide RNAs (including those exemplified above), and/or one or more types of modifications can be made to RNAs encoding M-SmallCas9 endonuclease (including those exemplified above).

By way of illustration, guide RNAs used in the CRISPR-M-SmallCas9 system or other smaller RNAs can be readily synthesized by chemical means, enabling a number of modifications to be readily incorporated, as illustrated below and described in the art. While chemical synthetic procedures are continually expanding, purifications of such RNAs by procedures such as high performance liquid chromatography (HPLC, which avoids the use of gels such as PAGE) tends to become more challenging as polynucleotide lengths increase significantly beyond a hundred or so nucleotides. One approach used for generating chemically-modified RNAs of greater length is to produce two or more molecules that are ligated together. Much longer RNAs, such as those encoding a M-SmallCas9 endonuclease, are more readily generated enzymatically. While fewer types of modifications are generally available for use in enzymatically produced RNAs, there are still modifications that can be used to, *e.g.* enhance stability, reduced the likelihood or degree of innate immune response, and/or enhance other attributes, as described further below and in the art; and new types of modifications are regularly being developed. By way of illustration of various types of modifications, especially those used frequently with smaller chemically synthesized RNAs, modifications can include one or more nucleotides modified at the 2' position of the sugar, such as a 2'-O-alkyl, 2'-O-alkyl-O-alkyl or 2'-fluoro-modified nucleotide. RNA modifications may include 2'-fluoro, 2'-amino and 2' O-methyl modifications on the ribose of pyrimidines, basic residues or an inverted base at the 3' end of the RNA. Such modifications are routinely incorporated into oligonucleotides and these oligonucleotides have been shown to have a higher Tm (*e.g.,* higher target binding affinity) than; 2'- deoxy oligonucleotides against a given target.

A number of nucleotide and nucleoside modifications have been shown to make the oligonucleotide into which they are incorporated more resistant to nuclease digestion than the native oligonucleotide; these modified oligonucleotides survive intact for a longer time than unmodified oligonucleotides. Specific examples of modified oligonucleotides include those comprising modified backbones, for example, phosphorothioates, phosphotriesters, methyl phosphonates, short chain alkyl or cycloalkyl intersugar linkages or short chain heteroatomic or heterocyclic intersugar linkages. Some oligonucleotides are oligonucleotides with phosphorothioate backbones and those with heteroatom backbones, particularly CH2 -NH-O-CH2, CH,-N(CH3)-O-CH2 (known as a methylene(methylimino) or MMI backbone), CH2-O-N (CH3)-CH2, CH2 -N (CH3)-N (CH3)-CH2 and O-N (CH3)-CH2 -CH2 backbones; amide backbones [see De Mesmaeker et al., Ace. Chem. Res., 28:366-374 (1995)]; morpholino backbone structures (see Summerton and Weller, US Patent No. 5,034,506); peptide nucleic acid (PNA) backbone (wherein the phosphodiester backbone of the oligonucleotide is replaced with a polyamide backbone, the nucleotides being bound directly or indirectly to the aza nitrogen atoms of the polyamide backbone, see Nielsen et al., Science 1991, 254, 1497). Phosphorus-containing linkages include, but are not limited to, phosphorothioates, chiral phosphorothioates, phosphorodithioates, phosphotriesters, aminoalkylphosphotriesters, methyl and other alkyl phosphonates including 3'alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'; see US patent Nos. 3,687,808; 4,469,863; 4,476,301; 5,023,243; 5,177, 196; 5,188,897; 5,264,423; 5,276,019; 5,278,302; 5,286,717; 5,321,131; 5,399,676; 5,405,939; 5,453,496; 5,455, 233; 5,466,677; 5,476,925; 5,519,126; 5,536,821; 5,541,306; 5,550,111; 5,563, 253; 5,571,799; 5,587,361; and 5,625,050.

Morpholino-based oligomeric compounds are described in Braasch and Corey, Biochemistry, 41(14): 4503-4510 (2002); Genesis, Volume 30, Issue 3, (2001); Heasman, Dev. Biol., 243:209-214 (2002); Nasevicius et al., Nat. Genet., 26:216-220 (2000); Lacenra etc., Proc. Nat/. Acad. Sci., 97: 9591-9596 (2000); and US Patent No. 5,034,506, issued Jul. 23, 1991. Cyclohexenyl nucleic acid oligonucleotide mimetics are described in Wang et al., J. Am. Chem. Soc., 122: 8595-8602 (2000).

Modified oligonucleotide backbones that do not include a phosphorus atom therein have backbones that are formed by short chain alkyl or cycloalkyl intemucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, 0, Sand CH2 component parts; see US patent nos. 5,034,506; 5,166,315; 5,185,444; 5,214,134; 5,216,141; 5,235,033; 5,264, 562; 5, 264,564; 5,405,938; 5,434,257; 5,466,677; 5,470,967, 5,489,677; 5,541,307; 5,561,225; 5,596, 086; 5,602,240; 5,610,289; 5,602,240; 5,608,046; 5,610,289; 5,618,704; 5,623, 070; 5,663,312; 5,633,360; 5,677,437; and 5,677,439.

One or more substituted sugar moieties can also be included, *e.g.* one of the following at the 2' position: OH, SH, SCH3, F, OCN, OCH3, OCH3 O(CH2)n CH3, O(CH2)n NH2 or O(CH2)n CH3 where n is from 1 to 10; C1 to C10 lower alkyl, alkoxyalkoxy, substituted lower alkyl, alkaryl or aralkyl; Cl; Br; CN; CF3 ; OCF3; O-, S-, or N-alkyl; O-, S-, or N-alkenyl: SOCH3; SO2CH3; ONO2; NO2; N3; NH2; heterocycloalkyl; heterocycloalkaryl; aminoalkylamino; polyalkylamino; substituted silyl; an RNA cleaving group; a reporter group; an intercalator; a group for improving the pharmacokinetic properties of an oligonucleotide; or a group for improving the pharmacodynamic properties of an oligonucleotide and other substituents having similar properties. A modification may include 2'-methoxyethoxy (2'-O-CH2CH2OCH3, also known as 2'-O-(2-methoxyethyl)) (Martinet a/, Helv. Chim. Acta, 1995, 78, 486). Other modifications include 2'-methoxy (2'-O-CH3), 2'-propoxy (2'-OCH2 CH2CH3) and 2'-fluoro (2'-F). Similar modifications may also be made at other positions on the oligonucleotide, particularly the 3' position of the sugar on the 3' terminal nucleotide and the 5' position of 5' terminal nucleotide. Oligonucleotides may also have sugar mimetics such as cyclobutyls in place of the pentofuranosyl group. Both a sugar and an internucleoside linkage, e.g., the backbone, of the nucleotide units may be replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, for example, an aminoethylglycine backbone. The nucleobases are retained and are bound directly or indirectly to aza nitrogen atoms of the amide portion of the backbone. Representative United States patents that teach the preparation of PNA compounds include, but are not limited to, US patent nos. 5,539,082; 5,714,331; and 5,719,262. Further teaching of PNA compounds can be found in Nielsen et al., Science, 254: 1497-1500 (1991).

Guide RNAs can also include, additionally or alternatively, nucleobase (often referred to in the art simply as "base") modifications or substitutions. As used herein, "unmodified" or "natural" nucleobases include adenine (A), guanine (G), thymine (T), cytosine (C) and uracil (U). Modified nucleobases include nucleobases found only infrequently or transiently in natural nucleic acids, e.g. hypoxanthine, 6-methyladenine, 5-Me pyrimidines, particularly 5-methylcytosine (also referred to as 5-methyl-2' deoxycytosine and often referred to in the art as 5-Me-C), 5-hydroxymethylcytosine (HMC), glycosyl HMC and gentobiosyl HMC, as well as synthetic nucleobases, *e.g.* 2-aminoadenine, 2-(methylamino)adenine, 2-(imidazolylalkyl)adenine, 2-(aminoalklyamino)adenine or other heterosubstituted alkyladenines, 2-thiouracil, 2-thiothymine, 5-bromouracil, 5-hydroxymethyluracil, 8-azaguanine, 7-deazaguanine, N6 (6-aminohexyl)adenine and 2,6-diaminopurine. Kornberg, A, DNA Replication, W. H. Freeman & Co., San Francisco, pp75-77 (1980); Gebeyehu et al., Nucl. Acids Res. 15:4513 (1997). A "universal" base known in the art, *e.g.* inosine, can also be included. 5-Me-C substitutions have been shown to increase nucleic acid duplex stability by 0.6-1.2 degrees C. (Sanghvi, Y. S., in Crooke, S. T. and Lebleu, B., eds., Antisense Research and Applications, CRC Press, Boca Raton, 1993, pp. 276-278) and are embodiments of base substitutions.

Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudo-uracil), 4-thiouracil, 8-halo, 8-amino, 8-thiol, 8-thioalkyl, 8-hydroxyl and other a-substituted adenines and guanines, 5-halo particularly 5-bromo, 5-trifluoromethyl and other 5-substituted uracils and cytosines, 7-methylquanine and 7-methyladenine, 8-azaguanine and 8-azaadenine, 7-deazaguanine and 7-deazaadenine and 3-deazaguanine and 3-deazaadenine.

Other useful nucleobases include those disclosed in United States Patent No. 3,687,808, those disclosed in "The Concise Encyclopedia of Polymer Science And Engineering", pages 858-859, Kroschwitz, J.I., ed. John Wiley & Sons, 1990, those disclosed by Englisch et al., Angewandte Chemie, International Edition, 1991, 30, page 613, and those disclosed in Sanghvi, Y. S., Chapter 15, Antisense Research and Applications, pages 289-302, Crooke, S.T. and Lebleu, B. ea., CRC Press, 1993. Certain of these nucleobases are particularly useful for increasing the binding affinity of the oligomeric compounds of the disclosure. These include 5-substituted pyrimidines, 6-azapyrimidines and N-2, N-6 and -O-6 substituted purines, including 2-aminopropyladenine, 5-propynyluracil and 5-propynylcytosine. 5-methylcytosine substitutions have been shown to increase nucleic acid duplex stability by 0.6-1.2 oc (Sanghvi, Y.S., Crooke, S.T. and Lebleu, B., eds, "Antisense Research and Applications", CRC Press, Boca Raton, 1993, pp. 276-278) and are embodiments of base substitutions, even more particularly when combined with 2'-O-methoxyethyl sugar modifications. Modified nucleobases are described in US patent nos. 3,687,808, as well as 4,845,205; 5,130,302; 5,134,066; 5,175, 273; 5, 367,066; 5,432,272; 5,457,187; 5,459,255; 5,484,908; 5,502,177; 5,525,711; 5,552,540; 5,587,469; 5,596,091; 5,614,617; 5,681,941; 5,750,692; 5,763,588; 5,830,653; 6,005,096; and US Patent Application Publication 20030158403.

It is not necessary for all positions in a given oligonucleotide to be uniformly modified, and in fact more than one of the aforementioned modifications may be incorporated in a single oligonucleotide or even at within a single nucleoside within an oligonucleotide.

The guide RNAs and/or mRNA encoding an endonuclease such as M-SmallCas9 of the disclosure may be capped using any one of current capping methods such as mCAP, ARCA or enzymatic capping methods to create viable mRNA constructs that remain biologically active and avoid self/non-self intracellular responses. The guide RNAs and/or mRNA encoding an endonuclease such as M-SmallCas9 of the disclosure may be capped by using a CleanCap^{™} (TriLink) co-transcriptional capping method.

The guide RNAs and/or mRNA encoding an endonuclease of the disclosure may include one or more modifications selected from the group consisting of pseudouridine, N¹-methylpseudouridine, and 5-methoxyuridine. One or more N¹-methylpseudouridines may be incorporated into the guide RNAs and/or mRNA encoding an endonuclease of the disclosure in order to provide enhanced RNA stability and/or protein expression and reduced immunogenicity in animal cells, such as mammalian cell (e.g., human and mice). The N¹-methylpseudouridine modifications may be incorporated in combination with one or more 5-methylcytidines.

The guide RNAs and/or mRNA (or DNA) encoding an endonuclease such as M-SmallCas9 may be chemically linked to one or more moieties or conjugates that enhance the activity, cellular distribution, or cellular uptake of the oligonucleotide. Such moieties include but are not limited to, lipid moieties such as a cholesterol moiety [Letsinger et al., Proc. Nat/. Acad. Sci. USA, 86: 6553-6556 (1989)]; cholic acid [Manoharan et al., Bioorg. Med. Chem. Let., 4: 1053-1060 (1994)]; a thioether, *e.g.* hexyl-S-tritylthiol [Manoharan eta/, Ann. N. Y Acad. Sci., 660: 306-309 (1992) and Manoharan et al., Bioorg. Med. Chem. Let., 3. 2765-2770 (1993)); a thiocholesterol [Oberhauser et al., Nucl. Acids Res., 20: 533-538 (1992)]; an aliphatic chain, *e.g.* dodecandiol or undecyl residues [Kabanov et al., FEBS Lett., 259: 327-330 (1990) and Svinarchuk et al., Biochimie, 75: 49-54 (1993)]; a phospholipid, *e.g.* di-hexadecyl-rac-glycerol or triethylammonium 1 ,2-di-O-hexadecyl-rac-glycero-3-H-phosphonate [Manoharan et al., Tetrahedron Lett., 36:3651-3654 (1995) and Shea et al., Nucl. Acids Res., 18: 3777-3783 (1990)]; a polyamine or a polyethylene glycol chain [Mancharan etc., Nucleosides & Nucleotides, 14: 969-973 (1995)]; adamantane acetic acid [Manoharan et al., Tetrahedron Lett., 36: 3651-3654 (1995)]; a palmityl moiety [(Mishra etc., Biochim. Biophys. Acta, 1264: 229-237 (1995)]; or an octadecylamine or hexylamino-carbonyl-t oxycholesterol moiety [Crooke et al., J. Pharmacol. Exp. Ther., 277: 923-937 (1996)]. See also US Patent Nos. 4,828,979; 4,948,882; 5,218,105; 5,525,465; 5,541,313; 5,545,730; 5,552, 538; 5,578,717, 5,580,731; 5,580,731; 5,591,584; 5,109,124; 5,118,802; 5,138,045; 5,414,077; 5,486, 603; 5,512,439; 5,578,718; 5,608,046; 4,587,044; 4,605,735; 4,667,025; 4,762, 779; 4,789,737; 4,824,941; 4,835,263; 4,876,335; 4,904,582; 4,958,013; 5,082, 830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391, 723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599, 928 and 5,688,941.

Sugars and other moieties can be used to target proteins and complexes including nucleotides, such as cationic polysomes and liposomes, to particular sites. For example, hepatic cell directed transfer can be mediated via asialoglycoprotein receptors (ASGPRs); see, *e.g.* Hu, et al., Protein Pept Lett. 21(1 0):1025-30 (2014). Other systems known in the art and regularly developed can be used to target biomolecules of use in the present case and/or complexes thereof to particular target cells of interest.

These targeting moieties or conjugates can include conjugate groups covalently bound to functional groups such as primary or secondary hydroxyl groups. Suitable conjugate groups include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugate groups include cholesterols, lipids, phospholipids, biotin, phenazine, folate, phenanthridine, anthraquinone, acridine, fluoresceins, rhodamines, coumarins, and dyes. Groups that are capable of enhancing the pharmacodynamic properties include groups that improve uptake, enhance resistance to degradation, and/or strengthen sequence-specific hybridization with the target nucleic acid. Groups that are capable of enhancing the pharmacokinetic properties include groups that improve uptake, distribution, metabolism or excretion of the compounds of the present disclosure. Representative conjugate groups are disclosed in International Patent Application No. PCT/US92/09196, filed Oct. 23, 1992, and US Patent No. 6,287,860. Conjugate moieties include, but are not limited to, lipid moieties such as a cholesterol moiety, cholic acid, a thioether, *e.g.* hexyl-5-tritylthiol, a thiocholesterol, an aliphatic chain, *e.g.* dodecandiol or undecyl residues, a phospholipid, *e.g.* di-hexadecyl-rac-glycerol or triethylammonium 1,2-di-0-hexadecyl-rac-glycero-3-H- phosphonate, a polyamine or a polyethylene glycol chain, or adamantane acetic acid, a palmityl moiety, or an octadecylamine or hexylamino-carbonyl-oxy cholesterol moiety. See, *e.g.* US Patent Nos. 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

Longer polynucleotides that are less amenable to chemical synthesis and are generally produced by enzymatic synthesis can also be modified by various means. Such modifications can include, for example, the introduction of certain nucleotide analogs, the incorporation of particular sequences or other moieties at the 5' or 3' ends of molecules, and other modifications. By way of illustration, the mRNA encoding M-SmallCas9 is approximately 4kb in length and can be synthesized by *in vitro* transcription. Modifications to the mRNA can be applied to, *e.g.* increase its translation or stability (such as by increasing its resistance to degradation with a cell), or to reduce the tendency of the RNA to elicit an innate immune response that is often observed in cells following introduction of exogenous RNAs, particularly longer RNAs such as that encoding M-SmallCas9.

Numerous such modifications have been described in the art, such as polyA tails, 5' cap analogs (*e.g.,* Anti Reverse Cap Analog (ARCA) or m7G(5')ppp(5')G (mCAP)), modified 5' or 3' untranslated regions (UTRs), use of modified bases (such as Pseudo-UTP, 2-Thio-UTP, 5-Methylcytidine-5'-Triphosphate (5-Methyl-CTP) or N6-Methyl-ATP), or treatment with phosphatase to remove 5' terminal phosphates. These and other modifications are known in the art, and new modifications of RNAs are regularly being developed.

There are numerous commercial suppliers of modified RNAs, including for example, TriLink Biotech, Axolabs, Bio-Synthesis Inc., Dharmacon and many others. As described by TriLink, for example, 5-Methyl-CTP can be used to impart desirable characteristics such as increased nuclease stability, increased translation or reduced interaction of innate immune receptors with *in vitro* transcribed RNA. 5'-Methylcytidine-5'-Triphosphate (5-Methyl-CTP), N6-Methyl-ATP, as well as Pseudo-UTP and 2-Thio-UTP, have also been shown to reduce innate immune stimulation in culture and *in vivo* while enhancing translation as illustrated in publications by Konmann *et al.* and Warren *et* al. referred to below.

It has been shown that chemically modified mRNA delivered *in vivo* can be used to achieve improved therapeutic effects; see, *e.g.* Kormann et al., Nature Biotechnology 29, 154-157 (2011). Such modifications can be used, for example, to increase the stability of the RNA molecule and/or reduce its immunogenicity. Using chemical modifications such as Pseudo-U, N6-Methyl-A, 2-Thio-U and 5-Methyl-C, it was found substituting just one quarter of the uridine and cytidine residues with 2-Thio-U and 5-Methyl-C respectively, resulted in a significant decrease in toll-like receptor (TLR) mediated recognition of the mRNA in mice. By reducing the activation of the innate immune system, these modifications can therefore be used to effectively increase the stability and longevity of the mRNA *in vivo,* see, *e.g.* Konmann *et al.,* supra.

It has also been shown that repeated administration of synthetic messenger RNAs incorporating modifications designed to bypass innate anti-viral responses can reprogram differentiated human cells to pluripotency. See, *e.g.* Warren, et al., Cell Stem Cell, 7(5):618-30 (2010). Such modified mRNAs that act as primary reprogramming proteins can be an efficient means of reprogramming multiple human cell types. Such cells are referred to as induced pluripotency stem cells (iPSCs). and it was found that enzymatically synthesized RNA incorporating 5-Methyl-CTP, Pseudo- UTP and an Anti Reverse Cap Analog (ARCA) could be used to effectively evade the cell's antiviral response; see, *e.g.* Warren *et al.,* supra. Other modifications of polynucleotides described in the art include, for example, the use of polyA tails, the addition of 5' cap analogs (such as m7G(5')ppp(5')G (mCAP)), modifications of 5' or 3' untranslated regions (UTRs), or treatment with phosphatase to remove 5' terminal phosphates-and new approaches are regularly being developed.

A number of compositions and techniques applicable to the generation of modified RNAs for use herein have been developed in connection with the modification of RNA interference (RNAi), including small-interfering RNAs (siRNAs). siRNAs present particular challenges *in vivo* because their effects on gene silencing via mRNA interference are generally transient, which can require repeat administration. In addition, siRNAs are double-stranded RNAs (dsRNA) and mammalian cells have immune responses that have evolved to detect and neutralize dsRNA, which is often a by-product of viral infection. Thus, there are mammalian enzymes such as PKR (dsRNA-responsive kinase), and potentially retinoic acid-inducible gene I (RIG-I), that can mediate cellular responses to dsRNA, as well as Toll-like receptors (such as TLR3, TLR7 and TLR8) that can trigger the induction of cytokines in response to such molecules; see, *e.g.* the reviews by Angart et al., Pharmaceuticals (Basel) 6(4): 440-468 (2013); Kanasty et al., Molecular Therapy 20(3): 513-524 (2012); Burnett et al., Biotechnol J. 6(9):1130-46 (2011); Judge and Maclachlan, Hum Gene Ther 19(2):111-24 (2008); and references cited therein.

A large variety of modifications have been developed and applied to enhance RNA stability, reduce innate immune responses, and/or achieve other benefits that can be useful in connection with the introduction of polynucleotides into human cells as described herein; see, *e.g.* the reviews by Whitehead KA et al., Annual Review of Chemical and Biomolecular Engineering, 2:77-96 (2011); Gaglione and Messere, Mini Rev Med Chem, 10(7):578-95 (2010); Chernolovskaya et al, Curr Opin Mol Ther., 12(2):158-67 (2010); Deleavey et al., Curr Protoc Nucleic Acid Chem Chapter 16:Unit 16.3 (2009); Behlke, Oligonucleotides 18(4):305-19 (2008): Fucini et al., Nucleic Acid Ther 22(3): 205-210 (2012); Bremsen et al., Front Genet 3:154 (2012).

As noted above, there are a number of commercial suppliers of modified RNAs, many of which have specialized in modifications designed to improve the effectiveness of siRNAs. A variety of approaches are offered based on various findings reported in the literature. For example, Dharmacon notes that replacement of a non-bridging oxygen with sulfur (phosphorothioate, PS) has been extensively used to improve nuclease resistance of siRNAs, as reported by Kale, Nature Reviews Drug Discovery 11:125-140 (2012). Modifications of the 2'-position of the ribose have been reported to improve nuclease resistance of the internucleotide phosphate bond while increasing duplex stability (Tm), which has also been shown to provide protection from immune activation. A combination of moderate PS backbone modifications with small, well-tolerated 2'-substitutions (2'-O-, 2'-Fluoro, 2'-Hydro) has been associated with highly stable siRNAs for applications *in vivo,* as reported by Soutschek et al. Nature 432:173-178 (2004); and 2'-O-Methyl modifications have been reported to be effective in improving stability as reported by Volkov, Oligonucleotides 19:191-202 (2009). With respect to decreasing the induction of innate immune responses, modifying specific sequences with 2'-O-Methyl, 2'-Fiuoro, 2'-Hydro have been reported to reduce TLR7/TLR8 interaction while generally preserving silencing activity; see, *e.g.* Judge et al., Mol. Ther. 13:494-505 (2006); and Cekaite et al., J. Mol. Biol. 365:90-108 (2007). Additional modifications, such as 2-thiouracil, pseudouracil, 5-methylcytosine, 5-methyluracil, and N6-methyladenosine have also been shown to minimize the immune effects mediated by TLR3, TLR7, and TLR8; see, *e.g.* Kariko et al., Immunity 23:165-175 (2005).

As is also known in the art, and commercially available, a number of conjugates can be applied to polynucleotides such as RNAs for use herein that can enhance their delivery and/or uptake by cells, including for example, cholesterol, tocopherol and folic acid, lipids, peptides, polymers, linkers and aptamers; see, *e.g.* the review by Winkler, Ther. Deliv. 4:791-809 (2013), and references cited therein.

### Additional Sequences

A guide RNA may comprise at least one additional segment at either the 5' or 3' end. For example, a suitable additional segment can comprise a 5' cap (*e.g.* a 7-methylguanylate cap (m7G)); a 3' polyadenylated tail (*e.g.,* a 3' poly(A) tail); a riboswitch sequence (*e.g.* to allow for regulated stability and/or regulated accessibility by proteins and protein complexes); a sequence that forms a dsRNA duplex (*e.g.,* a hairpin)); a sequence that targets the RNA to a subcellular location (*e.g.* nucleus, mitochondria, chloroplasts, and the like); a modification or sequence that provides for tracking (*e.g.* direct conjugation to a fluorescent molecule, conjugation to a moiety that facilitates fluorescent detection, a sequence that allows for fluorescent detection, *etc.*); a modification or sequence that provides a binding site for proteins (*e.g*. proteins that act on DNA, including transcriptional activators, transcriptional repressors, DNA methyltransferases, DNA demethylases, histone acetyltransferases, histone deacetylases, and the like) a modification or sequence that provides for increased, decreased, and/or controllable stability; and combinations thereof.

### Stability Control Sequence

A stability control sequence influences the stability of an RNA (*e.g.* a guide RNA). A non-limiting example of a suitable stability control sequence is a transcriptional terminator segment (*e.g*., a transcription termination sequence). A transcriptional terminator segment of a guide RNA can have a total length of from 10 nucleotides to 100 nucleotides, *e.g*. from 10 nucleotides (nt) to 20 nt, from 20 nt to 30 nt, from 30 nt to 40 nt, from 40 nt to 50 nt, from 50 nt to 60 nt, from 60 nt to 70 nt, from 70 nt to 80 nt, from 80 nt to 90 nt, or from 90 nt to 100 nt. For example, the transcriptional terminator segment can have a length of from 15 nucleotides (nt) to 80 nt, from 15 nt to 50 nt, from 15 nt to 40 nt, from 15 nt to 30 nt or from 15 nt to 25 nt.

The transcription termination sequence may be one that is functional in a eukaryotic cell. The transcription termination sequence may be one that is functional in a prokaryotic cell.

Nucleotide sequences that can be included in a stability control sequence (e.g. transcriptional termination segment, or in any segment of the guide RNA to provide for increased stability) include, for example, a Rho-independent trp termination site.

### Mimetics

A nucleic acid may be a nucleic acid mimetic. The term "mimetic" as it is applied to polynucleotides is intended to include polynucleotides wherein only the furanose ring or both the furanose ring and the internucleotide linkage are replaced with non-furanose groups, replacement of only the furanose ring is also referred to in the art as being a sugar surrogate. The heterocyclic base moiety or a modified heterocyclic base moiety is maintained for hybridization with an appropriate target nucleic acid. One such nucleic acid, a polynucleotide mimetic that has been shown to have excellent hybridization properties, is referred to as a peptide nucleic acid (PNA). In PNA**,** the sugar-backbone of a polynucleotide is replaced with an amide containing backbone, in particular an aminoethylglycine backbone. The nucleotides are retained and are bound directly or indirectly to aza nitrogen atoms of the amide portion of the backbone.

One polynucleotide mimetic that has been reported to have excellent hybridization properties is a peptide nucleic acid (PNA). The backbone in PNA compounds is two or more linked aminoethylglycine units, which gives PNA an amide containing backbone. The heterocyclic base moieties are bound directly or indirectly to aza nitrogen atoms of the amide portion of the backbone. Representative US patents that describe the preparation of PNA compounds include, but are not limited to: US Patent Nos. 5,539,082; 5,714,331; and 5,719,262.

Another class of polynucleotide mimetic that has been studied is based on linked morpholino units (morpholino nucleic acid) having heterocyclic bases attached to the morpholino ring. A number of linking groups have been reported that link the morpholino monomeric units in a morpholino nucleic acid. One class of linking groups has been selected to give a non-ionic oligomeric compound. The non-ionic morpholino-based oligomeric compounds are less likely to have undesired interactions with cellular proteins. Morpholino-based polynucleotides are nonionic mimics of oligonucleotides, which are less likely to form undesired interactions with cellular proteins (Dwaine A. Braasch and David R. Corey, Biochemistry, 2002, 41(14), 45034510). Morpholino-based polynucleotides are disclosed in US Patent No. 5,034,506. A variety of compounds within the morpholino class of polynucleotides have been prepared, having a variety of different linking groups joining the monomeric subunits.

A further class of polynucleotide mimetic is referred to as cyclohexenyl nucleic acids (GeNA). The furanose ring normally present in a DNA/RNA molecule is replaced with a cydohexenyl ring. GeNA DMT protected phosphoramidite monomers have been prepared and used for oligomeric compound synthesis following classical phosphoramidite chemistry. Fully modified GeNA oligomeric compounds and oligonucleotides having specific positions modified with GeNA have been prepared and studied (see Wang et al., J. Am. Chem. Soc., 2000, 122, 85958602). In general the incorporation of GeNA monomers into a DNA chain increases its stability of a DNA/RNA hybrid. GeNA oligoadenylates formed complexes with RNA and DNA complements with similar stability to the native complexes. The study of incorporating GeNA structures into natural nucleic acid structures was shown by NMR and circular dichroism to proceed with easy conformational adaptation.

A further modification includes Locked Nucleic Acids (LNAs) in which the 2'-hydroxyl group is linked to the 4' carbon atom of the sugar ring thereby forming a 2'-C,4'-C-oxymethylene linkage thereby forming a bicyclic sugar moiety. The linkage can be a methylene (-CH2-), group bridging the 2' oxygen atom and the 4' carbon atom wherein n is 1 or 2 (Singh et al., Chem. Commun., 1998, 4, 455-456). LNA and LNA analogs display very high duplex thermal stabilities with complementary DNA and RNA (Tm = +3 to +10 °C), stability towards 3'-exonucleolytic degradation and good solubility properties. Potent and nontoxic antisense oligonucleotides containing LNAs have been described (Wahlestedt et al., Proc. Natl. Acad. Sci. U.S.A, 2000, 97, 5633-5638).

The synthesis and preparation of the LNA monomers adenine, cytosine, guanine, 5-methylcytosine, thymine and uracil, along with their oligomerization, and nucleic acid recognition properties have been described (Koshkin et al., Tetrahedron, 1998, 54, 3607-3630). LNAs and preparation thereof are also described in WO 98/39352 and WO 99/14226.

### Modified sugar moieties

A nucleic acid can also include one or more substituted sugar moieties. Suitable polynucleotides include a sugar substituent group selected from: OH; F; O-, S-, or N-alkyl; O-, S-, or N-alkenyl; O-, S-or N-alkynyl; or O-alkyl-O-alkyl, wherein the alkyl, alkenyl and alkynyl may be substituted or unsubstituted C1 to C10 alkyl or C2 to C10 alkenyl and alkynyl. Particularly suitable are O((CH2)nO)mCH3, O(CH2)nOCH3, O(CHz)nNH2, O(CH2)CH3, O(CH2)nONH2, and O(CH2)nON((CH2)nCH3)2, where n and m are from 1 to about 10. Other suitable polynucleotides include a sugar substituent group selected from: C1 to C10 lower alkyl, substituted lower alkyl, alkenyl, alkynyl, alkaryl, aralkyl, O-alkaryl or O-aralkyl, SH, SCH3, OCN, Cl, Br, CN, CF3, OCF3, SOCH3, SO2CH3, ONO2, NO2, N3, NH2, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A suitable modification includes 2'-methoxyethoxy 2'-O-CH2-CH2OCH3, also known as -2'-O-(2-methoxyethyl) or 2'-MOE) (Martin et al., Hely. Chim. Acta, 1995, 78, 486-504) *e.g.,* an alkoxyalkoxy group. A further suitable modification includes 2'-dimethylaminooxyethoxy, *e.g.,* a O(CH2)2ON(CH3)2 group (2'-DMAOE), as described in examples herein below, and 2'- dimethylaminoethoxyethoxy (also known in the art as 2'-O-dimethyl-amino-ethoxy-ethyl or 2'- DMAEOE), *e.g.,* 2'-O-CH2-O-CH2-N(CH3)2.

Other suitable sugar substituent groups include methoxy (-O-CH3), aminopropoxy (-O-CH2CH2CH2NH2), allyl (-CH2-CH=CH2), -O-allyl (-O-CH2-CH=CH2) and fluoro (F). 2'-sugar substituent groups may be in the arabino (up) position or ribo (down) position. A suitable 2'-arabino modification is 2'-F. Similar modifications may also be made at other positions on the oligomeric compound, particularly the 3' position of the sugar on the 3' terminal nucleoside or in 2'-5' linked oligonucleotides and the 5' position of 5' terminal nucleotide. Oligomeric compounds may also have sugar mimetics such as cyclobutyl moieties in place of the pentofuranosyl sugar.

### Base modifications and Substitutions

A nucleic acid may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine. Further modified nucleobases include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (*e.g*. 9-(2-aminoethoxy)-H-pyrimido(5,4-(b) (1,4)benzoxazin-2(3H)- one), carbazole cytidine (2H-pyrimido(4,5-b)indol-2-one), pyridoindole cytidine (H-pyrido(3 ',2 ':4,5)pyrrolo(2,3-d)pyrimidin-2-one).

Heterocyclic base moieties may also include those in which the purine or pyrimidine base is replaced with other heterocycles, for example 7-deaza-adenine, 7-deazaguanosine, 2-aminopyridine and 2-pyridone. Further nucleobases include those disclosed in US Patent No. 3,687,808, those disclosed in The Concise Encyclopedia Of Polymer Science And Engineering, pages 858-859, Kroschwitz, J. 1., ed. John Wiley & Sons, 1990, those disclosed by Englisch et al., Angewandte Chemie, International Edition, 1991, 30, 613, and those disclosed by Sanghvi, Y. S., Chapter 15, Antisense Research and Applications, pages 289-302, Crooke, S. T. and Lebleu, B., ed., CRC Press, 1993. Certain of these nucleobases are useful for increasing the binding affinity of an oligomeric compound. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2 oc. (Sanghvi et al., eds., Antisense Research and Applications, CRC Press, Boca Raton, 1993, pp. 276-278) and are suitable base substitutions, *e.g.* when combined with 2'-O-methoxyethyl sugar modifications.

"Complementary" refers to the capacity for pairing, through base stacking and specific hydrogen bonding, between two sequences comprising naturally or non-naturally occurring (*e.g.* modified as described above) bases (nucleosides) or analogs thereof. For example, if a base at one position of a nucleic acid is capable of hydrogen bonding with a base at the corresponding position of a target, then the bases are considered to be complementary to each other at that position. Nucleic acids can include universal bases, or inert abasic spacers that provide no positive or negative contribution to hydrogen bonding. Base pairings may include both canonical Watson-Crick base pairing and non-Watson-Crick base pairing (*e.g.* Wobble base pairing and Hoogsteen base pairing).

It is understood that for complementary base pairings, adenosine-type bases (A) are complementary to thymidine-type bases (T) or uracil-type bases (U), that cytosine-type bases (C) are complementary to guanosine-type bases (G), and that universal bases such as such as 3-nitropyrrole or 5-nitroindole can hybridize to and are considered complementary to any A, C, U, or T. Nichols et al., Nature, 1994;369:492-493 and Loakes et al., Nucleic Acids Res., 1994;22:4039-4043. Inosine (I) has also been considered in the art to be a universal base and is considered complementary to any A, C, U, or T. See Watkins and Santalucia, Nucl. Acids Research, 2005; 33 (19): 6258-6267.

### Conjugates

Another possible modification of a nucleic acid involves chemically linking to the polynucleotide one or more moieties or conjugates which enhance the activity, cellular distribution or cellular uptake of the oligonucleotide. These moieties or conjugates can include conjugate groups covalently bound to functional groups such as primary or secondary hydroxyl groups. Conjugate groups include, but are not limited to, intercalators, reporter molecules, polyamines, polyamides, polyethylene glycols, polyethers, groups that enhance the pharmacodynamic properties of oligomers, and groups that enhance the pharmacokinetic properties of oligomers. Suitable conjugate groups include, but are not limited to, cholesterols, lipids, phospholipids, biotin, phenazine, folate, phenanthridine, anthraquinone, acridine, fluoresceins, rhodamines, coumarins, and dyes. Groups that enhance the pharmacodynamic properties include groups that improve uptake, enhance resistance to degradation, and/or strengthen sequence-specific hybridization with the target nucleic acid. Groups that enhance the pharmacokinetic properties include groups that improve uptake, distribution, metabolism or excretion of a nucleic acid.

Conjugate moieties include but are not limited to lipid moieties such as a cholesterol moiety (Letsinger et al., Proc. Natl. Acad. Sci. USA, 1989, 86, 6553-6556), cholic acid (Manoharan et al., Bioorg. Med. Chem. Let., 1994,4, 1053-1060), a thioether, *e.g.* hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, *e.g.* dodecandiol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, *e.g.* di-hexadecyl-rac-glycerol or triethylammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 36513654), a palmityl moiety (Mishra et al., Biochim. Biophys. Acta, 1995, 1264, 229-237), or an octadecylamine or hexylamino-carbonyl-oxycholesterol moiety (Crooke et al., J. Pharmacal. Exp. Ther., 1996,277, 923-937).

A conjugate may include a "Protein Transduction Domain" or PTD (also known as a CPP-cell penetrating peptide), which may refer to a polypeptide, polynucleotide, carbohydrate, or organic or inorganic compound that facilitates traversing a lipid bilayer, micelle, cell membrane, organelle membrane, or vesicle membrane. A PTD attached to another molecule, which can range from a small polar molecule to a large macromolecule and/or a nanoparticle, facilitates the molecule traversing a membrane, for example going from extracellular space to intracellular space, or cytosol to within an organelle. A PTD may be covalently linked to the amino terminus of an exogenous polypeptide (*e.g.* a M-SmallCas9 polypeptide or variant thereof). A PTD may be covalently linked to the C-terminus or the N-terminus of an exogenous polypeptide (*e.g.* a M-SmallCas9 polypeptide or variant thereof). A PTD may be covalently linked to a nucleic acid (*e.g.* a guide RNA, a polynucleotide encoding a guide RNA, a polynucleotide encoding a M-SmallCas9 polypeptide or variant thereof, *etc.*)*.* Exemplary PTDs include but are not limited to a minimal undecapeptide protein transduction domain (corresponding to residues 47-57 of HIV-1 TAT comprising YGRKKRRQRRR; a polyarginine sequence comprising a number of arginines sufficient to direct entry into a cell *(e.g.* 3, 4, 5, 6, 7, 8, 9, 10, or 10-50 arginines); a VP22 domain (Zender et al. (2002) Cancer Gene Ther. 9(6):489-96); an Drosophila Antennapedia protein transduction domain (Noguchi et al. (2003) Diabetes 52(7):1732-1737); a truncated human calcitonin peptide (Trehin et al. (2004) Pharm. Research 21:1248-1256); polylysine (Wender et al. (2000) Proc. Natl. Acad. Sci. USA 97:13003-13008). The PTD may be an activatable CPP (ACPP) (Aguilera et al. (2009) Integr Biol (Camb) June; 1(5-6): 371-381). ACPPs include a polycationic CPP (*e.g.* Arg9 or "R9") connected via a cleavable linker to a matching polyanion (*e.g.* Glu9 or "E9"), which reduces the net charge to nearly zero and thereby inhibits adhesion and uptake into cells. Upon cleavage of the linker, the polyanion is released, locally unmasking the polyarginine and its inherent adhesiveness, thus "activating" the ACPP to traverse the membrane. The PTD may be chemically modified in order to increase the bioavailability of the PTD. Exemplary modifications are disclosed in Expert Opin Drug Deliv. 2009 Nov;6(11):1195-205.

### Polypeptide modifications

A M-SmallCas9 polypeptide or variant thereof expressed from a codon-optimized polynucleotide sequence may be produced *in vitro* or by eukaryotic cells, by prokaryotic cells, or by in-vitro transcription and translation (IVTT) and it may be further processed by unfolding, *e.g.* heat denaturation, OTT reduction, *etc.* and may be further refolded, using methods known in the art.

Modifications of interest that do not alter primary sequence include chemical derivatization of polypeptides, *e.g.* acylation, acetylation, carboxylation, amidation, *etc.* Also included are modifications of glycosylation, *e.g.* those made by modifying the glycosylation patterns of a polypeptide during its synthesis and processing or in further processing steps; *e.g.* by exposing the polypeptide to enzymes which affect glycosylation, such as mammalian glycosylating or deglycosylating enzymes. Also embraced are sequences that have phosphorylated amino acid residues, e.g. phosphotyrosine, phosphoserine, or phosphothreonine.

M-SmallCas9 polypeptides or variants thereof may have been modified using ordinary molecular biological techniques and synthetic chemistry so as to improve their resistance to proteolytic degradation, to change the target sequence specificity, to optimize solubility properties, to alter protein activity (*e.g.* transcription modulatory activity, enzymatic activity, *etc.*) or to render them more suitable as a therapeutic agent. Analogs of such polypeptides include those containing residues other than naturally occurring L-amino acids, *e.g*. O-amino acids or unnatural, synthetic amino acids. D-amino acids may be substituted for some or all of the amino acid residues. The M-SmallCas9 polypeptides or variants thereof may be prepared by *in vitro* synthesis, using conventional methods as known in the art. Various commercial synthetic apparatuses are available, for example, automated synthesizers by Applied Biosystems, Inc., Beckman, *etc.* By using synthesizers, natural amino acids may be substituted with unnatural amino acids. The particular sequence and the manner of preparation may be determined by convenience, economics, purity required, and the like.

If desired, various groups may be introduced into the peptide during synthesis or during expression, which allow for linking to other molecules or to a surface. Thus cysteines can be used to make thioethers, histidines for linking to a metal ion complex, carboxyl groups for forming amides or esters, amino groups for forming amides, and the like.

### Recombinant Cells

The codon-optimized M-SmallCas9 system herein described may be used in eukaryotic, such as mammalian cells, for example, a human cell. Any human cell is suitable for use with the codon-optimized M-SmallCas9 system disclosed herein.

A cell *ex vivo* or *in vitro* may include: (a) a nucleic acid comprising a codon-optimized polynucleotide sequence encoding a M-SmallCas9 polypeptide or variant described herein, or a M-SmallCas9 polypeptide or variant thereof expressed from the nucleic acid; and (b) a gRNA or nucleic acid encoding the gRNA, wherein the gRNA is capable of guiding the M-SmallCas9 polypeptide or variant thereof to a target polynucleotide sequence. The cell may comprise the nucleic acid comprising the codon-optimized polynucleotide sequence. In some embodiments, the cell comprises the gRNA. In some embodiments, the cell comprises nucleic acid encoding the gRNA. In some embodiments, the gRNA is a single guide RNA (sgRNA). In some embodiments, the cell comprises one or more additional gRNAs or nucleic acid encoding the one or more additional gRNAs. The cell may further comprise a donor template.

In one aspect, disclosed herein is a method of transforming a cell that includes introducing into a host cell, such as an animal cell, a nucleic acid as provided herein, and selecting or screening for a transformed cell. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein. Techniques for transforming a wide variety of the above-mentioned host cells and species are known in the art and described in the technical and scientific literature. Accordingly, cell cultures comprising at least one recombinant cell as disclosed herein are also within the scope of this application. Methods and systems suitable for generating and maintaining cell cultures are known in the art.

In a related aspect, disclosed herein are recombinant host cells, for example, recombinant animal cells that include a nucleic acid described herein. The nucleic acid can be stably integrated in the host genome, or can be episomally replicating, or present in the recombinant host cell as a mini-circle expression vector for a stable or transient expression. Accordingly, the nucleic acid may be maintained and replicated in the recombinant host cell as an episomal unit. The nucleic acid may be stably integrated into the genome of the recombinant cell. The nucleic acid may be present in the recombinant host cell as a mini-circle expression vector for a stable or transient expression.

Host cells may be genetically engineered (*e.g*. transduced or transformed or transfected) with, for example, a vector construct of the present application that may be, for example, a vector for homologous recombination that includes nucleic acid sequences homologous to a portion of the genome of the host cell, or can be an expression vector for the expression of any or a combination of the genes of interest. The vector can be, for example, in the form of a plasmid, a viral particle, a phage, *etc.* A vector for expression of a polypeptide of interest may also be designed for integration into the host, *e.g.,* by homologous recombination.

The disclosure may provide a genetically modified host cell, *e.g.* isolated genetically modified host cell, where a genetically modified host cell includes: 1) an exogenous guide RNA; 2) an exogenous nucleic acid comprising a nucleotide sequence encoding a guide RNA; 3) an exogenous nucleic acid comprising a codon-optimized polynucleotide sequence encoding a M-SmallCas9 polypeptide or variant thereof; 4) an exogenous M-SmallCas9 polypeptide or variant thereof expressed from a nucleic acid comprising a codon-optimized polynucleotide sequence; or 5) any combination of the above. The genetically modified cell may be generated by genetically modifying a host cell with, for example: 1) an exogenous guide RNA; 2) an exogenous nucleic acid comprising a nucleotide sequence encoding a guide RNA; 3) an exogenous nucleic acid comprising a codon-optimized polynucleotide sequence encoding a M-SmallCas9 polypeptide or variant thereof; 4) an exogenous M-SmallCas9 polypeptide or variant thereof expressed from a nucleic acid comprising a codon-optimized polynucleotide sequence; or 5) any combination of the above.

All cells suitable to be a target cell as discussed above are also suitable to be a genetically modified host cell. For example, a genetically modified host cells of interest can be a cell from any organism, *e*.*g*., a bacterial cell, an archaeal cell, a cell of a single-cell eukaryotic organism, a plant cell, an algal cell (*e*.*g*., Botryococcus braunii, Chlamydomonas reinhardtii, Nannochloropsis gaditana, Chlorela pyrenoidosa, Sargassum patens (C. Agardh), and the like), a fungal cell *(e.g.,* a yeast cell), an animal cell, a cell from an invertebrate animal *(e.g.* fruit fly, cnidarian, echinoderm, nematode, *etc*.), *a* cell from a vertebrate animal *(e.g.,* fish, amphibian, reptile, bird, mammal), a cell from a mammal *(e.g.,* a pig, a cow, a goat, a sheep, a rodent. a rat, a mouse, a non-human primate, a human, *etc.).* The genetically modified host cell may be be any cell from a human.

The genetically modified host cell of the disclosure may have been genetically modified with an exogenous nucleic acid comprising a nucleotide sequence encoding a M-SmallCas9 polypeptide or variant thereof. The genetically modified host cell may have been genetically modified with an exogenous nucleic acid comprising a nucleotide sequence encoding for a M-SmallCas9 polypeptide or a variant described herein. The DNA of a genetically modified host cell can be targeted for modification by introducing into the cell a guide RNA (or a DNA encoding a guide RNA, which determines the genomic location/sequence to be modified) and optionally a donor nucleic acid. The nucleotide sequence encoding a M-SmallCas9 polypeptide or variant thereof may be operably linked to an inducible promoter (*e*.*g*. heat shock promoter, Tetracycline-regulated promoter, Steroid-regulated promoter, Metal-regulated promoter, estrogen receptor-regulated promoter, *etc.).* The codon-optimized nucleotide sequence encoding a M-SmallCas9 polypeptide or variant thereof may be operably linked to a spatially restricted and/or temporally restricted promoter (*e*.*g*. a tissue specific promoter, a cell type specific promoter, a cell cycle specific promoter). The codon-optimized nucleotide sequence encoding a M-SmallCas9 polypeptide or variant thereof may be operably linked to a constitutive promoter.

A genetically modified host cell may be *in vitro.* A genetically modified host cell may be a prokaryotic cell or may be derived from a prokaryotic cell. A genetically modified host cell may be a bacterial cell or may be derived from a bacterial cell. Agenetically modified host cell may be an archaeal cell or may be derived from an archaeal cell. A genetically modified host cell may be a eukaryotic cell or may be derived from a eukaryotic cell. A genetically modified host cell may be a plant cell or may be derived from a plant cell. A genetically modified host cell may be an animal cell or may be derived from an animal cell.A genetically modified host cell may be an invertebrate cell or may be derived from an invertebrate cell. A genetically modified host cell may be a vertebrate cell or may be derived from a vertebrate cell. A genetically modified host cell may be a mammalian cell or may be derived from a mammalian cell. A genetically modified host cell may be a rodent cell or may be derived from a rodent cell. A genetically modified host cell may be a human cell or may be derived from a human cell.

The present disclosure further provides progeny of a genetically modified cell, where the progeny can include the same exogenous nucleic acid or polypeptide as the genetically modified cell from which it was derived. The present disclosure further provides a composition comprising a genetically modified host cell.

A genetically modified host cell may be a genetically modified stem cell or progenitor cell. Suitable host cells include, *e.g.* stem cells (adult stem cells, embryonic stem cells, iPS cells, *etc.)* and progenitor cells *(e.g.,* cardiac progenitor cells, neural progenitor cells, *etc.).* Other suitable host cells include mammalian stem cells and progenitor cells, such as, *e*.*g*., rodent stem cells, rodent progenitor cells, human stem cells, human progenitor cells, *etc.* Other suitable host cells include *in vitro* host cells, *e*.*g*., isolated host cells. A genetically modified host cell may include an exogenous guide RNA nucleic acid. A genetically modified host cell may include an exogenous nucleic acid comprising a nucleotide sequence encoding a guide RNA. A genetically modified host cell may include an exogenous M-SmallCas9 polypeptide or variant thereof expressed from a codon-optimized nucleotide sequence. A genetically modified host cell may include an exogenous nucleic acid comprising a codon-optimized nucleotide sequence encoding a M-SmallCas9 polypeptide or variant thereof. A genetically modified host cell may include an exogenous nucleic acid comprising 1) a nucleotide sequence encoding a guide RNA and 2) a codon-optimized nucleotide sequence encoding a M-SmallCas9 polypeptide or variant thereof.

### Non-human Genetically Modified Organisms

A genetically modified host cell may be genetically modified with an exogenous nucleic acid comprising a codon-optimized nucleotide sequence encoding a M-SmallCas9 polypeptide or variant thereof. If such a cell is a eukaryotic single-cell organism, then the modified cell can be considered a genetically modified organism. The non-human genetically modified organism may be a M-SmallCas9 transgenic multicellular organism.

A genetically modified non-human host cell *(e.g.* a cell that has been genetically modified with an exogenous nucleic acid comprising a codon-optimized nucleotide sequence encoding a M-SmallCas9 polypeptide or variant thereof) may generate a genetically modified nonhuman organism *(e.g.* a mouse, a fish, a frog, a fly, a worm, *etc.).* For example, if the genetically modified host cell is a pluripotent stem cell *(e.g.,* PSC) or a germ cell *(e.g.* sperm, oocyte, *etc.),* an entire genetically modified organism can be derived from the genetically modified host cell. The genetically modified host cell may be a pluripotent stem cell *(e.g.* ESC, iPSC, pluripotent plant stem cell, *etc.)* or a germ cell *(e.g.* sperm cell, oocyte, *etc.),* either *in vivo* or *in vitro* that can give rise to a genetically modified organism. The genetically modified host cell may be a vertebrate PSC *(e.g.* ESC, iPSC, *etc.)* and is used to generate a genetically modified organism (*e*.*g*. by injecting a PSC into a blastocyst to produce a chimeric/mosaic animal, which could then be mated to generate non-chimeric/non-mosaic genetically modified organisms; grafting in the case of plants; *etc.).* Any suitable method/protocol for producing a genetically modified organism, including the methods described herein, is suitable for producing a genetically modified host cell comprising an exogenous nucleic acid comprising a codon-optimized nucleotide sequence encoding a M-SmallCas9 polypeptide or variant thereof. Methods of producing genetically modified organisms are known in the art. For example, see Cho et al., Curr Protoc Cell Biol. 2009 Mar; Chapter 19:Unit 19.11: Generation of transgenic mice; Gama et al., Brain Struct Funct. 2010 Mar; 214(2-3):91-109. Epub 2009 Nov 25: Animal transgenesis: an overview; Husaini et al., GM Crops. 2011 Jun-Dec;2(3):150-62. Epub 2011 Jun 1: Approaches for gene targeting and targeted gene expression in plants.

A genetically modified organism may comprise a target cell for methods of the disclosure, and thus can be considered a source for target cells. For example, if a genetically modified cell comprising an exogenous nucleic acid comprising a codon-optimized nucleotide sequence encoding a M-SmallCas9 polypeptide or variant thereof is used to generate a genetically modified organism, then the cells of the genetically modified organism comprise the exogenous nucleic acid comprising a codon-optimized nucleotide sequence encoding a M-SmallCas9 polypeptide or variant thereof. The DNA of a cell or cells of the genetically modified organism may be targeted for modification by introducing into the cell or cells a guide RNA (or a DNA encoding a guide RNA) and optionally a donor nucleic acid. For example, the introduction of a guide RNA (or a DNA encoding a guide RNA) into a subset of cells *(e.g.* brain cells, intestinal cells, kidney cells, lung cells, blood cells, *etc.)* of the genetically modified organism can target the DNA of such cells for modification, the genomic location of which will depend on the DNA-targeting sequence of the introduced guide RNA.

A genetically modified organism may be a source of target cells for methods of the disclosure. For example, a genetically modified organism comprising cells that are genetically modified with an exogenous nucleic acid comprising a codon-optimized nucleotide sequence encoding a M-SmallCas9 polypeptide or variant thereof can provide a source of genetically modified cells, for example PSCs *(e.g.* ESCs, iPSCs, sperm, oocytes, *etc.),* neurons, progenitor cells, cardiomyocytes, *etc.*

A genetically modified cell may be a PSC comprising an exogenous nucleic acid comprising a codon-optimized nucleotide sequence encoding a M-SmallCas9 polypeptide or variant thereof. As such, the PSC can be a target cell such that the DNA of the PSC can be targeted for modification by introducing into the PSC a guide RNA (or a DNA encoding a guide RNA) and optionally a donor nucleic acid, and the genomic location of the modification will depend on the DNA-targeting sequence of the introduced guide RNA. Thus, the methods described herein may be used to modify the DNA *(e.g.* delete and/or replace any desired genomic location) of PSCs derived from a genetically modified organism. Such modified PSCs can then be used to generate organisms having both (i) an exogenous nucleic acid comprising a codon-optimized nucleotide sequence encoding a M-SmallCas9 polypeptide or variant thereof and (ii) a DNA modification that was introduced into the PSC.

The exogenous nucleic acid may be under the control of (*e*.*g*., operably linked to) an unknown promoter (*e*.*g*. when the nucleic acid randomly integrates into a host cell genome) or can be under the control of (*e*.*g*., operably linked to) a known promoter. Suitable known promoters can be any known promoter and include constitutively active promoters (*e.g.* CMV promoter), inducible promoters *(e.g.* heat shock promoter, Tetracycline-regulated promoter, Steroid-regulated promoter, Metal-regulated promoter, estrogen receptor-regulated promoter, *etc.),* spatially restricted and/or temporally restricted promoters (*e.g.* a tissue specific promoter, a cell type specific promoter, *etc.), etc.*

A genetically modified organism (*e*.*g*. an organism whose cells comprise a codon-optimized nucleotide sequence encoding a M-SmallCas9 polypeptide or variant thereof) can be any organism including for example, a plant; algae; an invertebrate (*e*.*g*. a cnidarian, an echinoderm, a worm, a fly, *etc.);* a vertebrate (*e.g.* a fish (*e.g.* zebrafish, puffer fish, gold fish, *etc.*)*,* an amphibian (*e.g.* salamander, frog, *etc.),* a reptile, a bird, a mammal, *etc*.); an ungulate (*e.g.* a goat, a pig, a sheep, a cow, *etc*.); *a* rodent *(e.g.* a mouse, a rat, a hamster, a guinea pig); a lagomorpha (*e.g.* a rabbit); *etc.*

The active portion may be the RNase domains. The active portions may be the DNase domain.

### Transgenic non-human animals

As described above, a nucleic acid (*e.g.* a codon-optimized nucleotide sequence encoding a M-SmallCas9 polypeptide or variant thereof) or a recombinant expression vector may used as a transgene to generate a transgenic animal that produces a M-SmallCas9 polypeptide or variant thereof. Thus, the present disclosure further provides a transgenic non-human animal, which animal comprises a transgene comprising a nucleic acid comprising a codon-optimized nucleotide sequence encoding a M-SmallCas9 polypeptide or variant thereof, as described above. The genome of the transgenic non-human animal may comprise a codon-optimized nucleotide sequence encoding a M-SmallCas9 polypeptide or variant thereof. The transgenic non-human animal may be homozygous for the genetic modification. The transgenic non-human animal may be heterozygous for the genetic modification. The transgenic non-human animal may be a vertebrate, for example, a fish (*e*.*g*. zebra fish, gold fish, puffer fish, cave fish, *etc*.)*,* an amphibian (frog, salamander, *etc.*), a bird (*e.g.* chicken, turkey, *etc.),* a reptile (*e.g.* snake, lizard, *etc.),* a mammal (*e.g.* an ungulate, *e.g.* a pig, a cow, a goat, a sheep, *etc.;* a lagomorph (*e.g.* a rabbit); a rodent (*e.g.* a rat, a mouse); a nonhuman primate; *etc.), etc.*

The nucleic acid may be an exogenous nucleic acid comprising a codon-optimized nucleotide sequence encoding a M-SmallCas9 polypeptide or variant thereof.The exogenous nucleic acid may be under the control of (*e.g.,* operably linked to) an unknown promoter (*e.g.* when the nucleic acid randomly integrates into a host cell genome) or can be under the control of (*e*.*g*., operably linked to) a known promoter. Suitable known promoters can be any known promoter and include constitutively active promoters (*e.g.* CMV promoter), inducible promoters (*e.g.* heat shock promoter, Tetracycline-regulated promoter, Steroid-regulated promoter, Metal-regulated promoter, estrogen receptor-regulated promoter, *etc.),* spatially restricted and/or temporally restricted promoters (*e.g.* a tissue specific promoter, a cell type specific promoter, *etc.*)*, etc.*

### Introducing nucleic acids into a host cell

The methods of the disclosure may include introducing into a host cell (or a population of host cells) one or more nucleic acids comprising a nucleotide sequence encoding a guide RNA and/or a codon-optimized nucleotide sequence encoding a M-SmallCas9 polypeptide or variant thereof. A cell comprising a target DNA may be *in vitro.* A cell comprising a target DNA may be *in vivo* (not claimed). The nucleotide sequence encoding a guide RNA and/or a M-SmallCas9 polypeptide or variant thereof may be operably linked to an inducible promoter. A nucleotide sequence encoding a guide RNA and/or a M-SmallCas9 polypeptide or variant thereof may be operably linked to a constitutive promoter.

A guide RNA, or a nucleic acid comprising a nucleotide sequence encoding same, can be introduced into a host cell by any of a variety of well-known methods. Similarly, where a method involves introducing into a host cell a nucleic acid comprising a codon-optimized nucleotide sequence encoding a M-SmallCas9 polypeptide or variant thereof, such a nucleic acid can be introduced into a host cell by any of a variety of well-known methods. Guide polynucleotides (RNA or DNA) and/or M-SmallCas9 polynucleotides (RNA or DNA) can be delivered by viral or non-viral delivery vehicles known in the art.

Methods of introducing a nucleic acid into a host cell are known in the art, and any known method can be used to introduce a nucleic acid (*e*.*g*. an expression construct) into a stem cell or progenitor cell. Suitable methods include, *e*.*g*. viral or bacteriophage infection, transfection, conjugation, protoplast fusion, lipofection, electroporation, calcium phosphate precipitation, polyethyleneimine (PEI)-mediated transfection, DEAE-dextran mediated transfection, liposome-mediated transfection, particle gun technology, calcium phosphate precipitation, direct micro injection, nanoparticle-mediated nucleic acid delivery (see, *e*.*g*. Panyam et., al Adv Drug Deliv Rev. 2012 Sep 13. pii: 50169-409X(12)00283-9. doi: 10.1016/j.addr.2012.09.023 ), and the like, including but not limiting to exosome delivery.

Polynucleotides may be delivered by non-viral delivery vehicles including, but not limited to, nanoparticles, liposomes, ribonucleoproteins, positively charged peptides, small molecule RNA-conjugates, aptamer-RNA chimeras, and RNA-fusion protein complexes. Some exemplary non-viral delivery vehicles are described in Peer and Lieberman, Gene Therapy, 18: 1127-1133 (2011) (which focuses on non-viral delivery vehicles for siRNA that are also useful for delivery of other polynucleotides).

Suitable systems and techniques for delivering a nucleic acid of the disclosure (e.g., mRNA and sgRNA) for gene editing is include lipid nanoparticles (LNPs). As used herein, the term "lipid nanoparticles" includes liposomes irrespective of their lamellarity, shape or structure and lipoplexes as described for the introduction of nucleic acids and/or polypeptides into cells. These lipid nanoparticles can be complexed with biologically active compounds (e.g., nucleic acids and/or polypeptides) and are useful as in vivo delivery vehicles. In general, any method known in the art can be applied to prepare the lipid nanoparticles comprising one or more nucleic acids of the present disclosure and to prepare complexes of biologically active compounds and said lipid nanoparticles. Examples of such methods are widely disclosed, e.g. in Biochim Biophys Acta 1979, 557:9; Biochim et Biophys Acta 1980, 601:559; Liposomes: A practical approach (Oxford University Press, 1990); Pharmaceutica Acta Helvetiae 1995, 70:95; Current Science 1995, 68:715; Pakistan Journal of Pharmaceutical Sciences 1996, 19:65; Methods in Enzymology 2009, 464:343). Particularly suitable systems and techniques for preparing LNP formulations comprising one or more nucleic acids and/or polypeptides of the present disclosure include, but are not limited to, those developed by Intellia (see e.g., WO2017173054A1), Alnylam (see, e.g., WO2014008334A1), Modernatx (see., e.g., WO2017070622A1 and WO2017099823A1), TranslateBio, Acuitas (see, e.g., WO2018081480A1), Genevant Sciences, Arbutus Biopharma, Tekmira, Arcturus, Merck (see, e.g., WO2015130584A2), Novartis (see, e.g., WO2015095340A1), and Dicerna.

Suitable nucleic acids comprising nucleotide sequences encoding a guide RNA and/or a M-SmallCas9 polypeptide or variant thereof include expression vectors, where an expression vector comprising a nucleotide sequence encoding a guide. The expression vector may be a viral construct, *e.g.* a recombinant adeno-associated virus construct (see, *e.g.* US Patent No. 7,078,387), a recombinant adenoviral construct, a recombinant lentiviral construct, a recombinant retroviral construct, *etc.* Suitable expression vectors include, but are not limited to, viral vectors (*e.g*. viral vectors based on vaccinia virus; poliovirus; adenovirus (see, *e.g.* Li et al., Invest Opthalmol Vis Sci 35:2543 2549, 1994; Borras et al., Gene Ther 6:515 524, 1999; Li and Davidson, PNAS 92:7700 7704, 1995; Sakamoto et al., H Gene Ther 5:10881097, 1999; WO 94/12649, WO 93/03769; WO 93/19191; WO 94/28938; WO 95/11984 and WO 95/00655); adeno-associated virus (see, *e.g.* Ali et al., Hum Gene Ther 9:81 86,1998, Flannery et al., PNAS 94:6916 6921, 1997; Bennett et al., Invest Opthalmol Vis Sci 38:2857 2863, 1997; Jomary et al., Gene Ther 4:683-690, 1997, Rolling et al., Hum Gene Ther 10:641 648, 1999; Ali et al., Hum Mol Genet 5:591 594, 1996, Srivastava in WO 93/09239, Samulski et al., J. Vir. (1989) 63:3822-3828; Mendelson et al., Viral. (1988) 166:154-165; and Flotte et al., PNAS (1993) 90:10613-10617); SV40; herpes simplex virus; human immunodeficiency virus (see, *e.g.* Miyoshi et al., PNAS 94:10319 23, 1997; Takahashi et al., J Virol 73:7812 7816, 1999); a retroviral vector (*e*.*g*. Murine Leukemia Virus, spleen necrosis virus, and vectors derived from retroviruses such as Rous Sarcoma Virus, Harvey Sarcoma Virus, avian leukosis virus, a lentivirus, human immunodeficiency virus, myeloproliferative sarcoma virus, and mammary tumor virus); and the like.

A recombinant adeno-associated virus (AAV) vector may be used for delivery. Known techniques to produce rAAV particles in the art is to provide a cell with a polynucleotide to be delivered between two AAV invert terminal repeats (ITRs), AAV rep and cap genes and helper virus functions. Production of rAAV requires that the following components are present within a single cell (denoted herein as a packaging cell): a polynucleotide of interest between two ITRs, AAV rep and cap genes separate from (i.e., not in) the AAV genome, and helper virus functions. The AAV rep and cap genes may be from any AAV serotype for which recombinant virus can be derived and may be from a different serotype of AAV than that of ITRs on a packaged polynucleotide, including, but not limited to, AAV serotypes AAV-1, AAV-2, AAV-3, AAV-4, AAV-5, AAV-6, AAV-7, AAV-8, AAV-9, AAV-10, AAV-11, AAV-12, AAV-13 and AAV rh.74. Production of pseudotyped rAAV is disclosed in, for example, WO 01/83692.

| AAV Serotype | Genbank Accession No. |
|---|---|
| AAV-1 | NC_002077.1 |
| AAV-2 | NC_001401.2 |
| AAV-3 | NC_001725.1 |
| AAV-38 | AF028705.1 |
| AAV-4 | NC_001829.1 |
| AAV-5 | NC_006152.1 |
| AAV-6 | AF028704.1 |
| AAV-7 | NC_006260.1 |
| AAV-8 | NC_006261.1 |
| AAV-9 | AX753250.1 |
| AAV-10 | AY631965.1 |
| AAV-11 | AY631966.1 |
| AAV-12 | 00813647.1 |
| AAV-13 | EU285562.1 |

A method of generating a packaging cell is to create a cell line that stably expresses all the necessary components for AAV particle production. For example, a plasmid (or multiple plasmids) comprising a polynucleotide of interest between AAV ITRs, AAV rep and cap genes separate from the AAV genome, and a selectable marker, such as a neomycin resistance gene, are integrated into the genome of a cell. AAV genomes have been introduced into bacterial plasmids by procedures such as GC tailing (Samulski et al., 1982, Proc. Natl. Acad. Sci. USA, 79:2077-2081), addition of synthetic linkers containing restriction endonuclease cleavage sites (Laughlin et al., 1983, Gene, 23:65-73) or by direct, blunt-end ligation (Senapathy & Carter, 1984, J. Bioi. Chem., 259:4661-4666). The packaging cell line is then infected with a helper virus such as adenovirus. The advantages of this method are that the cells are selectable and are suitable for large-scale production of rAAV. Other examples of suitable methods employ adenovirus or baculovirus rather than plasmids to introduce rAAV genomes and/or rep and cap genes into packaging cells.

General principles of rAAV production are reviewed in, for example, Carter, 1992, Current Opinions in Biotechnology, 1533-539; and Muzyczka, 1992, Curr. Topics in Microbial. and Immunol., 158:97-129). Various approaches are described in Ratschin et al., Mol. Cell. Biol. 4:2072 (1984); Hermonat et al., Proc. Natl. Acad. Sci. USA, 81:6466 (1984); Tratschin et al., Mol. Cell. Biol. 5:3251 (1985); Mclaughlin et al., J. Virol., 62:1963 (1988); and Lebkowski et al., 1988 Mol. Cell. Biol., 7:349 (1988). Samulski et al. (1989, J. Virol., 63:3822-3828); US Patent No. 5,173,414; WO 95/13365 and corresponding US Patent No. 5,658.776; WO 95/13392; WO 96/17947; PCT/US98/18600; WO97/09441 (PCT/US96/14423); WO 97/08298 (PCT/US96/13872); WO 97/21825 (PCT/US96/20777); WO 97/06243 (PCT/FR96/01064); WO 99/11764; Perrin et al. (1995) Vaccine 13:1244-1250; Paul et al. (1993) Human Gene Therapy 4:609-615; Clark et al. (1996) Gene Therapy 3:1124-1132; US Patent. No. 5,786,211; US Patent No. 5,871,982; and US Patent. No. 6,258,595.

AAV vector serotypes used for transduction are dependent on target cell types. For example, the following exemplary cell types are known to be transduced by the indicated AAV serotypes among others.

| Tissue/Cell Type | Serotype |
|---|---|
| Liver | AAV8, AAV9 |
| Skeletal muscle | AAV1, AAV7, AAV6, AAV8, AAV9 |
| Central nervous system | AAV5, AAV1, AAV4 |
| RPE | AAV5, AAV4 |
| Photoreceptor cells | AAV5 |
| Lung | AAV9 |
| Heart | AAV8 |
| Pancreas | AAV8 |
| Kidney | AAV2 |

Numerous suitable expression vectors are known to those of skill in the art, and many are commercially available. The following vectors are provided by way of example; for eukaryotic host cells: pXT1, pSG5 (Stratagene), pSVK3, pBPV, pMSG, and pSVLSV40 (Pharmacia). However, any other vector may be used so long as it is compatible with the host cell.

Depending on the host/vector system utilized, any of a number of suitable transcription and translation control elements, including constitutive and inducible promoters, transcription enhancer elements, transcription terminators, *etc.* may be used in the expression vector (see *e.g.* Bitter et al. (1987) Methods in Enzymology, 153:516-544).

A guide RNA and/or a M-SmallCas9 polypeptide or variant thereof may be provided as RNA. In such cases, the guide RNA and/or the RNA encoding the M-SmallCas9 polypeptide or variant thereof can be produced by direct chemical synthesis or may be transcribed *in vitro* from a DNA encoding the guide RNA. Methods of synthesizing RNA from a DNA template are well known in the art. The guide RNA and/or the RNA encoding the M-SmallCas9 polypeptide or variant thereof may be synthesized in *vitro* using an RNA polymerase enzyme (*e.g.* T7 polymerase, T3 polymerase, SP6 polymerase, *etc.*)*.* Once synthesized, the RNA may directly contact a target DNA or may be introduced into a cell by any of the well-known techniques for introducing nucleic acids into cells (*e*.*g*. microinjection, electroporation, transfection, *etc*.)*.*

Nucleotides encoding a guide RNA (introduced either as DNA or RNA) and/or a M-SmallCas9 polypeptide or variant thereof (introduced as DNA or RNA) and/or a donor polynucleotide may be provided to the cells using well-developed transfection techniques; see, *e*.*g*. Angel and Yanik (2010) PLoS ONE 5(7): e 11756, and the commercially available TransMessenger^{®} reagents from Qiagen, Stemfect^{™} RNA Transfection Kit from Stemgent, and TransiT^{®}-mRNA Transfection Kit from Mims Bio. See also Beumer et al. (2008) Efficient gene targeting in Drosophila by direct embryo injection with zinc-finger nucleases. PNAS 105(50):19821-19826. In addition or alternatively, nucleic acids encoding a guide RNA and/or a M-SmallCas9 polypeptide or variant thereof and/or a M-SmallCas9 fusion polypeptide or variant thereof and/or a donor polynucleotide may be provided on DNA vectors. Many vectors, *e.g.,* plasmids, cosmids, minicircles, phage, viruses, *etc.,* useful for transferring nucleic acids into target cells are available. The vectors comprising the nucleic acid(s) may be maintained episomally, *e.g.* as plasmids, minicircle DNAs, viruses such cytomegalovirus, adenovirus, *etc.,* or they may be integrated into the target cell genome, through homologous recombination or random integration, *e.g.* retrovirus-derived vectors such as MMLV, HIV-1, ALV, *etc.*

Vectors may be provided directly to the cells. In other words, the cells are contacted with vectors comprising the nucleic acid encoding guide RNA and/or a M-SmallCas9 polypeptide or variant thereof and/or a M-SmallCas9 fusion polypeptide or variant thereof and/or a donor polynucleotide such that the vectors are taken up by the cells. Methods for contacting cells with nucleic acid vectors that are plasmids, including electroporation, calcium chloride transfection, microinjection, and lipofection are well known in the art. For viral vector delivery, the cells are contacted with viral particles comprising the nucleic acid encoding a guide RNA and/or a M-SmallCas9 polypeptide or variant thereof and/or a M-SmallCas9 fusion polypeptide or variant thereof and/or a donor polynucleotide. Retroviruses, for example, lentiviruses, are particularly suitable to the method of the present disclosure. Commonly used retroviral vectors are "defective", *e*.*g*., unable to produce viral proteins required for productive infection. Rather, replication of the vector requires growth in a packaging cell line. To generate viral particles comprising nucleic acids of interest, the retroviral nucleic acids comprising the nucleic acid are packaged into viral capsids by a packaging cell line. Different packaging cell lines provide a different envelope protein (ecotropic, amphotropic or xenotropic) to be incorporated into the capsid, this envelope protein determining the specificity of the viral particle for the cells (ecotropic for murine and rat; amphotropic for most mammalian cell types including human, dog and mouse; and xenotropic for most mammalian cell types except murine cells). The appropriate packaging cell line may be used to ensure that the cells are targeted by the packaged viral particles. Methods of introducing the retroviral vectors comprising the nucleic acid encoding the reprogramming factors into packaging cell lines and of collecting the viral particles that are generated by the packaging lines are well known in the art. Nucleic acids can also be introduced by direct microinjection (*e*.*g*. injection of RNA into a zebrafish embryo).

Vectors used for providing the nucleic acids encoding guide RNA and/or a M-SmallCas9 polypeptide or variant thereof and/or a M-SmallCas9 fusion polypeptide or variant thereof and/or a donor polynucleotide to the cells will generally comprise suitable promoters for driving the expression, that is, transcriptional activation, of the nucleic acid of interest. In other words, the nucleic acid of interest will be operably linked to a promoter. This may include ubiquitously active promoters, for example, the CMV-13-actin promoter, or inducible promoters, such as promoters that are active in particular cell populations or that respond to the presence of drugs such as tetracycline. By transcriptional activation, it is intended that transcription will be increased above basal levels in the target cell by at least 10 fold, by at least 100 fold, more typically by at least 1000 fold. In addition, vectors used for providing a guide RNA and/or a M-SmallCas9 polypeptide or variant thereof and/or a M-SmallCas9 fusion polypeptide or variant thereof and/or a donor polynucleotide to the cells may include nucleic acid sequences that code for selectable markers in the target cells, so as to identify cells that have taken up the guide RNA and/or a M-SmallCas9 polypeptide or variant thereof and/or a M-SmallCas9 fusion polypeptide or variant thereof and/or a donor polynucleotide.

A guide RNA and/or a M-SmallCas9 polypeptide or variant thereof and/or a M-SmallCas9 fusion polypeptide or variant thereof may instead be used to contact DNA or introduced into cells as RNA. Methods of introducing RNA into cells are known in the art and may include, for example, direct injection, transfection, or any other method used for the introduction of DNA. A M-SmallCas9 polypeptide or variant thereof may instead be provided to cells as a polypeptide. Such a polypeptide may optionally be fused to a polypeptide domain that increases solubility of the product. The domain may be linked to the polypeptide through a defined protease cleavage site, *e.g.* a TEV sequence, which is cleaved by TEV protease. The linker may also include one or more flexible sequences, *e*.*g*. from 1 to 10 glycine residues. The cleavage of the fusion protein may be performed in a buffer that maintains solubility of the product, *e*.*g*. in the presence of from 0.5 to 2 M urea, in the presence of polypeptides and/or polynucleotides that increase solubility, and the like. Domains of interest include endosomolytic domains, *e.g.* influenza HA domain; and other polypeptides that aid in production, *e.g.* IF2 domain, GST domain, GRPE domain, and the like. The polypeptide may be formulated for improved stability. For example, the peptides may be PEGylated, where the polyethyleneoxy group provides for enhanced lifetime in the blood stream.

Additionally or alternatively, the M-SmallCas9 polypeptide or variant thereof may be fused to a polypeptide permeant domain to promote uptake by the cell. A number of permeant domains are known in the art and may be used in the non-integrating polypeptides of the present disclosure, including peptides, peptidomimetics, and non-peptide carriers. For example, a permeant peptide may be derived from the third alpha helix of Drosophila melanogaster transcription factor Antennapaedia, referred to as penetratin, which comprises the amino acid sequence RQIKIWFQNRRMKWKK (this sequence is not a disclosure under this patent application). As another example, the permeant peptide comprises the HIV-1 tat basic region amino acid sequence, which may include, for example, amino acids 49-57 of naturally occurring tat protein.

Other permeant domains include poly-arginine motifs, for example, the region of amino acids 34-56 of HIV-1 rev protein, nona-arginine, acta-arginine, and the like. (See, for example, Futaki et al. (2003) Curr Protein Pept Sci. 2003 Apr; 4(2): 87-9 and 446; and Wender et al. (2000) Proc. Natl. Acad. Sci. U.S.A. 2000 Nov. 21; 97(24):13003-8; published US Patent Application Publications Nos. 20030220334; 20030083256; 20030032593; and 20030022831). The nona-arginine (R9) sequence is one of the more efficient PTDs that have been characterized (Wender *et al.* 2000; Uemura *et al.* 2002). The site at which the fusion is made may be selected in order to optimize the biological activity, secretion or binding characteristics of the polypeptide. The optimal site may be determined by routine experimentation. The polypeptide permeant domain may be chemically modified in order to increase the bioavailability of the PTD. Exemplary modifications are disclosed in Expert Opin Drug Deliv. 2009 Nov;6(11):1195-205.

Generally, an effective amount of the guide RNA and/or M-SmallCas9 polypeptide or variant thereof and/or donor polynucleotide is provided to the target DNA or cells to induce targeted modification. An effective amount of the guide RNA and/or M-SmallCas9 polypeptide or variant thereof and/or donor polynucleotide is the amount to induce a 2-fold increase or more in the amount of targeted modification observed with the gRNA relative to a negative control, e.g., a cell contacted with an empty vector or irrelevant polypeptide. That is to say, an effective amount or dose of the guide RNA and/or M-SmallCas9 polypeptide or variant thereof and/or donor polynucleotide will induce a 2-fold increase, a 3-fold increase, a 4-fold increase or more in the amount of target modification observed at a target DNA region, a 5-fold increase, a 6-fold increase or more, sometimes a 7-fold or 8-fold increase or more in the amount of recombination observed, *e*.*g*., an increase of 10-fold, 50-fold, or 100-fold or more, an increase of 200-fold, 500-fold, 700-fold, or 1000-fold or more, *e.g.,* a 5000-fold, or 10,000-fold increase in the amount of recombination observed. The amount of target modification may be measured by any suitable method. For example, a split reporter construct comprising complementary sequence to the spacer of the guide RNA flanked by homologous sequences that, when recombined, will reconstitute a nucleic acid encoding an active reporter may be cotransfected into the cells, and the amount of reporter protein assessed after contact with the guide RNA and/or M-SmallCas9 polypeptide or variant thereof and/or donor polynucleotide, *e.g.* 2 hours, 4 hours, 8 hours, 12 hours, 24 hours, 36 hours, 48 hours, 72 hours or more after contact with the guide RNA and/or M-SmallCas9 polypeptide or variant thereof and/or donor polynucleotide. As another, more sensitivity assay, for example, the extent of recombination at a genomic DNA region of interest comprising target DNA sequences may be assessed by PCR or Southern hybridization of the region after contact with a guide RNA and/or M-SmallCas9 polypeptide or variant thereof and/or donor polynucleotide, *e.g.* 2 hours, 4 hours, 8 hours, 12 hours, 24 hours, 36 hours, 48 hours, 72 hours or more after contact with the guide RNA and/or M-SmallCas9 polypeptide or variant thereof and/or donor polynucleotide.

Contacting the cells with a guide RNA and/or M-SmallCas9 polypeptide or variant thereof and/or donor polynucleotide may occur in any culture media and under any culture conditions that promote the survival of the cells. For example, cells may be suspended in any appropriate nutrient medium that is suitable, such as Iscove's modified DMEM or RPMI1640, supplemented with fetal calf serum or heat inactivated fetal bovine serum (about 5-10%), L-glutamine, a thiol, particularly 2-mercaptoethanol, and antibiotics, *e*.*g*. penicillin and streptomycin. The culture may contain growth factors to which the cells are responsive. Growth factors, as defined herein, are molecules capable of promoting survival, growth and/or differentiation of cells, either in culture or in the intact tissue, through specific effects on a transmembrane receptor. Growth factors include polypeptides and non-polypeptide factors. Conditions that promote the survival of cells are generally permissive of nonhomologous end joining and homology-directed repair. In applications in which it is desirable to insert a polynucleotide sequence into a target DNA sequence, a polynucleotide comprising a donor sequence to be inserted is also provided to the cell. By a "donor sequence" or "donor polynucleotide" it is meant a nucleic acid sequence to be inserted at the cleavage site induced by a M-SmallCas9 polypeptide or variant thereof. The donor polynucleotide will contain sufficient sequence homology to the flanking genomic regions of the cleavage site, *e.g.* 70%, 80%, 85%, 90%, 95%, or 100% sequence identity with the nucleotide sequences flanking the cleavage site, *e*.*g*. within about 50 bases or less of the cleavage site, *e*.*g*. within about 30 bases, within about 15 bases, within about 10 bases, within about 5 bases, or immediately flanking the cleavage site, to support homology-directed repair between it and the genomic sequence to which it bears homology. Approximately 25, 50, 100, or 200 nucleotides, or more than 200 nucleotides, of homologous sequences between a donor and a genomic sequence (or any integral value between 10 and 200 nucleotides, or more) will support homology-directed repair. Donor sequences can be of any length, *e*.*g*. 10 nucleotides or more, 50 nucleotides or more, 100 nucleotides or more, 250 nucleotides or more, 500 nucleotides or more, 1000 nucleotides or more, 5000 nucleotides or more, *etc.*

The donor sequence is generally not identical to the genomic sequence that it replaces. Rather, the donor sequence may contain at least one or more single base substitutions, insertions, deletions, inversions or rearrangements with respect to the genomic sequence, so long as sufficient sequence identity is present to support homology-directed repair. The donor sequence may comprise a non-homologous sequence flanked by two regions homologous to the target DNA region (also referred to as homology arms), such that homology- directed repair between the target DNA region and the two flanking homology arms results in insertion of the non-homologous sequence at the target region. Donor sequences may also comprise a vector backbone containing sequences that are not homologous to the DNA region of interest and that are not intended for insertion into the DNA region of interest. Generally, the homologous region(s) of a donor sequence will have at least 50% sequence identity to a genomic sequence with which recombination is desired.According to the discosure, 60%, 70%, 80%, 90%, 95%, 98%, 99%, or 99.9% sequence identity may be present. Any value between 1% and 100% sequence identity can be present, depending upon the length of the donor polynucleotide. The donor sequence may comprise certain sequence differences as compared to the genomic sequence, *e.g.* restriction sites, nucleotide polymorphisms, selectable markers (*e.g.* drug resistance genes, fluorescent proteins, enzymes *etc*.), *etc.,* which may be used to assess for successful insertion of the donor sequence at the cleavage site or in some cases may be used for other purposes (*e*.*g*. to signify expression at the targeted genomic locus). If located in a coding region, such nucleotide sequence differences may not change the amino acid sequence, or may make amino acid changes which do not substantially affect the structure or function of the protein. Alternatively, these sequences differences may include flanking recombination sequences such as FLPs, loxP sequences, or the like, that can be activated at a later time for removal of the marker sequence.

The donor sequence may be provided to the cell as single-stranded DNA, single-stranded RNA, double-stranded DNA, or double-stranded RNA. It may be introduced into a cell in linear or circular form. If introduced in linear form, the ends of the donor sequence may be protected (*e*.*g*. from exonucleolytic degradation) by methods known to those of skill in the art. For example, one or more dideoxynucleotide residues are added to the 3' terminus of a linear molecule and/or self-complementary oligonucleotides are ligated to one or both ends. See, for example, Chang et al. (1987) Proc. Natl. Acad. Sci. USA 84:4959-4963; Nehls et al. (1996) Science 272:886-889. Additional methods for protecting exogenous polynucleotides from degradation include, but are not limited to, addition of terminal amino group(s) and the use of modified internucleotide linkages such as, for example, phosphorothioates, phosphoramidates, and O-methyl ribose or deoxyribose residues. As an alternative to protecting the termini of a linear donor sequence, additional lengths of sequence may be included outside of the homology arms that can be degraded without impacting recombination. A donor sequence can be introduced into a cell as part of a vector molecule having additional sequences such as, for example, replication origins, promoters and genes encoding antibiotic resistance. Moreover, donor sequences can be introduced as naked (*e*.*g*. unmodified) nucleic acid, as nucleic acid complexed with an agent such as a liposome or poloxamer, or can be delivered by viruses (*e*.*g*. adenovirus, AAV), as described above for nucleic acids encoding a guide RNA and/or M-SmallCas9 polypeptide or variant thereof and/or donor polynucleotide.

Following the methods described above, a DNA region of interest may be cleaved and modified, e.g., "genetically modified", ex vivo. As when a selectable marker has been inserted into the DNA region of interest, the population of cells may be enriched for those comprising the genetic modification by separating the genetically modified cells from the remaining population. Prior to enriching, the "genetically modified" cells may make up only about 1% or more (*e.g.* 2% or more, 3% or more, 4% or more, 5% or more, 6% or more, 7% or more, 8% or more, 9% or more, 10% or more, 15% or more, or 20% or more) of the cellular population. Separation of "genetically modified" cells may be achieved by any suitable separation technique appropriate for the selectable marker used. For example, if a fluorescent marker has been inserted, cells may be separated by fluorescence activated cell sorting, whereas if a cell surface marker has been inserted, cells may be separated from the heterogeneous population by affinity separation techniques, *e*.*g*. magnetic separation, affinity chromatography, "panning" with an affinity reagent attached to a solid matrix, or other suitable technique. Techniques providing accurate separation include fluorescence activated cell sorters, which can have varying degrees of sophistication. Such as multiple color channels, low angle and obtuse light scattering detecting channels, impedance channels, *etc.* The cells may be selected against dead cells by employing dyes associated with dead cells (*e*.*g*. propidium iodide). Any technique may be employed which is not unduly detrimental to the viability of the genetically modified cells. Cell compositions that are highly enriched for cells comprising modified DNA are achieved in this manner. By "highly enriched", it is meant that the genetically modified cells will be 70% or more, 75% or more, 80% or more, 85% or more, 90% or more of the cell composition, for example, about 95% or more, or 98% or more of the cell composition. In other words, the composition may be a substantially pure composition of genetically modified cells.

Genetically modified cells produced by the methods described herein may be used immediately. In addition or alternatively, the cells may be frozen at liquid nitrogen temperatures and stored for long periods of time, being thawed and capable of being reused. In such cases, the cells will generally be frozen in 10% dimethylsulfoxide (DMSO), 50% serum, 40% buffered medium, or some other such solution as is commonly used in the art to preserve cells at such freezing temperatures, and thawed in a manner as commonly known in the art for thawing frozen cultured cells.

The genetically modified cells may be cultured *in vitro* under various culture conditions. The cells may be expanded in culture, *e*.*g*., grown under conditions that promote their proliferation. Culture medium may be liquid or semi-solid, *e.g.* containing agar, methylcellulose, *etc.* The cell population may be suspended in an appropriate nutrient medium, such as Iscove's modified DMEM or RPMI 1640, normally supplemented with fetal calf serum (about 5-10%), L-glutamine, a thiol, particularly 2-mercaptoethanol, and antibiotics, *e.g*. penicillin and streptomycin. The culture may contain growth factors to which the respective cells are responsive. Growth factors, as defined herein, are molecules capable of promoting survival, growth and/or differentiation of cells, either in culture or in the intact tissue, through specific effects on a transmembrane receptor. Growth factors include polypeptides and non-polypeptide factors. Cells that have been genetically modified in this way may be transplanted to a subject for purposes such as gene therapy, *e*.*g*. to treat a disease or as an antiviral, antipathogenic, or anticancer therapeutic, for the production of genetically modified organisms in agriculture, or for biological research. The subject may be a neonate, a juvenile, or an adult. Of particular interest are mammalian subjects. Mammalian species that may be treated with the present methods include canines and felines; equines; bovines; ovines; *etc.* and primates, particularly humans. Animal models, particularly small mammals (*e*.*g*. mouse, rat, guinea pig, hamster, lagomorpha (*e.g.* rabbit), *etc*.) may be used for experimental investigations.

Cells may be provided to the subject alone or with a suitable substrate or matrix, *e*.*g*. to support their growth and/or organization in the tissue to which they are being transplanted. Generally, at least 1x10³ cells will be administered, for example 5x10³ cells, 1x10⁴ cells, 5x10⁴ cells, 1x10⁵ cells, 1 x 10⁶ cells or more. The cells may be introduced to the subject via any of the following routes: parenteral, subcutaneous, intravenous, intracranial, intraspinal, intraocular, or into spinal fluid. The cells may be introduced by injection, catheter, or the like. Examples of methods for local delivery, that is, delivery to the site of injury, include, *e.g.* through an Ommaya reservoir, *e.g.* for intrathecal delivery (see *e.g.* US Patent Nos. 5,222,982 and 5,385,582); by bolus injection, *e.g.* by a syringe, *e.g.* into a joint; by continuous infusion, *e.g.* by cannulation, *e.g.* with convection (see *e.g.,* US Application No. 20070254842); or by implanting a device upon which the cells have been reversibly affixed (see *e.g.* US Application Nos. 20080081064 and 20090196903). Cells may also be introduced into an embryo (*e.g.* a blastocyst) for the purpose of generating a transgenic animal (*e.g.* a transgenic mouse, not claimed).

A nucleotide sequence encoding a guide RNA and/or a M-SmallCas9 polypeptide or variant thereof may be operably linked to a control element, *e*.*g*. a transcriptional control element, such as a promoter. The transcriptional control element is generally functional in either a eukaryotic cell, such as a mammalian cell (*e.g.,* human cell); or a prokaryotic cell (*e.g.* bacterial or archaeal cell). A nucleotide sequence encoding a guide RNA and/or a M-SmallCas9 polypeptide or variant thereof may be operably linked to multiple control elements that allow expression of the nucleotide sequence encoding a guide RNA and/or a M-SmallCas9 polypeptide or variant thereof in both prokaryotic and eukaryotic cells.

A promoter can be a constitutively active promoter (*e*.*g*., a promoter that is constitutively in an active "ON" state), it may be an inducible promoter (*e*.*g*., a promoter whose state, active/"ON" or inactive/"OFF", is controlled by an external stimulus, *e*.*g*. the presence of a particular temperature, compound, or protein.), it may be a spatially restricted promoter (*e*.*g*., transcriptional control element, enhancer, *etc*.)(*e.g.* tissue specific promoter, cell type specific promoter, *etc.),* and it may be a temporally restricted promoter (*e*.*g*., the promoter is in the "ON" state or "OFF" state during specific stages of embryonic development or during specific stages of a biological process, *e*.*g*. hair follicle cycle in mice).

Suitable promoters can be derived from viruses and can therefore be referred to as viral promoters, or they can be derived from any organism, including prokaryotic or eukaryotic organisms. Suitable promoters can be used to drive expression by any RNA polymerase (*e*.*g*. pol I, pol II, pol III). Exemplary promoters include, but are not limited to the SV40 early promoter, mouse mammary tumor virus long terminal repeat (LTR) promoter; adenovirus major late promoter (Ad MLP); a herpes simplex virus (HSV) promoter, a cytomegalovirus (CMV) promoter such as the CMV immediate early promoter region (CMVIE), a Rous sarcoma virus (RSV) promoter, a human U6 small nuclear promoter (U6) (Miyagishi et al., Nature Biotechnology 20, 497-500 (2002)), an enhanced U6 promoter *(e.g.,* Xia et al., Nucleic Acids Res. 2003 Sep 1;31(17)), a human H1 promoter (H1), and the like.

Examples of inducible promoters include, but are not limited to T7 RNA polymerase promoter, T3 RNA polymerase promoter, Isopropyl-beta-D-thiogalactopyranoside (IPTG)-regulated promoter, lactose induced promoter, heat shock promoter, Tetracycline-regulated promoter (*e*.*g*. Tet- ON, Tet-OFF, *etc.),* Steroid-regulated promoter, Metal-regulated promoter, estrogen receptor- regulated promoter, *etc.* Inducible promoters can therefore be regulated by molecules including, but not limited to, doxycycline; RNA polymerase, *e*.*g*. T7 RNA polymerase; an estrogen receptor; an estrogen receptor fusion; *etc.*

The promoter may be a spatially restricted promoter (*e*.*g*., cell type specific promoter, tissue specific promoter, *etc.*) such that in a multi-cellular organism, the promoter is active (*e.g.,* "ON") in a subset of specific cells. Spatially restricted promoters may also be referred to as enhancers, transcriptional control elements, control sequences, *etc.* Any suitable spatially restricted promoter may be used and the choice of suitable promoter (*e*.*g*. a brain specific promoter, a promoter that drives expression in a subset of neurons, a promoter that drives expression in the germline, a promoter that drives expression in the lungs, a promoter that drives expression in muscles, a promoter that drives expression in islet cells of the pancreas, *etc.*) will depend on the organism. For example, various spatially restricted promoters are known for plants, flies, worms, mammals, mice, *etc.* Thus, a spatially restricted promoter can be used to regulate the expression of a nucleic acid encoding a M-SmallCas9 polypeptide or variant thereof in a wide variety of different tissues and cell types, depending on the organism. Some spatially restricted promoters are also temporally restricted such that the promoter is in the "ON" state or "OFF" state during specific stages of embryonic development or during specific stages of a biological process (*e*.*g*. hair follicle cycle in mice).

For illustration purposes, examples of spatially restricted promoters include, but are not limited to, neuron-specific promoters, adipocyte-specific promoters, cardiomyocyte-specific promoters, smooth muscle-specific promoters, photoreceptor-specific promoters, *etc.* Neuron-specific spatially restricted promoters include, but are not limited to, a neuron-specific enolase (NSE) promoter (see, *e*.*g*. EMBL HSEN02, X51956); an aromatic amino acid decarboxylase (AADC) promoter; a neurofilament promoter (see, *e.g.* GenBank HUMNFL, L04147); a synapsin promoter (see, *e.g.* GenBank HUMSYNIB, M55301); a thy-1 promoter (see, *e.g.* Chen et al. (1987) Ce/151 :7-19; and Llewellyn, et al. (2010) Nat. Med. 16(10):1161-1166); a serotonin receptor promoter (see, *e.g.* GenBank S62283); a tyrosine hydroxylase promoter (TH) (see, *e.g.* Oh et al. (2009) Gene Ther 16:437; Sasaoka et al. (1992) Mol. Brain Res. 16:274; Boundy et al. (1998) J. Neurosci. 18:9989; and Kaneda et al. (1991) Neuron 6:583-594); a GnRH promoter (see, *e.g.* Radovick et al. (1991) Proc. Natl. Acad. Sci. USA 88:3402-3406); an L7 promoter (see, *e.g.* Oberdick et al. (1990) Science 248:223-226); a DNMT promoter (see, *e.g.* Bartge et al. (1988) Proc. Nat/. Acad. Sci. USA 85:3648-3652); an enkephalin promoter (see, *e.g.* Comb et al. (1988) EMBO J. 17:3793-3805); a myelin basic protein (MBP) promoter; a Ca2+-calmodulin-dependent protein kinase 11-alpha (CamKlla) promoter (see, *e.g.* Mayford et al. (1996) Proc. Nat/. Acad. Sci. USA 93:13250; and Casanova et al. (2001) Genesis 31:37); a CMV enhancer/platelet-derived growth factor-0 promoter (see, *e.g.* Liu et al. (2004) Gene Therapy 11:52-60); and the like.

Adipocyte-specific spatially restricted promoters include, but are not limited to aP2 gene promoter/enhancer, *e.g.* a region from -5.4 kb to +21 bp of a human aP2 gene (see, *e.g.* Tozzo et al. (1997) Endocrinol. 138:1604; Ross et al. (1990) Proc. Natl. Acad. Sci. USA 87:9590; and Pavjani et al. (2005) Nat. Med. 11:797); a glucose transporter-4 (GLUT4) promoter (see, *e.g.* Knight et al. (2003) Proc. Nat/. Acad. Sci. USA 100:14725); a fatty acid translocase (FAT/CD36) promoter (see, *e.g.* Kuriki et al. (2002) Biol. Pharm. Bull. 25:1476; and Sato et al. (2002) J. Biol. Chem. 277:15703); a stearoyl-CoA desaturase-1 (SCD1) promoter (Taboret al. (1999) J. Biol. Chem. 274:20603); a leptin promoter (see, *e.g.* Mason et al. (1998) Endocrinol. 139:1013; and Chen et al. (1999) Biochem. Biophys. Res. Comm. 262:187); an adiponectin promoter (see, *e.g.* Kita et al. (2005) Biochem. Biophys. Res. Comm. 331:484; and Chakrabarti (2010) Endocrinol. 151:2408); an adipsin promoter (see, *e.g.* Platt et al. (1989) Proc. Nat/. Acad. Sci. USA 86:7490); a resistin promoter (see, *e.g.* Seo et al. (2003) Malec. Endocrinol. 17:1522); and the like.

Cardiomyocyte-specific spatially restricted promoters include, but are not limited to control sequences derived from the following genes: myosin light chain-2, a-myosin heavy chain, AE3, cardiac troponin C, cardiac actin, and the like. Franz et al. (1997) Cardiovasc. Res. 35:560-566; Robbins et al. (1995) Ann. N.Y. Acad. Sci. 752:492-505; Linn et al. (1995) Circ. Res. 76:584591; Parmacek et al. (1994) Mol. Cell. Biol. 14:1870-1885; Hunter et al. (1993) Hypertension 22:608-617; and Sartorelli et al. (1992) Proc. Natl. Acad. Sci. USA 89:4047-4051.

Smooth muscle-specific spatially restricted promoters include, but are not limited to an SM22a promoter (see, *e.g.* Akyilrek et al. (2000) Mol. Med. 6:983; and US Patent No. 7,169,874); a smoothelin promoter (see, *e.g.* WO 2001/018048); an α-smooth muscle actin promoter; and the like. For example, a 0.4 kb region of the SM22a promoter, within which lie two CArG elements, has been shown to mediate vascular smooth muscle cell-specific expression (see, *e.g.* Kim, et al. (1997) Mol. Cell. Biol. 17, 2266-2278; Li, et al., (1996) J. Cell Biol. 132, 849-859; and Moessler, et al. (1996) Development 122, 2415-2425).

Photoreceptor-specific spatially restricted promoters include, but are not limited to, a rhodopsin promoter; a rhodopsin kinase promoter (Young et al. (2003) Ophthalmol. Vis. Sci. 44:4076); a beta phosphodiesterase gene promoter (Nicoud et al. (2007) J. Gene Med. 9:1015); a retinitis pigmentosa gene promoter (Nicoud *et al.* (2007) supra); an interphotoreceptor retinoid-binding protein (IRBP) gene enhancer (Nicoud *et al.* (2007) supra); an IRBP gene promoter (Yokoyama et al. (1992) Exp Eye Res. 55:225); and the like.

### Compositions Comprising a Guide RNA

A composition may comprise a guide RNA. The composition can comprise, in addition to the guide RNA, one or more of: a salt, *e.g.* NaCl, MgCl₂, KCI, MgSO₄, *etc.;* a buffering agent, *e.g.* a Tris buffer, N-(2-Hydroxyethyl)piperazine-N'-(2-ethanesulfonic acid) (HEPES), 2-(N-Morpholino)ethanesulfonic acid (MES), MES sodium salt, 3-(N-Morpholino)propanesulfonic acid (MOPS), N-tris[Hydroxymethyl]methyl-3-aminopropanesulfonic acid (TAPS), *etc.;* a solubilizing agent; a detergent, *e.g.* a non-ionic detergent such as Tween-20, *etc.;* a nuclease inhibitor; and the like. For example, a composition may comprise a guide RNA and a buffer for stabilizing nucleic acids.

A guide RNA present in a composition may be pure, *e*.*g*. at least 75%, at least 80%, at least 85%, at least 90%, at least 95%, at least 98%, at least 99%, or more than 99% pure, where"% purity" means that guide RNA is the recited percent free from other macromolecules, or contaminants that may be present during the production of the guide RNA.

### Compositions Comprising A M-SmallCas9 Polypeptide

In some embodiments, provided herein is a composition comprising a M-SmallCas9 polypeptide as defined in the claims. The composition may comprise, in addition to the M-SmallCas9 polypeptide, one or more of: a salt, *e.g.* NaCl, MgCl₂, KCI, MgSO₄, *etc.;* a buffering agent, *e.g.* a Tris buffer, HEPES, MES, MES sodium salt, MOPS, TAPS, *etc.;* a solubilizing agent; a detergent, *e.g.* a non-ionic detergent such as Tween-20, *etc.;* a protease inhibitor; a reducing agent *(e.g.* dithiothreitol); and the like.

A M-SmallCas9 polypeptide or variant thereof present in a composition may be pure, *e*.*g*. at least 75%, at least 80%, at least 85%, at least 90%, at least 95%, at least 98%, at least 99%, or more than 99% pure, where"% purity" means that the M-SmallCas9 polypeptide or variant thereof is the recited percent free from other proteins, other macromolecules, or contaminants that may be present during the production of the M-SmallCas9 polypeptide or variant thereof.

### Compositions Comprising A Guide RNA and A Site-Directed Modifying Polypeptide

A composition may comprise: (i) a guide RNA or a polynucleotide encoding the guide RNA; and ii) a nucleic acid comprising a codon-optimized polynucleotide sequence encoding a M-SmallCas9 polypeptide or variant thereof, or a M-SmallCas9 polypeptide or variant thereof expressed from the nucleic acid. The M-SmallCas9 polypeptide or variant thereof may exhibit enzymatic activity that modifies a target DNA. The M-SmallCas9 polypeptide or variant thereof may exhibit enzymatic activity that modifies a polypeptide encoded by a target DNA. The M-SmallCas9 polypeptide or variant thereof may modulate transcription from the target DNA.

The components of the composition may be individually pure, *e*.*g*. each of the components is at least 75%, at least 80%, at least 90%, at least 95%, at least 98%, at least 99%, or at least 99%, pure. The individual components of a composition may be pure before being added to the composition.

### Kits

In some embodiments, a kit is provided herein as defined in the claims. A kit may include one or more of: a M-SmallCas9 polypeptide or variant thereof expressed from, for example, a codon-optimized polynucleotide sequence; a guide RNA; a nucleic acid comprising a nucleotide sequence encoding a guide RNA. A kit can include a complex that includes two or more of: a M-SmallCas9 polypeptide or variant thereof; a nucleic acid comprising a nucleotide encoding a M-SmallCas9 polypeptide or variant thereof; a guide RNA; a nucleic acid comprising a nucleotide sequence encoding a guide RNA. A kit may include a M-SmallCas9 polypeptide or variant thereof, or a polynucleotide encoding the same. The activity portion of the M-SmallCas9 polypeptide or variant thereof may exhibit reduced or inactivated nuclease activity. The M-SmallCas9 polypeptide or variant thereof may be a M-SmallCas9 fusion protein.

A kit may include: (a) a nucleic acid comprising a codon-optimized polynucleotide sequence encoding a M-SmallCas9 polypeptide or variant thereof, or a M-SmallCas9 polypeptide or variant thereof expressed from the nucleic acid; and (b) a gRNA or nucleic acid encoding the gRNA, wherein the gRNA is capable of guiding the M-SmallCas9 polypeptide or variant thereof to a target polynucleotide sequence. The kit may comprise the nucleic acid comprising the codon-optimized polynucleotide sequence. A kit comprising a M-SmallCas9 polypeptide or variant thereof expressed from a codon-optimized polynucleotide sequence, or a nucleic acid comprising the codon-optimized polynucleotide sequence, can further include one or more additional reagents, where such additional reagents can be selected from: a buffer for introducing the M-SmallCas9 polypeptide or variant thereof into a cell; a wash buffer; a control reagent; a control expression vector or polyribonucleotide; a reagent for *in vitro* production of the M-SmallCas9 polypeptide or variant thereof from DNA, and the like.

In some embodiments the kit includes an sgRNA. In some embodiments, the kit includes two or more sgRNAs.

In some embodiments of any of in the kits described herein, a gRNA (including, e.g., two or more guide RNAs) may be provided as an array (*e*.*g*. an array of RNA molecules, an array of DNA molecules encoding the guide RNA(s), *etc.*)*.* Such kits can be useful, for example, for use in conjunction with the above described genetically modified host cells that include a M-SmallCas9 polypeptide or variant thereof.

The kit may further include a donor polynucleotide to effect the desired genetic modification.

Components of a kit can be in separate containers; or can be combined in a single container.

Any of the kits described herein can further include one or more additional reagents, where such additional reagents can be selected from: a dilution buffer; a reconstitution solution; a wash buffer; a control reagent; a control expression vector or Polyribonucleotide; a reagent for *in vitro* production of the M-SmallCas9 polypeptide or variant thereof from DNA, and the like.

In addition to above-mentioned components, a kit can further include instructions for using the components of the kit to practice the methods. The instructions for practicing the methods are generally recorded on a suitable recording medium. For example, the instructions may be printed on a substrate, such as paper or plastic, *etc.* As such, the instructions may be present in the kits as a package insert, in the labeling of the container of the kit or components thereof (*e*.*g*., associated with the packaging or subpackaging) *etc.* The instructions may be present as an electronic storage data file present on a suitable computer readable storage medium, *e.g.* CD-ROM, diskette, flash drive, *etc.* The actual instructions may not present in the kit, but means for obtaining the instructions from a remote source, *e*.*g*. via the internet, are provided. An example of this is a kit that includes a web address where the instructions can be viewed and/or from which the instructions can be downloaded. As with the instructions, this means for obtaining the instructions is recorded on a suitable substrate.

### Methods of The Disclosure

### Methods of Modifying a Target DNA and/or a Polypeptide Encoded by a Target DNA

Provided herein are methods for modifying a target DNA and/or a polypeptide encoded by a target DNA. The method may involve providing (i) a nucleic acid encoding SEQ ID NO: 1 or a variant thereof having at least 90% sequence identity to SEQ ID NO: 1 encoding a M-SmallCas9 polypeptide or variant thereof, or a M-SmallCas9 polypeptide or variant thereof expressed from the nucleic acid; and (ii) a gRNA or nucleic acid encoding the gRNA, wherein the gRNA is capable of guiding the M-SmallCas9 polypeptide or variant thereof to a target polynucleotide sequence, such that a complex (a "targeting complex") comprising the M-SmallCas9 polypeptide or variant thereof and the gRNA is formed and comes in contact with a target DNA comprising the target polynucleotide sequence.

In some embodiments, the method comprises (I) introducing a heterologous M-SmallCas9 polypeptide as defined in the claims or a nucleic acid encoding a M-SmallCas9 as defined in the claims into the in vitro environment; and (II) introducing one or more single heterologous guide RNA(s) (sgRNA(s)) or DNA(s) encoding such one or more sgRNA(s) in the in vitro environment, each sgRNA or DNA encoding the sgRNA comprising: (a) an engineered DNA targeting segment comprising an RNA and capable of hybridizing to a target sequence in a polynucleotide locus, (b) a tracr mate sequence comprised of RNA, and (c) a tracr RNA sequence comprised of RNA, wherein the tracr mate sequence hybridizes to the tracr RNA sequence, and wherein (a), (b), and (b) are arranged in a 5' to 3' orientation; and (III) creating one or more nicks or cuts or base edits in the target DNA, wherein the M-SmallCas9 polypeptide is directed to the target DNA by the sgRNA in its processed or unprocessed form. In the method a complex (a "targeting complex") comprising the M-SmallCas9 polypeptide and the gRNA is formed and comes in contact with a target DNA comprising the target polynucleotide sequence.

A method of targeting, editing, modifying, or manipulating a target DNA may be an *in vitro* environment, comprising introducing into the *in vitro* environment (a) a nucleic acid comprising *e.g.,* a codon-optimized polynucleotide sequence encoding a M-SmallCas9 polypeptide, or a M-SmallCas9 polypeptide or variant thereof expressed from the nucleic acid; and (b) a gRNA or nucleic acid encoding the gRNA, wherein the gRNA is capable of guiding the M-SmallCas9 polypeptide to a target polynucleotide sequence in the target DNA. the method may comprise introducing into the *in vitro* environment the nucleic acid comprising the codon-optimized polynucleotide sequence. In some embodiments, the method comprises introducing into the *in vitro* environment the M-SmallCas9 polypeptide as defined in claim 1 expressed from the nucleic acid. In some embodiments, the M-SmallCas9 polypeptide comprises (or consists of) the amino acid sequence of SEQ ID NOs: 7. In some embodiments, the method comprises introducing into the *in vitro* environment the gRNA. In some embodiments, the method comprises introducing into the *in vitro* environment nucleic acid encoding the gRNA. In some embodiments, the gRNA is a single guide RNA (sgRNA). In some embodiments, the method comprises introducing into the *in vitro* environment one or more additional gRNAs or nucleic acid encoding the one or more additional gRNAs targeting the target DNA. The method may further comprise introducing into the cell or *in vitro* environment a donor template.

As discussed above, a gRNA or sgRNA and a M-SmallCas9 polypeptide or variant thereof may form a ribonucleoprotein complex. The guide RNA provides target specificity to the complex by including a nucleotide sequence that is complementary to a sequence of a target DNA. The M-SmallCas9 polypeptide or variant thereof of the complex provides the endonuclease activity. A complex may modify a target DNA, leading to, for example, DNA cleavage, DNA methylation, DNA damage, DNA repair, *etc.* A complex may modify a target polypeptide associated with target DNA (*e.g.* a histone, a DNA-binding protein, *etc*.), leading to, for example, histone methylation, histone acetylation, histone ubiquitination, and the like. The target DNA may be, for example, naked (*e*.*g*. unbound by DNA associated proteins) DNA *in vitro,* chromosomal DNA in cells *in vitro,* chromosomal DNA in cells *in vivo* (not claimed), *etc.*

The nuclease activity of a M-SmallCas9 polypeptide or variant thereof described herein may cleave target DNA to produce double strand breaks. These breaks are then repaired by the cell in one of two ways: non-homologous end joining, and homology-directed repair. In non-homologous end joining (NHEJ), the double-strand breaks are repaired by direct ligation of the break ends to one another. In the process a few base pairs can be inserted or deleted at the cleavage site. In homology-directed repair, a donor polynucleotide with homology to the cleaved target DNA sequence is used as a template for repair of the cleaved target DNA sequence, resulting in the transfer of genetic information from the donor polynucleotide to the target DNA. As such, new nucleic acid material may be inserted/copied into the site. A target DNA may be contacted with a donor polynucleotide. A donor polynucleotide may be introduced into a cell. The modifications of the target DNA due to NHEJ and/or homology-directed repair lead to, for example, gene correction, gene replacement, gene tagging, transgene insertion, nucleotide deletion, nucleotide insertion, gene disruption, gene mutation, sequence replacement, *etc.* Accordingly, cleavage of DNA by a M-SmallCas9 polypeptide or variant thereof may be used to delete nucleic acid material from a target DNA sequence (*e.g.* to disrupt a gene that makes cells susceptible to infection (*e.g.* the CCRS or CXCR4 gene, which makes T cells susceptible to HIV infection, to remove disease-causing trinucleotide repeat sequences in neurons, to create gene knockouts and mutations as disease models in research, *etc.)* by cleaving the target DNA sequence and allowing the cell to repair the sequence in the absence of an exogenously provided donor polynucleotide. Thus, the methods can be used to knock out a gene (resulting in complete lack of transcription/translation or altered transcription/translation) or to knock in genetic material into a locus of choice in the target DNA

In addition or alternatively, if a guide RNA and a M-SmallCas9 polypeptide or variant thereof are co-administered to cells with a donor polynucleotide sequence that includes at least a segment with homology to the target DNA sequence, the subject methods may be used to add, e.g., insert or replace, nucleic acid material to a target DNA sequence (*e*.*g*. to "knock in" a nucleic acid that encodes for a protein, an siRNA, an miRNA, *etc.*)*,* to add a tag *(e.g.* 6xHis, a fluorescent protein (*e.g.* a green fluorescent protein; a yellow fluorescent protein, *etc.*)*,* hemagglutinin (HA), FLAG, *etc.*), to add a regulatory sequence to a gene (*e*.*g*. promoter, polyadenylation signal, internal ribosome entry sequence (IRES), 2A peptide, start codon, stop codon, splice signal, localization signal, *etc.),* to modify a nucleic acid sequence (*e*.*g*. introduce a mutation), and the like. As such, a complex comprising a guide RNA and a M-SmallCas9 polypeptide or variant thereof may be useful in any *in vitro* or *in vivo* application in which it is desirable to modify DNA in a site- specific, e.g., "targeted", way, for example gene knock-out, gene knock-in, gene editing, gene tagging, sequence replacement, *etc.,* as used in, for example, gene therapy, *e*.*g*. to treat a disease or as an antiviral, antipathogenic, or anticancer therapeutic, the production of genetically modified organisms in agriculture, the large scale production of proteins by cells for therapeutic, diagnostic, or research purposes, the induction of iPS cells, biological research, the targeting of genes of pathogens for deletion or replacement, *etc.*

The methods described herein may employ a M-SmallCas9 polypeptide or variant thereof including a heterologous sequence (*e*.*g*. a M-SmallCas9 fusion polypeptide). A heterologous sequence may provide for subcellular localization of the M-SmallCas9 polypeptide or variant thereof (*e*.*g*. a nuclear localization signal (NLS) for targeting to the nucleus; a mitochondrial localization signal for targeting to the mitochondria; a chloroplast localization signal for targeting to a chloroplast; an ER retention signal; and the like). A heterologous sequence may provide a tag for ease of tracking or purification (*e.g.* a fluorescent protein, *e.g.* green fluorescent protein (GFP), YFP, RFP, CFP, mCherry, tdTomato, and the like; a histidine tag, *e.g.* a 6XHis tag; a hemagglutinin (HA) tag; a FLAG tag; a Myc tag; and the like). The heterologous sequence may provide for increased or decreased stability.

The methods described herein may employ a guide RNA and a M-SmallCas9 polypeptide or variant thereof used as an inducible system for shutting off gene expression in target cells. Nucleic acids encoding an appropriate guide RNA and/or an appropriate M-SmallCas9 polypeptide or variant thereof may be incorporated into the chromosome of a target cell and are under control of an inducible promoter. When the guide RNA and/or the M-SmallCas9 polypeptide or variant thereof are induced, the target DNA is cleaved (or otherwise modified) at the location of interest (*e*.*g*. a target gene on a separate plasmid), when both the guide RNA and the M-SmallCas9 polypeptide or variant thereof are present and form a complex. As such, target cells may be engineered to include nucleic acid sequences encoding an appropriate M-SmallCas9 polypeptide or variant thereof in the genome and/or an appropriate guide RNA on a plasmid (*e*.*g*. under control of an inducible promoter), allowing experiments in which the expression of any targeted gene (expressed from a separate plasmid introduced into the strain) could be controlled by inducing expression of the guide RNA and the M-SmallCas9 polypeptide or variant thereof. The M-SmallCas9 polypeptide or variant thereof may have enzymatic activity that modifies target DNA in ways other than introducing double strand breaks. Enzymatic activity of interest that may be used to modify target DNA (*e*.*g*. by fusing a heterologous polypeptide with enzymatic activity to a M-SmallCas9 polypeptide or variant thereof, thereby generating a M-SmallCas9 fusion polypeptide or variant thereof) includes, but is not limited methyltransferase activity, demethylase activity, DNA repair activity, DNA damage activity, deamination activity, dismutase activity, alkylation activity, depurination activity, oxidation activity, pyrimidine dimer forming activity, integrase activity, transposase activity, recombinase activity, polymerase activity, ligase activity, helicase activity, photolyase activity or glycosylase activity). Methylation and demethylation is recognized in the art as an important mode of epigenetic gene regulation while repair of DNA damage is essential for cell survival and for proper genome maintenance in response to environmental stresses. As such, the methods herein find use in the epigenetic modification of target DNA and may be employed to control epigenetic modification of target DNA at any location in a target DNA by introducing the desired sequence into the spacer region of a guide RNA. The methods herein also find use in the intentional and controlled damage of DNA at any desired location within the target DNA. The methods herein also find use in the sequence- specific and controlled repair of DNA at any desired location within the target DNA. Methods to target DNA-modifying enzymatic activities to specific locations in target DNA find use in both research and clinical applications.

Multiple guide RNAs may be used to simultaneously modify different locations on the same target DNA or on different target DNAs. Two or more guide RNAs may target the same gene or transcript or locus. Two or more guide RNAs may target different unrelated loci. Two or more guide RNAs may target different, but related loci.

The M-SmallCas9 polypeptide or variant thereof may be provided directly as a protein. As one non-limiting example, fungi (*e*.*g*. yeast) can be transformed with exogenous protein and/or nucleic acid using spheroplast transformation (see Kawai et al., Bioeng Bugs. 2010 Nov-Dec;1(6):395-403 :'Transformation of Saccharomyces cerevisiae and other fungi: methods and possible underlying mechanism"; and Tanka et al., Nature. 2004 Mar 18;428(6980):323-8: "Conformational variations in an infectious protein determine prion strain differences"). Thus, a M-SmallCas9 polypeptide can be incorporated into a spheroplast (with or without nucleic acid encoding a guide RNA and with or without a donor polynucleotide) and the spheroplast can be used to introduce the content into a yeast cell. A M-SmallCas9 polypeptide or variant thereof can be introduced into a cell (provided to the cell) by any suitable method; such methods are known to those of ordinary skill in the art. As another non-limiting example, a M-SmallCas9 polypeptide or variant thereof can be injected directly into a cell (*e.g.* with or without nucleic acid encoding a guide RNA and with or without a donor polynucleotide), *e*.*g*. a cell of a zebrafish embryo, the pronucleus of a fertilized mouse oocyte, etc (not claimed).

### Methods of Modulating Transcription

Described herein are methods of modulating transcription of a target nucleic acid in a host cell. The methods generally involve contacting the target nucleic acid with an enzymatically inactive M-SmallCas9 polypeptide and a guide RNA. The methods are useful in a variety of applications, which are also described.

A transcriptional modulation method of the present disclosure overcomes some of the drawbacks of methods involving RNAi. A transcriptional modulation method of the present disclosure finds use in a wide variety of applications, including research applications, drug discovery (*e*.*g*. high throughput screening), target validation, industrial applications (*e*.*g*. crop engineering; microbial engineering, *etc.),* diagnostic applications, therapeutic applications, and imaging techniques.

Described herein is a method of selectively modulating transcription of a target DNA in a host cell, *e.g.,* a human cell. The method generally involves: a) introducing into the host cell: i) a guide RNA, or a nucleic acid comprising a nucleotide sequence encoding the guide RNA; and ii) a M-SmallCas9 polypeptide or variant thereof, or a nucleic acid comprising a nucleotide sequence encoding the M-SmallCas9 polypeptide or variant thereof, where the M-SmallCas9 polypeptide or variant thereof exhibits reduced endodeoxyribonuclease activity. The guide RNA and the M-SmallCas9 polypeptide or variant thereof form a complex in the host cell; the complex selectively modulates transcription of a target DNA in the host cell.

The methods described herein may employ a modified form of the M-SmallCas9 protein. The modified form of the M-SmallCas9 protein may include an amino acid change (*e*.*g*. deletion, insertion, or substitution) that reduces the nuclease activity of the M-SmallCas9 protein. For example, the modified form of the M-SmallCas9 protein may have less than 50%, less than 40%, less than 30%, less than 20%, less than 10%, less than 5%, or less than 1% of the nuclease activity of the corresponding unmodified M-SmallCas9 polypeptide. The modified form of the M-SmallCas9 polypeptide may have no substantial nuclease activity. When a M-SmallCas9 polypeptide or variant thereof is a modified form of the M-SmallCas9 polypeptide that has no substantial nuclease activity, it can be referred to as "dM-SmallCas9."

A transcription modulation method described herein may allow for selective modulation (*e*.*g*. reduction or increase) of a target nucleic acid in a host cell. For example, "selective" reduction of transcription of a target nucleic acid reduces transcription of the target nucleic acid by at least 10%, at least 20%, at least 30%, at least 40%, at least 50%, at least 60%, at least 70%, at least 80%, at least 90%, or greater than 90%, compared to the level of transcription of the target nucleic acid in the absence of a guide RNA/M-SmallCas9 polypeptide or variant thereof complex. Selective reduction of transcription of a target nucleic acid reduces transcription of the target nucleic acid, but does not substantially reduce transcription of a non-target nucleic acid, *e*.*g*. transcription of a non-target nucleic acid is reduced, if at all, by less than 10% compared to the level of transcription of the non-target nucleic acid in the absence of the guide RNA/M-SmallCas9 polypeptide or variant thereof complex.

The M-SmallCas9 polypeptide or variant thereof may have activity that modulates the transcription of target DNA (*e*.*g*. in the case of a M-SmallCas9 fusion polypeptide or variant thereof, *etc.).* A M-SmallCas9 fusion polypeptide or variant thereof may comprise a heterologous polypeptide that exhibits the ability to increase or decrease transcription (*e*.*g*. transcriptional activator or transcription repressor polypeptides) is used to increase or decrease the transcription of target DNA at a specific location in a target DNA, which is guided by the spacer of the guide RNA. Examples of source polypeptides for providing a M-SmallCas9 fusion polypeptide or variant thereof with transcription modulatory activity include, but are not limited to light-inducible transcription regulators, small molecule/drug-responsive transcription regulators, transcription factors, transcription repressors, *etc.* The method may be used to control the transcription of a targeted gene-coding RNA (protein-encoding mRNA) and/or a targeted non-coding RNA (*e*.*g*. tRNA, rRNA, snoRNA, siRNA, miRNA, long ncRNA. *etc.).* The M-SmallCas9 polypeptide or variant thereof may have enzymatic activity that modifies a polypeptide associated with DNA (*e*.*g*. histone). The enzymatic activity may be methyltransferase activity, demethylase activity, acetyltransferase activity, deacetylase activity, kinase activity, phosphatase activity, ubiquitin ligase activity (e.g., ubiquitination activity), deubiquitinating activity, adenylation activity, deadenylation activity, SUMOylating activity, deSUMOylating activity, ribosylation activity, deribosylation activity, myristoylation activity, demyristoylation activity glycosylation activity (*e*.*g*. from GlcNAc transferase) or deglycosylation activity. The enzymatic activities listed herein catalyze covalent modifications to proteins. Such modifications are known in the art to alter the stability or activity of the target protein (*e*.*g*. phosphorylation due to kinase activity can stimulate or silence protein activity depending on the target protein). Of particular interest as protein targets are histones. Histone proteins are known in the art to bind DNA and form complexes known as nucleosomes. Histones can be modified (*e*.*g*. by methylation, acetylation, ubiquitination, phosphorylation) to elicit structural changes in the surrounding DNA, thus controlling the accessibility of potentially large portions of DNA to interacting factors such as transcription factors, polymerases and the like. A single histone can be modified in many different ways and in many different combinations (*e*.*g*. trimethylation of lysine 27 of histone 3, H3K27, is associated with DNA regions of repressed transcription while trimethylation of lysine 4 of histone 3, H3K4, is associated with DNA regions of active transcription). Thus, a M-SmallCas9 fusion polypeptide or variant thereof with histone-modifying activity finds use in the site specific control of chromosomal structure and can be used to alter the histone modification pattern in a selected region of target DNA. Such methods find use in both research and clinical applications.

### Increased Transcription

"Selective" increased transcription of a target DNA can increase transcription from the target DNA by at least 1.1 fold (*e.g.* at least 1.2 fold, at least 1.3 fold, at least 1.4 fold, at least 1.5 fold, at least 1.6 fold, at least 1.7 fold, at least 1.8 fold, at least 1.9 fold, at least 2 fold, at least 2.5 fold, at least 3 fold, at least 3.5 fold, at least 4 fold, at least 4.5 fold, at least 5 fold, at least 6 fold, at least 7 fold, at least 8 fold, at least 9 fold, at least 10 fold, at least 12 fold, at least 15 fold, or at least 20-fold) compared to the level of transcription from the target DNA in the absence of a guide RNA/M-SmallCas9 polypeptide or variant thereof complex. Selective increase of transcription of a target DNA increases transcription from the target DNA, but does not substantially increase transcription of a non-target DNA, *e.g.* transcription of a non-target DNA is increased, if at all, by less than about 5-fold *(e.g.* less than about 4-fold, less than about 3-fold, less than about 2-fold, less than about 1.8-fold, less than about 1.6-fold, less than about 1.4-fold, less than about 1.2-fold, or less than about 1.1-fold) compared to the level of transcription of the non-targeted DNA in the absence of the guide RNA/M-SmallCas9 polypeptide or variant thereof complex.

As a non-limiting example, increased transcription can be achieved by fusing dM-SmallCas9 to a heterologous sequence. Suitable fusion partners include, but are not limited to, a polypeptide that provides an activity that indirectly increases transcription by acting directly on the target DNA or on a polypeptide (*e*.*g*. a histone or other DNA-binding protein) associated with the target DNA. Suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, crotonylation, decrotonylation, propionylation, depropionylation, myristoylation activity, or demyristoylation activity.

Additional suitable fusion partners include, but are not limited to, a polypeptide that directly provides for increased transcription of the target nucleic acid (*e.g.* a transcription activator or a fragment thereof, a protein or fragment thereof that recruits a transcription activator, a small molecule/drug-responsive transcription regulator, *etc.*).

A non-limiting example of a method using a dM-SmallCas9 fusion protein to increase transcription in a prokaryote includes a modification of the bacterial one-hybrid (B1H) or two-hybrid (B2H) system,. In the B1H system, a DNA binding domain (BD) is fused to a bacterial transcription activation domain (AD, *e.g.* the alpha subunit of the Escherichia coli RNA polymerase (RNAPa)). Thus, a dM-SmallCas9 can be fused to a heterologous sequence comprising an AD. When the dM-SmallCas9 fusion protein arrives at the upstream region of a promoter (targeted there by the guide RNA) the AD (*e*.*g*. RNAPa) of the dM-SmallCas9 fusion protein recruits the RNAP holoenzyme, leading to transcription activation. In the B2H system, the BD is not directly fused to the AD; instead, their interaction is mediated by a protein-protein interaction (*e*.*g*. GAL11P-GAL4 interaction). To modify such a system for use in the methods, dM-SmallCas9 can be fused to a first protein sequence that provides for protein-protein interaction (e*.g*. the yeast GAL11P and/or GAL4 protein) and RNAa can be fused to a second protein sequence that completes the protein-protein interaction (e*.g*. GAL4 if GAL11Pis fused to dM-SmallCas9, GAL11P if GAL4 is fused to dM-SmallCas9, *etc.*)*.* The binding affinity between GAL11P and GAL4 increases the efficiency of binding and transcription firing rate.

A non-limiting example of a method using a dM-SmallCas9 fusion protein to increase transcription in eukaryotes includes fusion of dM-SmallCas9 to an activation domain (AD) *(e.g.* GAL4, herpesvirus activation protein VP16 or VP64, human nuclear factor NF-KB p65 subunit, *etc.*)*.* To render the system inducible, expression of the dM-SmallCas9 fusion protein can be controlled by an inducible promoter (*e.g.* Tet-ON, Tet-OFF, *etc.*)*.* The guide RNA can be designed to target known transcription response elements *(e.g.* promoters, enhancers, *etc.*), known upstream activating sequences (UAS), sequences of unknown or known function that are suspected of being able to control expression of the target DNA, *etc.*

### Additional Fusion Partners

Non-limiting examples of fusion partners to accomplish increased or decreased transcription include, but are not limited to, transcription activator and transcription repressor domains (*e*.*g*. the Krüppel associated box (KRAB or SKD); the Mad mSIN3 interaction domain (SID); the ERF repressor domain (ERD), *etc.*)*.* In some such cases, the dM-SmallCas9 fusion protein is targeted by the guide RNA to a specific location (*e*.*g*., sequence) in the target DNA and exerts locus-specific regulation such as blocking RNA polymerase binding to a promoter (which selectively inhibits transcription activator function), and/or modifying the local chromatin status (*e*.*g*. when a fusion sequence is used that modifies the target DNA or modifies a polypeptide associated with the target DNA). The changes may be transient (*e.g.* transcription repression or activation). The changes may be inheritable (*e.g.* when epigenetic modifications are made to the target DNA or to proteins associated with the target DNA, *e*.*g*. nucleosomal histones). The heterologous sequence may be be fused to the C-terminus of the dM-SmallCas9 polypeptide. The heterologous sequence may be fused to the N-terminus of the dM-SmallCas9 polypeptide. The heterologous sequence may be fused to an internal portion (*e*.*g*., a portion other than the N-or C-terminus) of the dM-SmallCas9 polypeptide. The biological effects of a method using a dM-SmallCas9 fusion protein can be detected by any suitable method (*e*.*g*. gene expression assays; chromatin-based assays, *e.g.* Chromatin ImmunoPrecipitation (ChIP), Chromatin *in vivo* Assay (CiA), *etc.*).

A method may involve use of two or more different guide RNAs. For example, two different guide RNAs can be used in a single host cell, where the two different guide RNAs target two different target sequences in the same target nucleic acid. Use of two different guide RNAs targeting two different targeting sequences in the same target nucleic acid may provide for increased modulation (*e*.*g*. reduction or increase) in transcription of the target nucleic acid.

As another example, two different guide RNAs can be used in a single host cell, where the two different guide RNAs target two different target nucleic acids. Thus, for example, a transcriptional modulation method can further comprise introducing into the host cell a second guide RNA, or a nucleic acid comprising a nucleotide sequence encoding the second guide RNA.

A nucleic acid (*e.g.* a guide RNA, *e.g.* a single-molecule guide RNA; a donor polynucleotide; a nucleic acid encoding a M-SmallCas9 polypeptide or variant thereof; *etc.*) may comprise a modification or sequence that provides for an additional desirable feature (*e*.*g*. modified or regulated stability; subcellular targeting; tracking, *e.g*. a fluorescent label; a binding site for a protein or protein complex; *etc.*)*.* Non-limiting examples include: a 5' cap (*e.g.* a 7-methylguanylate cap (m 7G)); a 3' polyadenylated tail (*e.g.,* a 3' poly(A) tail); a riboswitch sequence or an aptamer sequence *(e.g.* to allow for regulated stability and/or regulated accessibility by proteins and/or protein complexes); a terminator sequence; a sequence that forms a dsRNA duplex (*e.g.,* a hairpin)); a modification or sequence that targets the RNA to a subcellular location (*e*.*g*. nucleus, mitochondria, chloroplasts, and the like); a modification or sequence that provides for tracking (*e*.*g*. direct conjugation to a fluorescent molecule, conjugation to a moiety that facilitates fluorescent detection, a sequence that allows for fluorescent detection, *etc*.); a modification or sequence that provides a binding site for proteins *(e.g.* proteins that act on DNA, including transcriptional activators, transcriptional repressors, DNA methyltransferases, DNA demethylases, histone acetyltransferases, histone deacetylases, and the like); a modification of RNA that alters the structure of such RNA, consequently the M-SmallCas9 ribonucleoprotein; and combinations thereof.

### Multiple Simultaneous Guide RNAs

Multiple guide RNAs may be used simultaneously in the same cell to simultaneously modulate transcription at different locations on the same target DNA or on different target DNAs. Two or more guide RNAs may target the same gene or transcript or locus. Two or more guide RNAs may target different unrelated loci. Two or more guide RNAs may target different, but related loci.

Because the guide RNAs are small and robust they can be simultaneously present on the same expression vector and can even be under the same transcriptional control if so desired. Two or more *(e.g.* 3 or more, 4 or more, 5 or more, 10 or more, 15 or more, 20 or more, 25 or more, 30 or more, 35 or more, 40 or more, 45 or more, or 50 or more) guide RNAs may be simultaneously expressed in a target cell (from the same or different vectors/ from the same or different promoters). Multiple guide RNAs may be be encoded in an array mimicking naturally occurring CRISPR arrays of targeter RNAs. The targeting segments are encoded as approximately 30 nucleotide long sequences (can be about 16 to about 100 nt) and are separated by CRISPR repeat sequences. The array may be introduced into a cell by DNAs encoding the RNAs or as RNAs.

To express multiple guide RNAs, an artificial RNA processing system mediated by the Csy4 endoribonuclease can be used. For example, multiple guide RNAs can be concatenated into a tandem array on a precursor transcript (*e*.*g*. expressed from a U6 promoter), and separated by Csy4-specific RNA sequence. Co-expressed Csy4 protein cleaves the precursor transcript into multiple guide RNAs. Advantages for using an RNA processing system include: first, there is no need to use multiple promoters; second, since all guide RNAs are processed from a precursor transcript, their concentrations are normalized for similar dM-SmallCas9-binding.

Csy4 is a small endoribonuclease (RNase) protein derived from bacteria Pseudomonas aeruginosa. Csy4 specifically recognizes a minimal 17-bp RNA hairpin, and exhibits rapid (<1 min) and highly efficient (>99.9%) RNA cleavage. Unlike most RNases, the cleaved RNA fragment remains stable and functionally active. The Csy4-based RNA cleavage can be repurposed into an artificial RNA processing system. In this system, the 17-bp RNA hairpins are inserted between multiple RNA fragments that are transcribed as a precursor transcript from a single promoter. Co-expression of Csy4 is effective in generating individual RNA fragments.

### Host Cells

The methods of the disclosure may be employed to induce transcriptional modulation in mitotic or post-mitotic cells *in vivo* (not claimed) and/or ex vivo and/or *in vitro.*The methods of the disclosure may be employed to induce DNA cleavage, DNA modification, and/or transcriptional modulation in mitotic or post-mitotic cells *in vivo* and/or ex vivo and/or *in vitro* (*e.g.* to produce genetically modified cells that can be reintroduced into an individual).

Because the guide RNA provides specificity by hybridizing to target DNA, a mitotic and/or post-mitotic cell can be any of a variety of host cell, where suitable host cells include, but are not limited to, a bacterial cell; an archaeal cell; a single-celled eukaryotic organism; a plant cell; an algal cell, *e*.*g*. Botryococcus braunii, Chlamydomonas reinhardtii, Nannochloropsis gaditana, Chlorella pyrenoidosa, Sargassum patens, C. agardh, and the like; a fungal cell; an animal cell; a cell from an invertebrate animal (*e.g.* an insect, a cnidarian, an echinoderm, a nematode, *etc*.); a eukaryotic parasite (*e.g.* a malarial parasite, *e.g.* Plasmodium fakiparum; a helminth; *etc*.); a cell from a vertebrate animal (*e.g.* fish, amphibian, reptile, bird, mammal); a mammalian cell, *e.g.* a rodent cell, a human cell, a non-human primate cell, *etc.* The host cell may be be any human cell. Suitable host cells include naturally occurring cells; genetically modified cells (*e.g.* cells genetically modified in a laboratory, *e.g.* by the "hand of man"); and cells manipulated *in vitro* in any way. In some embodiments, a host cell is isolated.

Any type of cell may be of interest (*e.g.* a stem cell, *e.g.* an embryonic stem (ES) cell, an induced pluripotent stem (iPS) cell, a germ cell; a somatic cell, *e.g.* a fibroblast, a hematopoietic cell, a neuron, a muscle cell, a bone cell, a hepatocyte, a pancreatic cell; an *in vitro* or *in vivo* (not claimed) embryonic cell of an embryo at any stage, *e.g.* a 1-cell, 2-cell, 4-cell, 8-cell, *etc.* stage zebrafish embryo; *etc*.). Cells may be from established cell lines or they may be primary cells, where "primary cells", "primary cell lines", and "primary cultures" are used interchangeably herein to refer to cells and cells cultures that have been derived from a subject and allowed to grow *in vitro* for a limited number of passages, *e.g.,* splittings, of the culture. For example, primary cultures include cultures that may have been passaged 0 times, 1 time, 2 times, 4 times, 5 times, 10 times, or 15 times, but not enough times go through the crisis stage. Primary cell lines can be maintained for fewer than 10 passages *in vitro.* Target cells are unicellular organisms, or are grown in culture.

If the cells are primary cells, such cells may be harvested from an individual by any suitable method. For example, leukocytes may be suitably harvested by apheresis, leukocytapheresis, density gradient separation, *etc.,* while cells from tissues such as skin, muscle, bone marrow, spleen, liver, pancreas, lung, intestine, stomach, *etc.* are most suitably harvested by biopsy. An appropriate solution may be used for dispersion or suspension of the harvested cells. Such solution will generally be a balanced salt solution, *e*.*g*. normal saline, phosphate-buffered saline (PBS), Hank's balanced salt solution, *etc.,* suitably supplemented with fetal calf serum or other naturally occurring factors, in conjunction with an acceptable buffer at low concentration, *e*.*g*. from 5-25 mM. Suitable buffers include HEPES, phosphate buffers, lactate buffers, *etc.* The cells may be used immediately, or they may be stored, frozen, for long periods of time, being thawed and capable of being reused. In such cases, the cells will generally be frozen in 10% dimethyl sulfoxide (DMSO), 50% serum, 40% buffered medium, or some other such solution as is commonly used in the art to preserve cells at such freezing temperatures, and thawed in a manner as commonly known in the art for thawing frozen cultured cells.

### Uses

A method for modulating transcription according to the present disclosure finds use in a variety of applications. Applications include research applications; diagnostic applications; industrial applications; and therapeutic applications.

Research applications include, *e*.*g*. determining the effect of reducing or increasing transcription of a target nucleic acid on, *e*.*g*. development, metabolism, expression of a downstream gene, and the like. High through-put genomic analysis can be carried out using a transcription modulation method, in which only the spacer of the guide RNA needs to be varied, while the protein-binding segment and the transcription termination segment can (in some cases) be held constant. A library comprising a plurality of nucleic acids used in the genomic analysis would include: a promoter operably linked to a guide RNA-encoding nucleotide sequence, where each nucleic acid would include a common protein-binding segment, a different spacer, and a common transcription termination segment. A chip could contain over 5 x 10⁴ unique guide RNAs. Applications would include large-scale phenotyping, gene-to-function mapping, and meta-genomic analysis.

The methods disclosed herein find use in the field of metabolic engineering. Because transcription levels can be efficiently and predictably controlled by designing an appropriate guide RNA, as disclosed herein, the activity of metabolic pathways (*e*.*g*. biosynthetic pathways) can be precisely controlled and tuned by controlling the level of specific enzymes (*e*.*g*. via increased or decreased transcription) within a metabolic pathway of interest. Metabolic pathways of interest include those used for chemical (fine chemicals, fuel, antibiotics, toxins, agonists, antagonists, *etc.)* and/or drug production.

Biosynthetic pathways of interest include but are not limited to (1) the mevalonate pathway (*e*.*g*. HMG-CoA reductase pathway) (converts acetyl-GoA to dimethylallyl pyrophosphate (DMAPP) and isopentenyl pyrophosphate (IPP), which are used for the biosynthesis of a wide variety of biomolecules including terpenoids/isoprenoids), (2) the non-mevalonate pathway (*e*.*g*., the "2-C- methyl-D-erythritol 4-phosphate/1-deoxy-D-xylulose 5-phosphate pathway" or "MEP/DOXP pathway" or "DXP pathway")(also produces DMAPP and IPP, instead by converting pyruvate and glyceraldehyde 3-phosphate into DMAPP and IPP via an alternative pathway to the mevalonate pathway), (3) the polyketide synthesis pathway (produces a variety of polyketides via a variety of polyketide synthase enzymes. Polyketides include naturally occurring small molecules used for chemotherapy (e. g., tetracyclin, and macrolides) and industrially important polyketides include rapamycin (immunosuppressant), erythromycin (antibiotic), lovastatin (anticholesterol drug), and epothilone B (anticancer drug)), (4) fatty acid synthesis pathways, (5) the DAHP (3-deoxy-D-arabino- heptulosonate ?-phosphate) synthesis pathway, (6) pathways that produce potential biofuels (such as short-chain alcohols and alkane, fatty acid methyl esters and fatty alcohols, isoprenoids, *etc.), etc.*

### Networks and Cascades

The methods disclosed herein can be used to design integrated networks (*e*.*g*., a cascade or cascades) of control. For example, a guide RNA and M-SmallCas9 polypeptide or variant thereof may be used to control (*e.g.,* modulate, *e.g.* increase, decrease) the expression of another DNA-targeting RNA or another M-SmallCas9 polypeptide or variant thereof. For example, a first guide RNA may be designed to target the modulation of transcription of a second fusion dM-SmallCas9 polypeptide with a function that is different than the first M-SmallCas9 polypeptide or variant thereof (*e*.*g*. methyltransferase activity, demethylase activity, acetyltransferase activity, deacetylase activity, *etc.).* The second fusion dM-SmallCas9 polypeptide may be be selected such that it may not interact with the first guide RNA. The second fusion dM-SmallCas9 polypeptide may be selected such that it does interact with the first guide RNA. In some such cases, the activities of the two (or more) dM-SmallCas9 proteins may compete (*e.g.* if the polypeptides have opposing activities) or may synergize (*e.g.* if the polypeptides have similar or synergistic activities). Likewise, as noted above, any of the complexes (*e.g.,* guide RNA/dM-SmallCas9 polypeptide) in the network can be designed to control other guide RNAs or dM-SmallCas9 polypeptides. Because a guide RNA and M-SmallCas9 polypeptide or variant thereof can be targeted to any desired DNA sequence, the methods described herein can be used to control and regulate the expression of any desired target. The integrated networks (e.g., cascades of interactions) that can be designed range from very simple to very complex, and are without limit.

In a network wherein two or more components (*e*.*g*. guide RNAs and dM-SmallCas9 polypeptides) are each under regulatory control of another guide RNA/dM-SmallCas9 polypeptide complex, the level of expression of one component of the network may affect the level of expression (*e*.*g*. may increase or decrease the expression) of another component of the network. Through this mechanism, the expression of one component may affect the expression of a different component in the same network, and the network may include a mix of components that increase the expression of other components, as well as components that decrease the expression of other components. As would be readily understood by one of skill in the art, the above examples whereby the level of expression of one component may affect the level of expression of one or more different component(s) are for illustrative purposes, and are not limiting. An additional layer of complexity may be optionally introduced into a network when one or more components are modified (as described above) to be manipulable (*e.g.,* under experimental control, *e.g.* temperature control; drug control, *e.g.,* drug inducible control; light control; *etc*.)*.*

As one non-limiting example, a first guide RNA can bind to the promoter of a second guide RNA, which controls the expression of a target therapeutic/metabolic gene. In such a case, conditional expression of the first guide RNA indirectly activates the therapeutic/metabolic gene. RNA cascades of this type are useful, for example, for easily converting a repressor into an activator, and can be used to control the logics or dynamics of expression of a target gene.

A transcription modulation method can also be used for drug discovery and target validation.

### Methods of Treating a Disease or Condition

In some aspects of the disclosure, the guide RNA and/or M-SmallCas9 polypeptide or variant thereof and/or donor polynucleotide are employed to modify cellular DNA *in vivo,* for purposes such as gene therapy, *e*.*g*. to treat a disease or as an antiviral, antipathogenic, or anticancer therapeutic, for the production of genetically modified organisms in agriculture, or for biological research. Components of a CRISPR/M-SmallCas9 system including (i) a guide RNA or nucleic acid encoding the gRNA; (ii) a nucleic acid comprising a codon-optimized polynucleotide sequence encoding a M-SmallCas9 polypeptide or variant thereof, or a M-SmallCas9 polypeptide or variant thereof expressed from the nucleic acid; and/or (iii) a donor polynucleotide are administered to the individual. Administration may be by any well-known method in the art for the administration of peptides, small molecules and nucleic acids to a subject. The CRISPR/M-SmallCas9 system components can be incorporated into a variety of formulations. More particularly, the CRISPR/M-SmallCas9 system components of the present disclosure can be formulated into pharmaceutical compositions by combination with appropriate pharmaceutically acceptable carriers or diluents.

Pharmaceutical preparations or compositions may comprise components of a CRISPR/M-SmallCas9 system including (i) a guide RNA or nucleic acid encoding the gRNA; (ii) a nucleic acid comprising a codon-optimized polynucleotide sequence encoding a M-SmallCas9 polypeptide or variant thereof, or a M-SmallCas9 polypeptide or variant thereof expressed from the nucleic acid; and/or (iii) a donor polynucleotide present in a pharmaceutically acceptable vehicle. "Pharmaceutically acceptable vehicles" may be vehicles approved by a regulatory agency of the Federal or a state government or listed in the US Pharmacopeia or other generally recognized pharmacopeia for use in mammals, such as humans. The term "vehicle" refers to a diluent, adjuvant, excipient, or carrier with which a compound of the disclosure is formulated for administration to a mammal. Such pharmaceutical vehicles can be lipids, *e.g.* liposomes, *e.g.* liposome dendrimers; liquids, such as water and oils, including those of petroleum, animal, vegetable or synthetic origin, such as peanut oil, soybean oil, mineral oil, sesame oil and the like, saline; gum acacia, gelatin, starch paste, talc, keratin, colloidal silica, urea, and the like. In addition, auxiliary, stabilizing, thickening, lubricating and coloring agents may be used. Pharmaceutical compositions may be formulated into preparations in solid, semisolid, liquid or gaseous forms, such as tablets, capsules, powders, granules, ointments, solutions, suppositories, injections, inhalants, gels, microspheres, and aerosols. As such, administration of the CRISPR/M-SmallCas9 system components can be achieved in various ways, including oral, buccal, rectal, parenteral, intraperitoneal, intradermal, transdermal, intra-tracheal, intraocular, *etc.,* administration. The active agent may be systemic after administration or may be localized by the use of regional administration, intramural administration, or use of an implant that acts to retain the active dose at the site of implantation. The active agent may be formulated for immediate activity or it may be formulated for sustained release.

For some conditions, particularly central nervous system conditions, it may be necessary to formulate agents to cross the blood-brain barrier (BBB). One strategy for drug delivery through the BBB entails disruption of the BBB, either by osmotic means such as mannitol or leukotrienes, or biochemically by the use of vasoactive substances such as bradykinin. The potential for using BBB opening to target specific agents to brain tumors is also an option. A BBB disrupting agent can be co-administered with the therapeutic compositions of the disclosure when the compositions are administered by intravascular injection. Other strategies to go through the BBB may entail the use of endogenous transport systems, including Caveolin-1 mediated transcytosis, carrier-mediated transporters such as glucose and amino acid carriers, receptor-mediated transcytosis for insulin or transferrin, and active efflux transporters such asp-glycoprotein. Active transport moieties may also be conjugated to the therapeutic compounds for use in the disclosure to facilitate transport across the endothelial wall of the blood vessel. In addition or alternatively, drug delivery of therapeutics agents behind the BBB may be by local delivery, for example by intrathecal delivery, *e*.*g*. through an Ommaya reservoir (see *e.g.* US Patent Nos. 5,222,982 and 5385582); by bolus injection, *e.g.* by a syringe, *e.g.* intravitreally or intracranially; by continuous infusion, *e.g.* by cannulation, *e.g.* with convection (see *e.g.,* US Application No. 20070254842); or by implanting a device upon which the agent has been reversibly affixed (see *e*.*g*. US Application Nos. 20080081064 and 20090196903).

Generally, an effective amount of components of a CRISPR/M-SmallCas9 system including (i) a guide RNA or nucleic acid encoding the gRNA; (ii) a nucleic acid comprising a codon-optimized polynucleotide sequence encoding a M-SmallCas9 polypeptide or variant thereof, or a M-SmallCas9 polypeptide or variant thereof expressed from the nucleic acid; and/or (iii) a donor polynucleotide are described. As discussed above with regard to ex vivo methods, an effective amount or effective dose of the CRISPR/M-SmallCas9 system components *in vivo* is the amount to induce a 2-fold increase or more in the amount of recombination observed between two homologous sequences relative to a negative control, *e.g*. a cell contacted with an empty vector or irrelevant polypeptide. The amount of recombination may be measured by any suitable method, *e*.*g*. as described above and known in the art. The calculation of the effective amount or effective dose of the CRISPR/M-SmallCas9 system components to be administered is within the skill of one of ordinary skill in the art, and will be routine to those persons skilled in the art. The final amount to be administered will be dependent upon the route of administration and upon the nature of the disorder or condition that is to be treated.

The effective amount given to a particular subject will depend on a variety of factors, several of which will differ from subject to subject. A competent clinician will be able to determine an effective amount of a therapeutic agent to administer to a subject to halt or reverse the progression the disease condition as required. Utilizing LD50 animal data, and other information available for the agent, a clinician can determine the maximum safe dose for an individual, depending on the route of administration. For instance, an intravenously administered dose may be more than an intrathecally administered dose, given the greater body of fluid into which the therapeutic composition is being administered. Similarly, compositions, which are rapidly cleared from the body may be administered at higher doses, or in repeated doses, in order to maintain a therapeutic concentration. Utilizing ordinary skill, the competent clinician will be able to optimize the dosage of a particular therapeutic in the course of routine clinical trials.

For inclusion in a medicament, the CRISPR/M-SmallCas9 system components may be obtained from a suitable commercial source. As a general proposition, the total pharmaceutically effective amount of the CRISPR/M-SmallCas9 system components administered parenterally per dose will be in a range that can be measured by a dose response curve.

Therapies based on the CRISPR/M-SmallCas9 system components, *e*.*g*., preparations of (i) a guide RNA or nucleic acid encoding the gRNA; (ii) a nucleic acid comprising a codon-optimized polynucleotide sequence encoding a M-SmallCas9 polypeptide or variant thereof, or a M-SmallCas9 polypeptide or variant thereof expressed from the nucleic acid; and/or (iii) a donor polynucleotide to be used for therapeutic administration, must be sterile. Sterility is readily accomplished by filtration through sterile filtration membranes (*e.g.* 0.2 micrometer membranes). Therapeutic compositions generally are placed into a container having a sterile access port, for example, an intravenous solution bag or vial having a stopper pierceable by a hypodermic injection needle. The therapies based on the CRISPR/M-SmallCas9 system components may be stored in unit or multi-dose containers, for example, sealed ampules or vials, as an aqueous solution or as a lyophilized formulation for reconstitution. As an example of a lyophilized formulation, 10-ml vials are filled with 5 ml of sterile-filtered 1% (w/v) aqueous solution of compound, and the resulting mixture is lyophilized. The infusion solution is prepared by reconstituting the lyophilized compound using bacteriostatic Water-for-Injection.

Pharmaceutical compositions can include, depending on the formulation desired, pharmaceutically acceptable, non-toxic carriers of diluents, which are defined as vehicles commonly used to formulate pharmaceutical compositions for animal or human administration. The diluent is selected so as not to affect the biological activity of the combination. Examples of such diluents are distilled water, buffered water, physiological saline, PBS, Ringer's solution, dextrose solution, and Hank's solution. In addition, the pharmaceutical composition or formulation can include other carriers, adjuvants, or non-toxic, nontherapeutic, nonimmunogenic stabilizers, excipients and the like. The compositions can also include additional substances to approximate physiological conditions, such as pH adjusting and buffering agents, toxicity adjusting agents, wetting agents and detergents.

The composition can also include any of a variety of stabilizing agents, such as an antioxidant for example. When the pharmaceutical composition includes a polypeptide, the polypeptide can be complexed with various well-known compounds that enhance the *in vivo* stability of the polypeptide, or otherwise enhance its pharmacological properties (*e*.*g*. increase the half-life of the polypeptide, reduce its toxicity, enhance solubility or uptake). Examples of such modifications or complexing agents include sulfate, gluconate, citrate and phosphate. The nucleic acids or polypeptides of a composition can also be complexed with molecules that enhance their *in vivo* attributes. Such molecules include, for example, carbohydrates, polyamines, amino acids, other peptides, ions (*e*.*g*. sodium, potassium, calcium, magnesium, manganese}, and lipids.

Further guidance regarding formulations suitable for various types of administration can be found in Remington's Pharmaceutical Sciences, Mace Publishing Company, Philadelphia, Pa., 20th ed. (2003) and in The United States Pharmacopeia: The National Formulary (USP 24 NF19) published in 1999. For a brief review of methods for drug delivery, see, Langer, Science 249:1527-1533 (1990).

The pharmaceutical compositions can be administered for prophylactic and/or therapeutic treatments. Toxicity and therapeutic efficacy of the active ingredient can be determined according to standard pharmaceutical procedures in cell cultures and/or experimental animals, including, for example, determining the LD50 (the dose lethal to 50% of the population) and the ED50 (the dose therapeutically effective in 50% of the population}. The dose ratio between toxic and therapeutic effects is the therapeutic index and it can be expressed as the ratio LD50/ED50. Therapies that exhibit large therapeutic indices are generally preferred.

The data obtained from cell culture and/or animal studies can be used in formulating a range of dosages for humans. The dosage of the active ingredient generally lines within a range of circulating concentrations that include the ED50 with low toxicity. The dosage can vary within this range depending upon the dosage form employed and the route of administration utilized. The components used to formulate the pharmaceutical compositions are generally of high purity and are substantially free of potentially harmful contaminants (*e*.*g*., at least National Food (NF) grade, generally at least analytical grade, and more typically at least pharmaceutical grade). Moreover, compositions intended for *in vivo* use are generally sterile. To the extent that a given compound must be synthesized prior to use, the resulting product is generally substantially free of any potentially toxic agents, particularly any endotoxins, which may be present during the synthesis or purification process. Compositions for parental administration are also sterile, substantially isotonic and made under GMP conditions.

The effective amount of a therapeutic composition to be given to a particular subject will depend on a variety of factors, several of which will differ from subject to subject. A competent clinician will be able to determine an effective amount of a therapeutic agent to administer to a subject to halt or reverse the progression the disease condition as required. Utilizing LD50 animal data, and other information available for the agent, a clinician can determine the maximum safe dose for an individual, depending on the route of administration. For instance, an intravenously administered dose may be more than an intrathecally administered dose, given the greater body of fluid into which the therapeutic composition is being administered. Similarly, compositions that are rapidly cleared from the body may be administered at higher doses, or in repeated doses, in order to maintain a therapeutic concentration. Utilizing ordinary skill, the competent clinician will be able to optimize the dosage of a particular therapeutic in the course of routine clinical trials.

The number of administrations of treatment to a subject may vary. Introducing the genetically modified cells into the subject may be a one-time event; but in certain situations, such treatment may elicit improvement for a limited period of time and require an on-going series of repeated treatments. In certain situations, multiple administrations of the genetically modified cells may be required before an effect is observed. The exact protocols depend upon the disease or condition, the stage of the disease and parameters of the individual subject being treated.

### Examples

The following examples further illustrate embodiments of the inventions described herein. Examples which do not fall under the scope of the appended claims do not form part of the present invention.

### Example 1: Screening M-Small Cas9 nucleases

To determine whether mutated Cas9 nucleases had improved features, variants of M-SmallCas9: M-SauCas9-R420A, according to SEQ ID NO. 6; M-SluCas9-R414A, according to SEQ ID NO. 7; M-Gib11SpaCas9-1-M417L (according to SEQ ID NO. 133); and MGib11SpaCas9-3-E410A, according to SEQ ID NO. 8 are tested in comparison to
a) SluCas9 (SEQ ID NO. 9), and
b) SauCas9 (NCBI GenPept database; Accession No. CRI31653.1; https://www.ncbi.nlm.nih.gov/protein/CRI31653.1; accessed on November 21, 2018.)
   in a fluorescence polarization-based biochemical cleavage assay. Protein variants are adjusted to same activity levels. Upon incubation of the variants- or SluCas9-RNP complex targeting the R01 or BFP oligonucleotide dsDNA substrate sequence or all possible single nucleotide exchanges of these sequence- cleavage is observed by changes in the fluorescence polarization and fluorescence intensity signal (decrease of polarization values and increase in fluorescence intensity over time upon successful cleavage).

As a quantitative estimate of the cleavage reaction, the initial slope of the graph is analyzed. Specificity is assessed by comparing the cleavage (slope) of any one of the 60 single nucleotide mismatch oligonucleotides to the cleavage (slope) of the original R01 and BFP sequence.

The results indicate that the variants M-SauCas9-R420A, M-SluCas9-R414A, and M-Gib11SpaCas9-3-E410A, M-Gib11SpaCas9-1-M417L, as well as the wildtype SluCas9 and SauCas9 successfully cleave an oligonucleotide substrate. Moreover the fidelity of the variants appears to be significantly increased when compared to the respective wildtype protein.

### Example 2: Determination of biochemical specificity profile for SluCas9 and M-SluCas9R414A

To assess the specificity profile of SluCas9 (according to SEQ ID NO. 9) and M-SluCas9R414A (SEQ ID NO. 7), biochemical cleavage of an on-target sequence and all 60-derived single nucleotide mismatched dsDNA substrates (according to SEQ ID Nos. 11 to 132 used as pairs e.g. R01-1-A and R01-1-B with SEQ ID NOs. 11 and 12 represented one duplex pair) were determined. Oligonucleotide duplexes were prepared in 10 mM Tris (pH 7.8) 50 mM NaCl as 10 µM solutions (from 100 µM stocks) and annealed at 95 °C for 5 minutes then slowly cooled down in thermo cycler (6°C per minute). The stocks were subsequently diluted in 10 mM Tris (pH 7.8), 50 mM NaCl, and 0.05 % Pluronic. 20 µl of each oligonucleotide (20 nM) was immobilized on streptavidin coated plates, washed twice after 10 minutes and then incubated with a 20 µL sample for a kinetics of 60 minutes (excitation wavelength: 635 nm; emission wavelength: 670 nm). Prior to the cleavage the reaction RNP was formed. RNP was assembled by mixing 12.03 µl of the respective Cas9 protein (1.21 µg/µl) in 3200 µl 1xPBS + 5mM MgCl2 with and without sgRNA (SEQ ID NO. 10; 60 nM final concentration), RNPs were incubated 5 min at 37°C prior to the reaction. 20 µl RNP was added to each well and the polarization was measured for 60 minutes at 37°C.

The cleavage kinetics were analyzed by calculating the initial slope of the oligonucleotide cleavage reaction. The slopes were calculated for each of the 61 substrates and normalized to the value of the on-target substrate (defined as 1). The normalized cleavage values for all 60 off-target substrates where then grouped according to their position. For each of the 20 nucleotide positions in the target sequence, the normalized cleavage value of the three single nucleotide mismatches was blotted to illustrate the position-specific nucleotide tolerance of the tested nuclease.

The results are shown in Figure 1. Apparently M-SluCas9R414A was more specific than SluCas9 wild type.

### Overall specificity of M-SmallCas nucleases:

The cleavage kinetics for a)SluCas9 (SEQ ID NO: 9); b) M-SluCas9R414A (SEQ ID NO. 7); c) *Staphylococcus pyogenes* wild type (New England Biolabs), and d) *Staphylococcus pyogenes* HiFi (Integrated DNA Technologies), were analyzed by calculating the initial slope of the oligonucleotide cleavage reaction. The slopes were calculated for each of the 61 substrates and are normalized to the value of the on-target substrate (defined as 1). All normalized values across the entire panel of 60 off-target substrates were summed to yield an overall specificity value (as depicted in the bar graph in Figure 2).

### Reference for Sequence Listing

| **SEQ ID NO:** | **Identifier** | **Remarks** |
|---|---|---|
| 1 | M-SauCas9_X | |
| 2 | M-SluCas9_x | |
| 3 | M-SpaCas9_X | |
| 4 | M-ShyCas9_X | |
| 5 | M-SmiCas9_X | |
| 6 | M-SauCas9R420A | |
| 7 | M-SluCas9R414A | |
| 8 | MGib11SpaCas9-3E410A | |
| 9 | SluCas9 | Wildtype sequence of SluCas9 used in Examples |
| 10 | sgRNA_Ex1 | sgRNA sequence for Example 1 |
| 11 | R01-1-A | 5'-ATTO647N- |
| 12 | R01-1-B | 3'-Biotin |
| 13 | R01-2-A | 5'-ATTO647N- |
| 14 | R01-2-B | 3'-Biotin |
| 15 | R01-3-A | 5'-ATTO647N- |
| 16 | R01-3-B | 3'-Biotin |
| 17 | R01-4-A | 5'-ATTO647N- |
| 18 | R01-4-B | 3'-Biotin |
| 19 | R01-5-A | 5'-ATTO647N- |
| 20 | R01-5-B | 3'-Biotin |
| 21 | R01-6-A | 5'-ATTO647N- |
| 22 | R01-6-B | 3'-Biotin |
| 23 | R01-7-A | 5'-ATT0647N- |
| 24 | R01-7-B | 3'-Biotin |
| 25 | R01-8-A | 5'-ATTO647N- |
| 26 | R01-8-B | 3'-Biotin |
| 27 | R01-9-A | 5'-ATTO647N- |
| 28 | R01-9-B | 3'-Biotin |
| 29 | R01-10-A | 5'-ATTO647N- |
| 30 | R01-10-B | 3'-Biotin |
| 31 | R01-11-A | 5'-ATTO647N- |
| 32 | R01-11-B | 3'-Biotin |
| 33 | R01-12-A | 5'-ATTO647N- |
| 34 | R01-12-B | 3'-Biotin |
| 35 | R01-13-A | 5'-ATTO647N- |
| 36 | R01-13-B | 3'-Biotin |
| 37 | R01-14-A | 5'-ATTO647N- |
| 38 | R01-14-B | 3'-Biotin |
| 39 | R01-15-A | 5'-ATTO647N- |
| 40 | R01-15-B | 3'-Biotin |
| 41 | R01-16-A | 5'-ATTO647N- |
| 42 | R01-16-B | 3'-Biotin |
| 43 | R01-17-A | 5'-ATTO647N- |
| 44 | R01-17-B | 3'-Biotin |
| 45 | R01-18-A | 5'-ATTO647N- |
| 46 | R01-18-B | 3'-Biotin |
| 47 | R01-19-A | 5'-ATTO647N- |
| 48 | R01-19-B | 3'-Biotin |
| 49 | R01-20-A | 5'-ATTO647N- |
| 50 | R01-20-B | 3'-Biotin |
| 51 | R01-21-A | 5'-ATTO647N- |
| 52 | R01-21-B | 3'-Biotin |
| 53 | R01-22-A | 5'-ATTO647N- |
| 54 | R01-22-B | 3'-Biotin |
| 55 | R01-23-A | 5'-ATTO647N- |
| 56 | R01-23-B | 3'-Biotin |
| 57 | R01-24-A | 5'-ATTO647N- |
| 58 | R01-24-B | 3'-Biotin |
| 59 | R01-25-A | 5'-ATTO647N- |
| 60 | R01-25-B | 3'-Biotin |
| 61 | R01-26-A | 5'-ATTO647N- |
| 62 | R01-26-B | 3'-Biotin |
| 63 | R01-27-A | 5'-ATTO647N- |
| 64 | R01-27-B | 3'-Biotin |
| 65 | R01-28-A | 5'-ATTO647N- |
| 66 | R01-28-B | 3'-Biotin |
| 67 | R01-29-A | 5'-ATTO647N- |
| 68 | R01-29-B | 3'-Biotin |
| 69 | R01-30-A | 5'-ATTO647N- |
| 70 | R01-30-B | 3'-Biotin |
| 71 | R01-31-A | 5'-ATTO647N- |
| 72 | R01-31-B | 3'-Biotin |
| 73 | R01-32-A | 5'-ATTO647N- |
| 74 | R01-32-B | 3'-Biotin |
| 75 | R01-33-A | 5'-ATTO647N- |
| 76 | R01-33-B | 3'-Biotin |
| 77 | R01-34-A | 5'-ATTO647N- |
| 78 | R01-34-B | 3'-Biotin |
| 79 | R01-35-A | 5'-ATTO647N- |
| 80 | R01-35-B | 3'-Biotin |
| 81 | R01-36-A | 5'-ATTO647N- |
| 82 | R01-36-B | 3'-Biotin |
| 83 | R01-37-A | 5'-ATTO647N- |
| 84 | R01-37-B | 3'-Biotin |
| 85 | R01-38-A | 5'-ATTO647N- |
| 86 | R01-38-B | 3'-Biotin |
| 87 | R01-39-A | 5'-ATTO647N- |
| 88 | R01-39-B | 3'-Biotin |
| 89 | R01-40-A | 5'-ATTO647N- |
| 90 | R01-40-B | 3'-Biotin |
| 91 | R01-41-A | 5'-ATTO647N- |
| 92 | R01-41-B | 3'-Biotin |
| 93 | R01-42-A | 5'-ATTO647N- |
| 94 | R01-42-B | 3'-Biotin |
| 95 | R01-43-A | 5'-ATTO647N- |
| 96 | R01-43-B | 3'-Biotin |
| 97 | R01-44-A | 5'-ATTO647N- |
| 98 | R01-44-B | 3'-Biotin |
| 99 | R01-45-A | 5'-ATTO647N- |
| 100 | R01-45-B | 3'-Biotin |
| 101 | R01-46-A | 5'-ATTO647N- |
| 102 | R01-46-B | 3'-Biotin |
| 103 | R01-47-A | 5'-ATTO647N- |
| 104 | R01-47-B | 3'-Biotin |
| 105 | R01-48-A | 5'-ATTO647N- |
| 106 | R01-48-B | 3'-Biotin |
| 107 | R01-49-A | 5'-ATTO647N- |
| 108 | R01-49-B | 3'-Biotin |
| 109 | R01-50-A | 5'-ATTO647N- |
| 110 | R01-50-B | 3'-Biotin |
| 111 | R01-51-A | 5'-ATTO647N- |
| 112 | R01-51-B | 3'-Biotin |
| 113 | R01-52-A | 5'-ATTO647N- |
| 114 | R01-52-B | 3'-Biotin |
| 115 | R01-53-A | 5'-ATTO647N- |
| 116 | R01-53-B | 3'-Biotin |
| 117 | R01-54-A | 5'-ATTO647N- |
| 118 | R01-54-B | 3'-Biotin |
| 119 | R01-55-A | 5'-ATTO647N- |
| 120 | R01-55-B | 3'-Biotin |
| 121 | R01-56-A | 5'-ATTO647N- |
| 122 | R01-56-B | 3'-Biotin |
| 123 | R01-57-A | 5'-ATTO647N- |
| 124 | R01-57-B | 3'-Biotin |
| 125 | R01-58-A | 5'-ATTO647N- |
| 126 | R01-58-B | 3'-Biotin |
| 127 | R01-59-A | 5'-ATTO647N- |
| 128 | R01-59-B | 3'-Biotin |
| 129 | R01-60-A | 5'-ATTO647N- |
| 130 | R01-60-B | 3'-Biotin |
| 131 | R01-61-A | 5'-ATTO647N- |
| 132 | R01-61-B | 3'-Biotin |
| 133 | Gib11SpaCas9-1-M417L | |

### SEQUENCE LISTING

| **SEQ ID NO** | **Sequence** | **Description** |
|---|---|---|
| 1 | MKRNYILGLDIGITSVGYGIIDYETRDVIDAGVRLFKEANVENNEGRRSKRGARRLKRRR RHRIQRVKKLLFDYNLLTDHSELSGINPYEARVKGLSQKLSEEEFSAALLHLAKRRGVHN VNEVEEDTGNELSTKEQISRNSKALEEKYVAELQLERLKKDGEVRGSINRFKTSDYVKEA KQLLKVQKAYHQLDQSFIDTYIDLLETRRTYYEGPGEGSPFGWKDIKEWYEMLMGHCTYF PEELRSVKYAYNADLYNALNDLNNLVITRDENEKLEYYEKFQIIENVFKQKKKPTLKQIA KEILVNEEDIKGYRVTSTGKPEFTNLKVYHDIKDITARKEIIENAELLDQIAKILTIYQS SEDIQEELTNLNSELTQEEIEQISNLKGYTGTHNLSLKAINLILDXLWHTNXNQIXIFNX LKLVPKKVDLSQQKEIPTTLVDDFILSPVVKRSFIQSIKVINAIIKKYGLPNDIIIELAR EKNSKDAQKMINEMQKRNRQTNERIEEIIRTTGKENAKYLIEKIKLHDMQEGKCLYSLEA IPLEDLLNNPFNYEVDHIIPRSVSFDNSFNNKVLVKQEENSKKGNRTPFQYLSSSDSKIS YETFKKHILNLAKGKGRISKTKKEYLLEERDINRFSVQKDFINRNLVDTRYATRGLMNLL RSYFRVNNLDVKVKSINGGFTSFLRRKWKFKKERNKGYKHHAEDALIIANADFIFKEWKK LDKAKKVMENQMFEEKQAESMPEIETEQEYKEIFITPHQIKHIKDFKDYKYSHRVDKKPN RELINDTLYSTRKDDKGNTLIVNNLNGLYDKDNDKLKKLINKSPEKLLMYHHDPQTYQKL KLIMEQYGDEKNPLYKYYEETGNYLTKYSKKDNGPVIKKIKYYGNKLNAHLDITDDYPNS RNKVVKLSLKPYRFDVYLDNGVYKFVTVKNLDVIKKENYYEVNSKCYEEAKKLKKISNQA EFIASFYNNDLIKINGELYRVIGVNNDLLNRIEVNMIDITYREYLENMNDKRPPRIIKTI ASKTQSIKKYSTDILGNLYEVKSKKHPQIIKKG | M-SauCas9_X |
| 2 | MNQKFILGLDIGITSVGYGLIDYETKNIIDAGVRLFPEANVENNEGRRSKRGSRRLKRRR IHRLERVKKLLEDYNLLDQSQIPQSTNPYAIRVKGLSEALSKDELVIALLHIAKRRGIHK IDVIDSNDDVGNELSTKEQLNKNSKLLKDKFVCQIQLERMNEGQVRGEKNRFKTADIIKE IIQLLNVQKNFHQLDENFINKYIELVEMRREYFEGPGKGSPYGWEGDPKAWYETLMGHXT YFPDELRSVKYAYSADLFNALNDLNNLVIQRDGLSKLEYHEKYHIIENVFKQKKKPTLKQ IANEINVNPEDIKGYRITKSGKPQFTEFKLYHDLKSVLFDQSILENEDVLDQIAEILTIY QDKDSIKSKLTELDILLNEEDKENIAQLTGYTGTHRLSLKXIRLVLEXQWYSSXNQMXIF TXLNIKPKKINLTAANKIPKAMIDEFILSPVVKRTFGQAINLINKIIEKYGVPEDIIIEL ARENNSKDKQKFINEMQKKNENTRKRINEIIGKYGNQNAKRLVEKIRLHDEQEGKCLYSL ESIPLEDLLNNPNHYEVDHIIPRSVSFDNSYHNKVLVKQSENSKKSNLTPYQYFNSGKSK LSYNQFKQHILNLSKSQDRISKKKKEYLLEERDINKFEVQKEFINRNLVDTRYATRELTN YLKAYFSANNMNVKVKTINGSFTDYLRKVWKFKKERNHGYKHHAEDALIIANADFLFKEN KKLKAVNSVLEKPEIETKQLDIQVDSEDNYSEMFIIPKQVQDIKDFRNFKYSHRVDKKPN RQLINDTLYSTRKKDNSTYIVQTIKDIYAKDNTTLKKQFDKSPEKFLMYQHDPRTFEKLE VIMKQYANEKNPLAKYHEETGEYLTKYSKKNNGPIVKSLKYIGNKLGSHLDVTHQFKSST KKLVKLSIKPYRFDVYLTDKGYKFITISYLDVLKKDNYYYIPEQKYDKLKLGKAIDKNAK FIASFYKNDLIKLDGEIYKIIGVNSDTRNMIELDLPDIRYKEYCELNNIKGEPRIKKTIG KKVNSIEKLTTDVLGNVFTNTQYTKPQLLFKRGNGG | M-SluCas9_x |
| 3 | MKEKYILGLDLGITSVGYGIINFETKKIIDAGVRLFPEANVDNNEGRRSKRGSRRLKRRR IHRLERVKLLLTEYDLINKEQIPTSNNPYQIRVKGLSEILSKDELAIALLHLAKRRGIHN INVSSEDEDASNELSTKEQINRNNKLLKDKYVCEVQLQRLKEGQIRGEKNRFKTTDILKE IDQLLKVQKDYHNLDIDFINQYKEIVETRREYFEGPGQGSPFGWNGDLKKWYEMLMGHXT | M-SpaCas9_X |
| | YFPQELRSVKYAYSADLFNALNDLNNLIIQRDNSEKLEYHEKYHIIENVFKQKKKPTLKQ IAKEIGVNPEDIKGYRITKSGTPQFTEFKLYHDLKSIVFDKSILENEAILDQIAEILTIY QDEQSIKEELNKLPEILNEQDKAEIAKLIGYNGTHRLSLKXIHLINEXLWQTSXNQMXIF NXLNIKPNKVDLSEQNKIPKDMVNDFILSPVVKRTFIQSINVINKVIEKYGIPEDIIIEL ARENNSDDRKKFINNLQKKNEATRKRINEIIGQTGNQNAKRIVEKIRLHDQQEGKCLYSL ESIALMDLLNNPQNYEVDHIIPRSVAFDNSIHNKVLVKQIENSKKGNRTPYQYLNSSDAK LSYNQFKQHILNLSKSKDRISKKKKDYLLEERDINKFEVQKEFINRNLVDTRYATRELTS YLKAYFSANNMDVKVKTINGSFTNHLRKVWRFDKYRNHGYKHHAEDALIIANADFLFKEN KKLQNTNKILEKPTIENNTKKVTVEKEEDYNNVFETPKLVEDIKQYRDYKFSHRVDKKPN RQLINDTLYSTRMKDEHDYIVQTITDIYGKDNTNLKKQFNKNPEKFLMYQNDPKTFEKLS IIMKQYSDEKNPLAKYYEETGEYLTKYSKKNNGPIVKKIKLLGNKVGNHLDVTNKYENST KKLVKLSIKNYRFDVYLTEKGYKFVTIAYLNVFKKDNYYYIPKDKYQELKEKKKIKDTDQ FIASFYKNDLIKLNGDLYKIIGVNSDDRNIIELDYYDIKYKDYCEINNIKGEPRIKKTIG KKTESIEKFTTDVLGNLYLHSTEKAPQLIFKRGL | |
| 4 | MNNYILGLDIGITSVGYGIVDSDTREIKDAGVRLFPEANVDNNEGRRSKRGARRLKRRRI HRLDRVKHLLAEYDLLDLTNIPKSTNPYQTRVKGLNEKLSKDELVIALLHIAKRRGIHNV NVMMDDNDSGNELSTKDQLKKNAKALSDKYVCELQLERFEQDYKVRGEKNRFKTEDFVRE ARKLLETQSKFFEIDQTFIMRYIELIETRREYFEGPGKGSPFGWEGNIKKWFEQMMGHXT YFPEELRSVKYSYSAELFNALNDLNNLVITRDEDAKLNYGEKFQIIENVFKQKKTPNLKQ IAIEIGVHETEIKGYRVNKSGKPEFTQFKLYHDLKNIFKDPKYLNDIQLMDNIAEIITIY QDAESIIKELNQLPELLSEREKEKISALSGYSGTHRLSLKXINLLLDXLWESSXNQMXLF TXLNLKPKKIDLSQQHKIPSKLVDDFILSPVVKRAFIQSIQVVNAIIDKYGLPEDIIIEL ARENNSDDRRKFLNQLQKQNEETRKQVEKVLREYGNDNAKRIVQKIKLHNMQEGKCLYSL KDIPLEDLLRNPHHYEVDHIIPRSVAFDNSMHNKVLVRADENSKKGNRTPYQYLNSSESS LSYNEFKQHILNLSKTKDRITKKKREYLLEERDINKFDVQKEFINRNLVDTRYATRELTS LLKAYFSANNLDVKVKTINGSFTNYLRKVWKFDKDRNKGYKHHAEDALIIANADFLFKHN KKLRNINKVLDAPSKEVDKKRVTVQSEDEYNQIFEDTQKAQAIKKFEIRKFSHRVDKKPN RQLINDTLYSTRNIDGIEYVVESIKDIYSVNNDKVKTKFKKDPHRLLMYRNDPQTFEKFE KVFKQYESEKNPFAKYYEETGEKIRKFSKTGQGPYINKIKYLRERLGRHCDVTNKYINSR NKIVQLKIYSYRFDIYQYGNNYKMITISYIDLEQKSNYYYISREKYEQKKKDKQIDDSYK FIGSFYKNDIINYNGEMYRVIGVNDSEKNKIQLDMIDISIKDYMELNNIKKTGVIYKTIG KSTTHIEKYTTDILGNLYKAAPPKKPQLIFK | M-ShyCas9_X |
| 5 | MEKDYILGLDIGIGSVGYGLIDYDTKSIIDAGVRLFPEANADNNLGRRAKRGARRLKRRR IHRLERVKSLLSEYKIISGLAPTNNQPYNIRVKGLTEQLTKDELAVALLHIAKRRGIHNV DVAADKEETASDSLSTKDQINKNAKFLESRYVCELQKERLENEGHVRGVENRFLTKDIVR EAKKIIDTQMQYYPEIDETFKEKYISLVETRREYYEGPGKGSPYGWDADVKKWYQLMMGH XTYFPVEFRSVKYAYTADLYNALNDLNNLTIARDDNPKLEYHEKYHIIENVFKQKRNPTL KQIAKEIGVNDINISGYRVTKSGKPQFTSFKLFHDLKKVVKDHAILDDIDLLNQIAEILT IYQDKDSIVAELGQLEYLMSEADKQSISELTGYTGTHSLSLKXMNMIIDXLWHSSXNQMX VFTXLNMRPKKYELKGYQRIPTDMIDDAILSPVVKRSFKQAIGVVNAIIKKYGLPKDIII ELARESNSAEKSRYLRAIQKKNEKTRERIEAIIKEYGNENAKGLVQKIKLHDAQEGKCLY SLKDIPLEDLLRNPNNYDIDHIIPRSVSFDDSMHNKVLVRREQNAKKNNQTPYQYLTSGY ADIKYSVFKQHVLNLAENKDRMTKKKREYLLEERNINKYDVQKEFINRNLVDTRYTTREL TTLLKTYFTINNLDVKVKTINGSFTDFLRKRWGFKKNRDEGYKHHAEDALIIANADYLFK EHKLLKEIKDVSDLAGDERNSNVKDEDQYEEVFGGYFKIEDIKKYKIKKFSHRVDKKPNR QLINDTIYSTRVKDDKRYLINTLKNLYDKSNGDLKERMQKDPESLLMYHHDPQTFEKLKI VMSQYENEKNPLAKYFEETGQYLTKYAKHDNGPAIHKIKYYGNKLVEHLDITKNYHNPQN KVVQLSQKSFRFDVYQTDKGYKFISIAYLTLKNEKNYYAISQEKYDQLKSEKKISNNAVF IGSFYTSDIIEINNEKFRVIGVNSDKNNLIEVDRIDIRQKEFIELEEEKKNNRIKVTIGR KTTNIEKFHTDILGNMYKSKRPKAPQLVFKKG | M-SmiCas9_X |
| 6 | MKRNYILGLDIGITSVGYGIIDYETRDVIDAGVRLFKEANVENNEGRRSKRGARRLKRRR RHRIQRVKKLLFDYNLLTDHSELSGINPYEARVKGLSQKLSEEEFSAALLHLAKRRGVHN VNEVEEDTGNELSTKEQISRNSKALEEKYVAELQLERLKKDGEVRGSINRFKTSDYVKEA KQLLKVQKAYHQLDQSFIDTYIDLLETRRTYYEGPGEGSPFGWKDIKEWYEMLMGHCTYF PEELRSVKYAYNADLYNALNDLNNLVITRDENEKLEYYEKFQIIENVFKQKKKPTLKQIA KEILVNEEDIKGYRVTSTGKPEFTNLKVYHDIKDITARKEIIENAELLDQIAKILTIYQS SEDIQEELTNLNSELTQEEIEQISNLKGYTGTHNLSLKAINLILDELWHTNDNQIAIFNA LKLVPKKVDLSQQKEIPTTLVDDFILSPVVKRSFIQSIKVINAIIKKYGLPNDIIIELAR EKNSKDAQKMINEMQKRNRQTNERIEEIIRTTGKENAKYLIEKIKLHDMQEGKCLYSLEA IPLEDLLNNPFNYEVDHIIPRSVSFDNSFNNKVLVKQEENSKKGNRTPFQYLSSSDSKIS YETFKKHILNLAKGKGRISKTKKEYLLEERDINRFSVQKDFINRNLVDTRYATRGLMNLL RSYFRVNNLDVKVKSINGGFTSFLRRKWKFKKERNKGYKHHAEDALIIANADFIFKEWKK LDKAKKVMENQMFEEKQAESMPEIETEQEYKEIFITPHQIKHIKDFKDYKYSHRVDKKPN RELINDTLYSTRKDDKGNTLIVNNLNGLYDKDNDKLKKLINKSPEKLLMYHHDPQTYQKL KLIMEQYGDEKNPLYKYYEETGNYLTKYSKKDNGPVIKKIKYYGNKLNAHLDITDDYPNS RNKVVKLSLKPYRFDVYLDNGVYKFVTVKNLDVIKKENYYEVNSKCYEEAKKLKKISNQA EFIASFYNNDLIKINGELYRVIGVNNDLLNRIEVNMIDITYREYLENMNDKRPPRIIKTI ASKTQSIKKYSTDILGNLYEVKSKKHPQIIKKG | M-SauCas9R420 A |
| 7 | MNQKFILGLDIGITSVGYGLIDYETKNIIDAGVRLFPEANVENNEGRRSKRGSRRLKRRR IHRLERVKKLLEDYNLLDQSQIPQSTNPYAIRVKGLSEALSKDELVIALLHIAKRRGIHK IDVIDSNDDVGNELSTKEQLNKNSKLLKDKFVCQIQLERMNEGQVRGEKNRFKTADIIKE IIQLLNVQKNFHQLDENFINKYIELVEMRREYFEGPGKGSPYGWEGDPKAWYETLMGHCT YFPDELRSVKYAYSADLFNALNDLNNLVIQRDGLSKLEYHEKYHIIENVFKQKKKPTLKQ IANEINVNPEDIKGYRITKSGKPQFTEFKLYHDLKSVLFDQSILENEDVLDQIAEILTIY QDKDSIKSKLTELDILLNEEDKENIAQLTGYTGTHRLSLKCIRLVLEEQWYSSANQMEIF THLNIKPKKINLTAANKIPKAMIDEFILSPVVKRTFGQAINLINKIIEKYGVPEDIIIEL ARENNSKDKQKFINEMQKKNENTRKRINEIIGKYGNQNAKRLVEKIRLHDEQEGKCLYSL ESIPLEDLLNNPNHYEVDHIIPRSVSFDNSYHNKVLVKQSENSKKSNLTPYQYFNSGKSK LSYNQFKQHILNLSKSQDRISKKKKEYLLEERDINKFEVQKEFINRNLVDTRYATRELTN YLKAYFSANNMNVKVKTINGSFTDYLRKVWKFKKERNHGYKHHAEDALIIANADFLFKEN KKLKAVNSVLEKPEIETKQLDIQVDSEDNYSEMFIIPKQVQDIKDFRNFKYSHRVDKKPN RQLINDTLYSTRKKDNSTYIVQTIKDIYAKDNTTLKKQFDKSPEKFLMYQHDPRTFEKLE VIMKQYANEKNPLAKYHEETGEYLTKYSKKNNGPIVKSLKYIGNKLGSHLDVTHQFKSST KKLVKLSIKPYRFDVYLTDKGYKFITISYLDVLKKDNYYYIPEQKYDKLKLGKAIDKNAK FIASFYKNDLIKLDGEIYKIIGVNSDTRNMIELDLPDIRYKEYCELNNIKGEPRIKKTIG KKVNSIEKLTTDVLGNVFTNTQYTKPQLLFKRGNGG | M-SluCas9R414 A |
| 8 | MNQKFILGLDIGITSVGYGLIDYETKNIIDAGVRLFPEANVENNEGRRSKRGSRRLKRRR IHRLERVKLLLTEYDLINKEQIPTSNNPYQIRVKGLSEILSKDELAIALLHLAKRRGIHN VDVAADKEETASDSLSTKDQINKNAKFLESRYVCELQKERLENEGHVRGVENRFLTKDIV REAKKIIDTQMQYYPEIDETFKEKYISLVETRREYFEGPGQGSPFGWNGDLKKWYEMLMG HCTYFPQELRSVKYAYSADLFNALNDLNNLIIQRDNSEKLEYHEKYHIIENVFKQKKKPT LKQIAKEIGVNPEDIKGYRITKSGTPEFTSFKLFHDLKKVVKDHAILDDIDLLNQIAEIL TIYQDKDSIVAELGQLEYLMSEADKQSISELTGYTGTHSLSLKCMNMIIDALWHSSMNQM EVFTYLNMRPKKYELKGYQRIPTDMIDDAILSPVVKRTFIQSINVINKVIEKYGIPEDII IELARENNSDDRKKFINNLQKKNEATRKRINEIIGQTGNQNAKRIVEKIRLHDQQEGKCL YSLESIPLEDLLNNPNHYEVDHIIPRSVSFDNSYHNKVLVKQSENSKKSNLTPYQYFNSG KSKLSYNQFKQHILNLSKSQDRISKKKKEYLLEERDINKFEVQKEFINRNLVDTRYATRE LTSYLKAYFSANNMDVKVKTINGSFTNHLRKVWRFDKYRNHGYKHHAEDALIIANADFLF KENKKLQNTNKILEKPTIENNTKKVTVEKEEDYNNVFETPKLVEDIKQYRDYKFSHRVDK KPNRQLINDTLYSTRMKDEHDYIVQTITDIYGKDNTNLKKQFNKNPEKFLMYQNDPKTFE KLSIIMKQYSDEKNPLAKYYEETGEYLTKYSKKNNGPIVKKIKLLGNKVGNHLDVTNKYE NSTKKLVKLSIKNYRFDVYLTEKGYKFVTIAYLNVFKKDNYYYIPKDKYQELKEKKKIKD TDQFIASFYKNDLIKLNGDLYKIIGVNSDDRNIIELDYYDIKYKDYCEINNIKGEPRIKK TIGKKTESIEKFTTDVLGNLYLHSTEKAPQLIFKRGL | MGib11SpaC as9-3E410A |
| 9 | MNQKFILGLDIGITSVGYGLIDYETKNIIDAGVRLFPEANVENNEGRRSKRGSRRLKRRR IHRLERVKKLLEDYNLLDQSQIPQSTNPYAIRVKGLSEALSKDELVIALLHIAKRRGIHK IDVIDSNDDVGNELSTKEQLNKNSKLLKDKFVCQIQLERMNEGQVRGEKNRFKTADIIKE IIQLLNVQKNFHQLDENFINKYIELVEMRREYFEGPGKGSPYGWEGDPKAWYETLMGHCT YFPDELRSVKYAYSADLFNALNDLNNLVIQRDGLSKLEYHEKYHIIENVFKQKKKPTLKQ IANEINVNPEDIKGYRITKSGKPQFTEFKLYHDLKSVLFDQSILENEDVLDQIAEILTIY QDKDSIKSKLTELDILLNEEDKENIAQLTGYTGTHRLSLKCIRLVLEEQWYSSRNQMEIF THLNIKPKKINLTAANKIPKAMIDEFILSPVVKRTFGQAINLINKIIEKYGVPEDIIIEL ARENNSKDKQKFINEMQKKNENTRKRINEIIGKYGNQNAKRLVEKIRLHDEQEGKCLYSL ESIPLEDLLNNPNHYEVDHIIPRSVSFDNSYHNKVLVKQSENSKKSNLTPYQYFNSGKSK LSYNQFKQHILNLSKSQDRISKKKKEYLLEERDINKFEVQKEFINRNLVDTRYATRELTN YLKAYFSANNMNVKVKTINGSFTDYLRKVWKFKKERNHGYKHHAEDALIIANADFLFKEN KKLKAVNSVLEKPEIETKQLDIQVDSEDNYSEMFIIPKQVQDIKDFRNFKYSHRVDKKPN RQLINDTLYSTRKKDNSTYIVQTIKDIYAKDNTTLKKQFDKSPEKFLMYQHDPRTFEKLE VIMKQYANEKNPLAKYHEETGEYLTKYSKKNNGPIVKSLKYIGNKLGSHLDVTHQFKSST KKLVKLSIKPYRFDVYLTDKGYKFITISYLDVLKKDNYYYIPEQKYDKLKLGKAIDKNAK FIASFYKNDLIKLDGEIYKIIGVNSDTRNMIELDLPDIRYKEYCELNNIKGEPRIKKTIG KKVNSIEKLTTDVLGNVFTNTQYTKPQLLFKRGNGG | SluCas9 |
| 10 | ggugaacguggaugaaguugguuuuaguacucuggaaacagaaucuacugaaacaagacaa uaugucguguuuaucccaucaauuuauuggugggauuuuuuucuagcauaaccccuugggg ccucuaaacgggucuugagggguuuuuu | sgRNA_Ex1 |
| 11 | ggtgaacgtggatgaagttgtggggtac | R01-1-A |
| 12 | gcggtaccccacaacttcatccacgttcaccgg | R01-1-B |
| 13 | agtgaacgtggatgaagttgtggggtac | R01-2-A |
| 14 | gcggtaccccacaacttcatccacgttcactgg | R01-2-B |
| 15 | cgtgaacgtggatgaagttgtggggtac | R01-3-A |
| 16 | gcggtaccccacaacttcatccacgttcacggg | R01-3-B |
| 17 | tgtgaacgtggatgaagttgtggggtac | R01-4-A |
| 18 | gcggtaccccacaacttcatccacgttcacagg | R01-4-B |
| 19 | gatgaacgtggatgaagttgtggggtac | R01-5-A |
| 20 | gcggtaccccacaacttcatccacgttcatcgg | R01-5-B |
| 21 | gctgaacgtggatgaagttgtggggtac | R01-6-A |
| 22 | gcggtaccccacaacttcatccacgttcagcgg | R01-6-B |
| 23 | gttgaacgtggatgaagttgtggggtac | R01-7-A |
| 24 | gcggtaccccacaacttcatccacgttcaacgg | R01-7-B |
| 25 | ggagaacgtggatgaagttgtggggtac | R01-8-A |
| 26 | gcggtaccccacaacttcatccacgttctccgg | R01-8-B |
| 27 | ggcgaacgtggatgaagttgtggggtac | R01-9-A |
| 28 | gcggtaccccacaacttcatccacgttcgccgg | R01-9-B |
| 29 | ggggaacgtggatgaagttgtggggtac | R01-10-A |
| 30 | gcggtaccccacaacttcatccacgttccccgg | R01-10-B |
| 31 | ggtaaacgtggatgaagttgtggggtac | R01-11-A |
| 32 | gcggtaccccacaacttcatccacgtttaccgg | R01-11-B |
| 33 | ggtcaacgtggatgaagttgtggggtac | R01-12-A |
| 34 | gcggtaccccacaacttcatccacgttgaccgg | R01-12-B |
| 35 | ggttaacgtggatgaagttgtggggtac | R01-13-A |
| 36 | gcggtaccccacaacttcatccacgttaaccgg | R01-13-B |
| 37 | ggtgcacgtggatgaagttgtggggtac | R01-14-A |
| 38 | gcggtaccccacaacttcatccacgtgcaccgg | R01-14-B |
| 39 | ggtgtacgtggatgaagttgtggggtac | R01-15-A |
| 40 | gcggtaccccacaacttcatccacgtacaccgg | R01-15-B |
| 41 | ggtggacgtggatgaagttgtggggtac | R01-16-A |
| 42 | gcggtaccccacaacttcatccacgtccaccgg | R01-16-B |
| 43 | ggtgaccgtggatgaagttgtggggtac | R01-17-A |
| 44 | gcggtaccccacaacttcatccacggtcaccgg | R01-17-B |
| 45 | ggtgatcgtggatgaagttgtggggtac | R01-18-A |
| 46 | gcggtaccccacaacttcatccacgatcaccgg | R01-18-B |
| 47 | ggtgagcgtggatgaagttgtggggtac | R01-19-A |
| 48 | gcggtaccccacaacttcatccacgctcaccgg | R01-19-B |
| 49 | ggtgaaagtggatgaagttgtggggtac | R01-20-A |
| 50 | gcggtaccccacaacttcatccactttcaccgg | R01-20-B |
| 51 | ggtgaatgtggatgaagttgtggggtac | R01-21-A |
| 52 | gcggtaccccacaacttcatccacattcaccgg | R01-21-B |
| 53 | ggtgaaggtggatgaagttgtggggtac | R01-22-A |
| 54 | gcggtaccccacaacttcatccaccttcaccgg | R01-22-B |
| 55 | ggtgaacatggatgaagttgtggggtac | R01-23-A |
| 56 | gcggtaccccacaacttcatccatgttcaccgg | R01-23-B |
| 57 | ggtgaacctggatgaagttgtggggtac | R01-24-A |
| 58 | gcggtaccccacaacttcatccaggttcaccgg | R01-24-B |
| 59 | ggtgaacttggatgaagttgtggggtac | R01-25-A |
| 60 | gcggtaccccacaacttcatccaagttcaccgg | R01-25-B |
| 61 | ggtgaacgaggatgaagttgtggggtac | R01-26-A |
| 62 | gcggtaccccacaacttcatcctcgttcaccgg | R01-26-B |
| 63 | ggtgaacgcggatgaagttgtggggtac | R01-27-A |
| 64 | gcggtaccccacaacttcatccgcgttcaccgg | R01-27-B |
| 65 | ggtgaacggggatgaagttgtggggtac | R01-28-A |
| 66 | gcggtaccccacaacttcatccccgttcaccgg | R01-28-B |
| 67 | ggtgaacgtagatgaagttgtggggtac | R01-29-A |
| 68 | gcggtaccccacaacttcatctacgttcaccgg | R01-29-B |
| 69 | ggtgaacgtcgatgaagttgtggggtac | R01-30-A |
| 70 | gcggtaccccacaacttcatcgacgttcaccgg | R01-30-B |
| 71 | ggtgaacgttgatgaagttgtggggtac | R01-31-A |
| 72 | gcggtaccccacaacttcatcaacgttcaccgg | R01-31-B |
| 73 | ggtgaacgtgaatgaagttgtggggtac | R01-32-A |
| 74 | gcggtaccccacaacttcattcacgttcaccgg | R01-32-B |
| 75 | ggtgaacgtgcatgaagttgtggggtac | R01-33-A |
| 76 | gcggtaccccacaacttcatgcacgttcaccgg | R01-33-B |
| 77 | ggtgaacgtgtatgaagttgtggggtac | R01-34-A |
| 78 | gcggtaccccacaacttcatacacgttcaccgg | R01-34-B |
| 79 | ggtgaacgtggctgaagttgtggggtac | R01-35-A |
| 80 | gcggtaccccacaacttcagccacgttcaccgg | R01-35-B |
| 81 | ggtgaacgtggttgaagttgtggggtac | R01-36-A |
| 82 | gcggtaccccacaacttcaaccacgttcaccgg | R01-36-B |
| 83 | ggtgaacgtgggtgaagttgtggggtac | R01-37-A |
| 84 | gcggtaccccacaacttcacccacgttcaccgg | R01-37-B |
| 85 | ggtgaacgtggaagaagttgtggggtac | R01-38-A |
| 86 | gcggtaccccacaacttcttccacgttcaccgg | R01-38-B |
| 87 | ggtgaacgtggacgaagttgtggggtac | R01-39-A |
| 88 | gcggtaccccacaacttcgtccacgttcaccgg | R01-39-B |
| 89 | ggtgaacgtggaggaagttgtggggtac | R01-40-A |
| 90 | gcggtaccccacaacttcctccacgttcaccgg | R01-40-B |
| 91 | ggtgaacgtggataaagttgtggggtac | R01-41-A |
| 92 | gcggtaccccacaactttatccacgttcaccgg | R01-41-B |
| 93 | ggtgaacgtggatcaagttgtggggtac | R01-42-A |
| 94 | gcggtaccccacaacttgatccacgttcaccgg | R01-42-B |
| 95 | ggtgaacgtggattaagttgtggggtac | R01-43-A |
| 96 | gcggtaccccacaacttaatccacgttcaccgg | R01-43-B |
| 97 | ggtgaacgtggatgcagttgtggggtac | R01-44-A |
| 98 | gcggtaccccacaactgcatccacgttcaccgg | R01-44-B |
| 99 | ggtgaacgtggatgtagttgtggggtac | R01-45-A |
| 100 | gcggtaccccacaactacatccacgttcaccgg | R01-45-B |
| 101 | ggtgaacgtggatggagttgtggggtac | R01-46-A |
| 102 | gcggtaccccacaactccatccacgttcaccgg | R01-46-B |
| 103 | ggtgaacgtggatgacgttgtggggtac | R01-47-A |
| 104 | gcggtaccccacaacgtcatccacgttcaccgg | R01-47-B |
| 105 | ggtgaacgtggatgatgttgtggggtac | R01-48-A |
| 106 | gcggtaccccacaacatcatccacgttcaccgg | R01-48-B |
| 107 | ggtgaacgtggatgaggttgtggggtac | R01-49-A |
| 108 | gcggtaccccacaacctcatccacgttcaccgg | R01-49-B |
| 109 | ggtgaacgtggatgaaattgtggggta | R01-50-A |
| 110 | gcggtaccccacaatttcatccacgttcaccgg | R01-50-B |
| 111 | ggtgaacgtggatgaacttgtggggtac | R01-51-A |
| 112 | gcggtaccccacaagttcatccacgttcaccgg | R01-51-B |
| 113 | ggtgaacgtggatgaatttgtggggtac | R01-52-A |
| 114 | gcggtaccccacaaattcatccacgttcaccgg | R01-52-B |
| 115 | ggtgaacgtggatgaagatgtggggtac | R01-53-A |
| 116 | gcggtaccccacatcttcatccacgttcaccgg | R01-53-B |
| 117 | ggtgaacgtggatgaagctgtggggtac | R01-54-A |
| 118 | gcggtaccccacagcttcatccacgttcaccgg | R01-54-B |
| 119 | ggtgaacgtggatgaaggtgtggggtac | R01-55-A |
| 120 | gcggtaccccacaccttcatccacgttcaccgg | R01-55-B |
| 121 | ggtgaacgtggatgaagtagtggggtac | R01-56-A |
| 122 | gcggtaccccactacttcatccacgttcaccgg | R01-56-B |
| 123 | ggtgaacgtggatgaagtcgtggggtac | R01-57-A |
| 124 | gcggtaccccacgacttcatccacgttcaccgg | R01-57-B |
| 125 | ggtgaacgtggatgaagtggtggggtac | R01-58-A |
| 126 | gcggtaccccaccacttcatccacgttcaccgg | R01-58-B |
| 127 | ggtgaacgtggatgaagttatggggtac | R01-59-A |
| 128 | gcggtaccccataacttcatccacgttcaccgg | R01-59-B |
| 129 | ggtgaacgtggatgaagttctggggtac | R01-60-A |
| 130 | gcggtaccccagaacttcatccacgttcaccgg | R01-60-B |
| 131 | ggtgaacgtggatgaagttttggggtac | R01-61-A |
| 132 | gcggtaccccaaaacttcatccacgttcaccgg | R01-61-B |
| 133 | MNQKFILGLDIGITSVGYGLIDYETKNIIDAGVRLFPEANVENNEGRRSKRGSRRLKRRR IHRLERVKLLLTEYDLINKEQIPTSNNPYQIRVKGLSEILSKDELAIALLHLAKRRGIHN VDVAADKEETASDSLSTKDQINKNAKFLESRYVCELQKERLENEGHVRGVENRFLTKDIV REAKKIIDTQMQYYPEIDETFKEKYISLVETRREYFEGPGQGSPFGWNGDLKKWYEMLMG HCTYFPQELRSVKYAYSADLFNALNDLNNLIIQRDNSEKLEYHEKYHIIENVFKQKKKPT LKQIAKEIGVNPEDIKGYRITKSGTPEFTSFKLFHDLKKVVKDHAILDDIDLLNQIAEIL TIYQDKDSIVAELGQLEYLMSEADKQSISELTGYTGTHSLSLKCMNMIIDELWHSSLNQM EVFTYLNMRPKKYELKGYQRIPTDMIDDAILSPVVKRTFIQSINVINKVIEKYGIPEDII IELARENNSDDRKKFINNLQKKNEATRKRINEIIGQTGNQNAKRIVEKIRLHDQQEGKCL YSLESIPLEDLLNNPNHYEVDHIIPRSVSFDNSYHNKVLVKQSENSKKSNLTPYQYFNSG KSKLSYNQFKQHILNLSKSQDRISKKKKEYLLEERDINKFEVQKEFINRNLVDTRYATRE LTNYLKAYFSANNMNVKVKTINGSFTDYLRKVWKFKKERNHGYKHHAEDALIIANADFLF KENKKLKAVNSVLEKPEIETKQLDIQVDSEDNYSEMFIIPKQVQDIKDFRNFKYSHRVDK KPNRQLINDTLYSTRKKDNSTYIVQTIKDIYAKDNTTLKKQFDKSPEKFLMYQHDPRTFE KLEVIMKQYANEKNPLAKYHEETGEYLTKYSKKNNGPIVKSLKYIGNKLGSHLDVTHQFK SSTKKLVKLSIKNYRFDVYLTEKGYKFVTIAYLNVFKKDNYYYIPKDKYQELKEKKKIKD TDQFIASFYKNDLIKLNGDLYKIIGVNSDDRNIIELDYYDIKYKDYCEINNIKGEPRIKK TIGKKTESIEKFTTDVLGNLYLHSTEKAPQLIFKRGL | Gib11SpaCas 9-1-M417L |

## Claims

1. A M-SmallCas9 polypeptide comprising an amino acid sequence according to SEQ ID NO. 7 (M-SluCas9-R414A).

2. A composition comprising a M-SmallCas9 polypeptide according to claim 1.

3. A composition comprising
(I) a M-SmallCas9 polypeptide according to claim 1; and
(II) one or more single heterologous guide RNA(s) (sgRNA(s)) or DNA(s) that allow the generation of such one or more sgRNA(s) in situ, each sgRNA or DNA encoding an sgRNA comprising:
(a) an engineered DNA targeting segment that can hybridize to a target sequence in a polynucleotide locus,
(b) a tracr mate sequence, and
(c) a tracr RNA sequence,
wherein the tracr mate sequence can hybridize to the tracr RNA sequence, and
wherein (a), (b), and (c) are arranged in a 5' to 3' orientation.

4. The composition according to claim 2 or 3, wherein the composition is a pharmaceutical composition.

5. The composition according to claim 4, wherein the pharmaceutical composition is for use in gene therapy.

6. Method of targeting, editing, modifying, or manipulating a target DNA in vitro, the method comprising
(I) introducing a heterologous M-SmallCas9 polypeptide according to claim 1 or a nucleic acid encoding a M-SmallCas9 of claim 1 into the in vitro environment; and
(II) introducing one or more single heterologous guide RNA(s) (sgRNA(s)) or DNA(s) encoding such one or more sgRNA(s) in the in vitro environment, each sgRNA or DNA encoding the sgRNA comprising:
(a) an engineered DNA targeting segment comprising an RNA and capable of hybridizing to a target sequence in a polynucleotide locus,
(b) a tracr mate sequence comprised of RNA, and
(c) a tracr RNA sequence comprised of RNA,
wherein the tracr mate sequence hybridizes to the tracr RNA sequence, and
wherein (a), (b), and (c) are arranged in a 5' to 3' orientation; and
(III) creating one or more nicks or cuts or base edits in the target DNA, wherein the M-SmallCas9 polypeptide is directed to the target DNA by the sgRNA in its processed or unprocessed form.

7. Use of a composition comprising
(I) a M-SmallCas9 polypeptide according to claim 1 or a nucleic acid encoding the same; and optionally
(II) one or more single heterologous guide RNA(s) (sgRNA(s)) or DNA(s) suitable for the generation of such one or more sgRNA(s) in situ, each comprising:
(a) an engineered DNA targeting segment comprised of RNA and capable of hybridizing to such target sequence in a polynucleotide locus,
(b) a tracr mate sequence comprised of RNA, and
(c) a tracr RNA sequence comprised of RNA,
wherein the tracr mate sequence hybridizes to the tracr RNA sequence, and wherein (a), (b), and (c) are arranged in a 5' to 3' orientation;
for targeting, editing, modifying, or manipulating a target DNA in vitro.

8. An isolated cell comprising
(I) a M-SmallCas9 polypeptide according to claim 1, or a nucleic acid encoding a M-SmallCas9 polypeptide according to claim 1; and
(II) one or more single heterologous guide RNA(s) (sgRNA(s)) or DNA(s) suitable for the generation of such one or more sgRNA(s) in situ, each comprising:
(a) an engineered DNA targeting segment that can hybridize to a target sequence in a polynucleotide locus,
(b) a tracr mate sequence, and
(c) a tracr RNA sequence,
wherein the tracr mate sequence can hybridize to the tracr RNA sequence, and
wherein (a), (b), and (c) are arranged in a 5' to 3' orientation.

9. A kit comprising
(I) a nucleic acid sequence encoding a M-SmallCas9 polypeptide according to claim 1, wherein the nucleic acid sequence encoding the M-SmallCas9 is operably linked to a promoter; and
(II) one or more single heterologous guide RNA(s) (sgRNA(s)) or DNA(s) suitable for the generation of such one or more sgRNA(s) in situ, each sgRNA comprising:
(a) an engineered DNA targeting segment that can hybridize to a target sequence in a polynucleotide locus,
(b) a tracr mate sequence, and
(c) a tracr RNA sequence,
wherein the tracr mate sequence can hybridize to the tracr RNA sequence, and
wherein (a), (b), and (c) are arranged in a 5' to 3' orientation.

## Patentansprüche

1. M-SmallCas9-Polypeptid, umfassend eine Aminosäuresequenz nach SEQ ID NO:7 (M-SluCas9-R414A).

2. Zusammensetzung, umfassend ein M-SmallCas9-Polypeptid nach Anspruch 1.

3. Zusammensetzung umfassend:
(I) ein M-SmallCas9-Polypeptid nach Anspruch 1; und
(II) eine oder mehrere heterologe Single-Guide-RNA(s) (sgRNA(s)) oder DNA(s), die die Erzeugung einer oder mehrerer solcher sgRNA(s) *in situ* ermöglichen, wobei jede sgRNA oder DNA, die eine sgRNA codiert, umfasst:
(a) ein gentechnisch hergestelltes DNA-Targeting-Segment, das an eine Zielsequenz in einem Polynucleotid-Locus hybridisieren kann,
(b) eine tracr-mate-Sequenz, und
(c) eine tracr-RNA-Sequenz,
wobei die tracr-mate-Sequenz an die tracr-RNA-Sequenz hybridisieren kann und wobei (a), (b) und (c) in einer 5' → 3'-Orientierung angeordnet sind.

4. Zusammensetzung nach Anspruch 2 oder 3, wobei die Zusammensetzung eine pharmazeutische Zusammensetzung ist.

5. Zusammensetzung nach Anspruch 4, wobei die pharmazeutische Zusammensetzung zur Verwendung in der Gentherapie ist.

6. Verfahren zum Targeting, Editieren, Modifizieren oder Manipulieren einer Ziel-DNA in *vitro,* wobei das Verfahren umfasst:
(I) Einbringen eines heterologen M-SmallCas9-Polypeptids nach Anspruch 1 oder einer Nucleinsäure, die ein M-SmallCas9 nach Anspruch 1 codiert, in die *in* vitro-Umgebung; und
(II) Einbringen einer oder mehrerer heterologer Single-Guide-RNA(s) (sgRNA(s)) oder DNA(s), die eine oder mehrere solcher sgRNA(s) codieren, in die *in vitro-*Umgebung, wobei jede sgRNA oder DNA, die die sgRNA codiert, umfasst:
(a) ein gentechnisch hergestelltes DNA-Targeting-Segment, das eine RNA umfasst und das in der Lage ist, an eine Zielsequenz in einem Polynucleotid-Locus zu hybridisieren,
(b) eine tracr-mate-Sequenz, die aus RNA besteht, und
(c) eine tracr-RNA-Sequenz, die aus RNA besteht,
wobei die tracr-mate-Sequenz an die tracr-RNA-Sequenz hybridisiert und wobei (a), (b) und (c) in einer 5' → 3'-Orientierung angeordnet sind; und
(III) Erzeugen eines oder mehrerer Nicks oder Schnitte oder Baseneditierungen in der Ziel-DNA, wobei das M-SmallCas9-Polypeptid durch die sgRNA in ihrer prozessierten oder nicht prozessierten Form auf die Ziel-DNA gerichtet wird.

7. Verwendung einer Zusammensetzung, umfassend:
(I) ein M-SmallCas9-Polypeptid nach Anspruch 1 oder einer Nucleinsäure, die dieses codiert; und gegebenenfalls
(II) eine oder mehrere heterologe Single-Guide-RNA(s) (sgRNA(s)) oder DNA(s), die für die Erzeugung einer oder mehrerer solcher sgRNA(s) *in situ* geeignet sind, wobei jede umfasst:
(a) ein gentechnisch hergestelltes DNA-Targeting-Segment, das aus RNA besteht und in der Lage ist, an eine solche Zielsequenz in einem Polynucleotid-Locus zu hybridisieren;
(b) eine tracr-mate-Sequenz, die aus RNA besteht, und
(c) eine tracr-RNA-Sequenz, die aus RNA besteht,
wobei die tracr-mate-Sequenz an die tracr-RNA-Sequenz hybridisiert und wobei (a), (b) und (c) in einer 5' → 3'-Orientierung angeordnet sind;
zum Targeting, Editieren, Modifizieren oder Manipulieren einer Ziel-DNA in *vitro.*

8. Isolierte Zelle, umfassend:
(I) ein M-SmallCas9-Polypeptid nach Anspruch 1 oder eine Nucleinsäure, die ein M-SmallCas9-Polypeptid nach Anspruch 1 codiert; und
(II) eine oder mehrere heterologe Single-Guide-RNA(s) (sgRNA(s)) oder DNA(s), die für die Erzeugung einer oder mehrerer solcher sgRNA(s) *in situ* geeignet sind, wobei jede umfasst:
(a) ein gentechnisch hergestelltes DNA-Targeting-Segment, das an eine Zielsequenz in einem Polynucleotid-Locus hybridisieren kann,
(b) eine tracr-mate-Sequenz, und
(c) eine tracr-RNA-Sequenz,
wobei die tracr-mate-Sequenz an die tracr-RNA-Sequenz hybridisieren kann und wobei (a), (b) und (c) in einer 5' → 3'-Orientierung angeordnet sind.

9. Kit, umfassend:
(I) eine Nucleinsäuresequenz, die ein M-SmallCas9-Polypeptid nach Anspruch 1 codiert, wobei die Nucleinsäuresequenz, die das M-SmallCas9 codiert, funktionell mit einem Promotor verknüpft ist; und
(II) eine oder mehrere heterologe Single-Guide-RNA(s) (sgRNA(s)) oder DNA(s), die für die Erzeugung einer oder mehrerer solcher sgRNA(s) *in situ* geeignet sind, wobei jede sgRNA umfasst:
(a) ein gentechnisch hergestelltes DNA-Targeting-Segment, das an eine Zielsequenz in einem Polynucleotid-Locus hybridisieren kann,
(b) eine tracr-mate-Sequenz, und
(c) eine tracr-RNA-Sequenz,
wobei die tracr-mate-Sequenz an die tracr-RNA-Sequenz hybridisieren kann und wobei (a), (b) und (c) in einer 5' → 3'-Orientierung angeordnet sind.

## Revendications

1. Polypeptide M-SmallCas9 comprenant une séquence d'acides aminés conformément à la SEQ ID NO. 7 (M-SluCas9-R414A).

2. Composition comprenant un polypeptide M-SmallCas9 selon la revendication 1.

3. Composition comprenant
(I) un polypeptide M-SmallCas9 selon la revendication 1 ; et
(II) un ou plusieurs ARN(s) guides hétérologues simples (ARN(s)gs) ou ADN(s) qui permettent la génération de tel ou tels ARN(s)gs in situ, chaque ARNgs ou ADN codant un ARNgs comprenant :
(a) un segment ciblant l'ADN manipulé qui peut s'hybrider à une séquence cible dans un locus de polynucléotide,
(b) une séquence mate tracr, et
(c) une séquence d'ARN tracr,
où la séquence mate tracr peut s'hybrider à la séquence d'ARN tracr, et
où (a), (b) et (c) sont disposés dans une orientation 5' à 3'.

4. Composition selon la revendication 2 ou 3, où la composition est une composition pharmaceutique.

5. Composition selon la revendication 4, où la composition pharmaceutique est destinée à une utilisation en thérapie génique.

6. Méthode de ciblage, d'édition, de modification ou de manipulation d'un ADN cible in vitro, ladite méthode comprenant
(I) l'introduction d'un polypeptide M-SmallCas9 hétérologue selon la revendication 1 ou d'un acide nucléique codant pour un M-SmallCas9 selon la revendication 1 dans l'environnement in vitro ; et
(II) l'introduction d'un ou de plusieurs ARN(s) guides hétérologues simples (ARN(s)gs) ou ADN(s) codant pour tel ou tels ARN(s)gs dans l'environnement in vitro, chaque ARNgs ou ADN codant l'ARNgs comprenant :
(a) un segment ciblant l'ADN manipulé comprenant un ARN et susceptible de s'hybrider à une séquence cible dans un locus de polynucléotide,
(b) une séquence mate tracr constituée d'ARN, et
(c) une séquence d'ARN tracr constituée d'ARN,
où la séquence mate tracr s'hybride à la séquence d'ARN tracr, et
où (a), (b) et (c) sont disposés dans une orientation 5' à 3' ; et
(III) la création d'une ou de plusieurs cassures ou coupures ou modifications de base dans l'ADN cible, où le polypeptide M-SmallCas9 est dirigé sur l'ADN cible par l'ARNgs dans sa forme traitée ou non traitée.

7. Utilisation d'une composition comprenant
(I) un polypeptide M-SmallCas9 selon la revendication 1 ou un acide nucléique codant pour celui-ci ; et éventuellement
(II) un ou plusieurs ARN(s) guides hétérologues simples (ARN(s)gs) ou ADN(s) appropriés pour la génération de tel ou tels ARN(s)gs in situ, chacun comprenant :
(a) un segment ciblant l'ADN manipulé constitué d'ARN et susceptible de s'hybrider à une telle séquence cible dans un locus de polynucléotide,
(b) une séquence mate tracr constituée d'ARN, et
(c) une séquence d'ARN tracr constituée d'ARN,
où la séquence mate tracr s'hybride à la séquence d'ARN tracr, et
où (a), (b) et (c) sont disposés dans une orientation 5' à 3' ;
pour le ciblage, l'édition, la modification ou la manipulation d'un ADN cible in vitro.

8. Cellule isolée comprenant
(I) un polypeptide M-SmallCas9 selon la revendication 1 ou un acide nucléique codant pour un polypeptide M-SmallCas9 selon la revendication 1 ; et
(II) un ou plusieurs ARN(s) guides hétérologues simples (ARN(s)gs) ou ADN(s) appropriés pour la génération de tel ou tels ARN(s)gs in situ, chacun comprenant :
(a) un segment ciblant l'ADN manipulé qui peut s'hybrider à une séquence cible dans un locus de polynucléotide,
(b) une séquence mate tracr, et
(c) une séquence d'ARN tracr,
où la séquence mate tracr peut s'hybrider à la séquence d'ARN tracr,
et où (a), (b) et (c) sont disposés dans une orientation 5' à 3'.

9. Kit comprenant :
(I) une séquence d'acide nucléique codant pour un polypeptide M-SmallCas9 selon la revendication 1, où la séquence d'acide nucléique codant pour le M-SmallCas9 est liée fonctionnellement à un promoteur ; et
(II) un ou plusieurs ARN(s) guides hétérologues simples (ARN(s)gs) ou ADN(s) appropriés pour la génération de tel ou tels ARN(s)gs in situ, chaque ARNgs comprenant :
(a) un segment ciblant l'ADN manipulé qui peut s'hybrider à une séquence cible dans un locus de polynucléotide,
(b) une séquence mate tracr, et
(c) une séquence d'ARN tracr,
où la séquence mate tracr peut s'hybrider à la séquence d'ARN tracr, et
où (a), (b) et (c) sont disposés dans une orientation 5' à 3'.
